# EUROPEAN PATENT APPLICATION

(11) **EP 4 009 049 A1**
(43) Date of publication of application: **08.06.2022**
(21) Application number: 20210959.1
(22) Date of filing: 01.12.2020
(51) Int. Cl.: G01N 33/543, G01N 33/569, C02F 1/48

(54) **IMMOBILIZATION AND MAGNETIC EXTRACTION OF PATHOGENS AND PATHOGEN COMPONENTS**

(71) Applicant: Friedrich-Alexander-Universität Erlangen-Nürnberg, 91054 Erlangen (DE)
(72) Inventor: ALEXIOU, Christoph, 91052 Erlangen (DE); LYER, Stefan, 91052 Erlangen (DE); TIETZE, Rainer, 91074 Herzogenaurach (DE); FRIEDRICH, Bernhard, 90762 Fürth (DE)
(74) Representative: Wichmann, Hendrik

(57) **Abstract**

The application describes a method for reducing the concentration of pathogens and/or pathogen components in an aqueous or body fluid sample. Specifically, the method relates to incubating the sample with superparamagnetic iron-based particles attached to a target binding peptide and immobilising the superparamagnetic iron-based particles with a magnetic field and thereby separating the pathogen-bound and/or pathogen component-bound superparamagnetic iron-based particles from the sample. Furthermore, the application relates to a method for identifying pathogens in an aqueous or body fluid sample a use of superparamagnetic iron-based particles for reducing the concentration of pathogens and/or pathogen components in an aqueous or body fluid sample. In addition, a use of superparamagnetic iron-based particles for identifying pathogens in an aqueous or body fluid sample is disclosed. Finally, superparamagnetic ironoxide nanoparticles (SPIONs) are disclosed, wherein the SPIONs are linked to a target binding peptide, wherein the target is a pathogen, and/or a pathogen component.

SEQUENCE LISTING

<110>  Friedrich-Alexander-Universität, Erlangen-Nürnberg

<120>  Immobilization and magnetic extraction of pathogens and pathogen
       components

<130>  EPA-149 063

<160>  47

<170>  PatentIn version 3.5

<210>  1
<211>  18
<212>  PRT
<213>  Artificial Sequence

<220>
<223>  synthetic peptide

<400>  1

Arg Lys Gln Gly Arg Val Glu Val Leu Tyr Arg Ala Ser Trp Gly Thr
1               5                   10                  15

Val Cys

<210>  2
<211>  18
<212>  PRT
<213>  Artificial sequence

<220>
<223>  synthetic peptide

<400>  2

Arg Lys Gln Gly Arg Val Glu Ile Leu Tyr Arg Gly Ser Trp Gly Thr
1               5                   10                  15

Val Cys

<210>  3
<211>  18
<212>  PRT
<213>  Artificial Sequence

<220>
<223>  syntheic peptide

<220>
<221>  MOD_RES
<222>  (18)..(18)
<223>  PHOSPHORYLATION

<400>  3

Arg Lys Gln Gly Arg Val Glu Val Leu Tyr Arg Ala Ser Trp Gly Thr
1               5                   10                  15

Val Ser

<210>  4
<211>  2413
<212>  PRT
<213>  Homo sapiens

<400>  4

Met Gly Ile Ser Thr Val Ile Leu Glu Met Cys Leu Leu Trp Gly Gln
1               5                   10                  15

Val Leu Ser Thr Gly Gly Trp Ile Pro Arg Thr Thr Asp Tyr Ala Ser
            20                  25                  30

Leu Ile Pro Ser Glu Val Pro Leu Asp Pro Thr Val Ala Glu Gly Ser
        35                  40                  45

Pro Phe Pro Ser Glu Ser Thr Leu Glu Ser Thr Val Ala Glu Gly Ser
    50                  55                  60

Pro Ile Ser Leu Glu Ser Thr Leu Glu Ser Thr Val Ala Glu Gly Ser
65                  70                  75                  80

Leu Ile Pro Ser Glu Ser Thr Leu Glu Ser Thr Val Ala Glu Gly Ser
                85                  90                  95

Asp Ser Gly Leu Ala Leu Arg Leu Val Asn Gly Asp Gly Arg Cys Gln
            100                 105                 110

Gly Arg Val Glu Ile Leu Tyr Arg Gly Ser Trp Gly Thr Val Cys Asp
        115                 120                 125

Asp Ser Trp Asp Thr Asn Asp Ala Asn Val Val Cys Arg Gln Leu Gly
    130                 135                 140

Cys Gly Trp Ala Met Ser Ala Pro Gly Asn Ala Trp Phe Gly Gln Gly
145                 150                 155                 160

Ser Gly Pro Ile Ala Leu Asp Asp Val Arg Cys Ser Gly His Glu Ser
                165                 170                 175

Tyr Leu Trp Ser Cys Pro His Asn Gly Trp Leu Ser His Asn Cys Gly
            180                 185                 190

His Gly Glu Asp Ala Gly Val Ile Cys Ser Ala Ala Gln Pro Gln Ser
        195                 200                 205

Thr Leu Arg Pro Glu Ser Trp Pro Val Arg Ile Ser Pro Pro Val Pro
    210                 215                 220

Thr Glu Gly Ser Glu Ser Ser Leu Ala Leu Arg Leu Val Asn Gly Gly
225                 230                 235                 240

Asp Arg Cys Arg Gly Arg Val Glu Val Leu Tyr Arg Gly Ser Trp Gly
                245                 250                 255

Thr Val Cys Asp Asp Tyr Trp Asp Thr Asn Asp Ala Asn Val Val Cys
            260                 265                 270

Arg Gln Leu Gly Cys Gly Trp Ala Met Ser Ala Pro Gly Asn Ala Gln
        275                 280                 285

Phe Gly Gln Gly Ser Gly Pro Ile Val Leu Asp Asp Val Arg Cys Ser
    290                 295                 300

Gly His Glu Ser Tyr Leu Trp Ser Cys Pro His Asn Gly Trp Leu Thr
305                 310                 315                 320

His Asn Cys Gly His Ser Glu Asp Ala Gly Val Ile Cys Ser Ala Pro
                325                 330                 335

Gln Ser Arg Pro Thr Pro Ser Pro Asp Thr Trp Pro Thr Ser His Ala
            340                 345                 350

Ser Thr Ala Gly Pro Glu Ser Ser Leu Ala Leu Arg Leu Val Asn Gly
        355                 360                 365

Gly Asp Arg Cys Gln Gly Arg Val Glu Val Leu Tyr Arg Gly Ser Trp
    370                 375                 380

Gly Thr Val Cys Asp Asp Ser Trp Asp Thr Ser Asp Ala Asn Val Val
385                 390                 395                 400

Cys Arg Gln Leu Gly Cys Gly Trp Ala Thr Ser Ala Pro Gly Asn Ala
                405                 410                 415

Arg Phe Gly Gln Gly Ser Gly Pro Ile Val Leu Asp Asp Val Arg Cys
            420                 425                 430

Ser Gly Tyr Glu Ser Tyr Leu Trp Ser Cys Pro His Asn Gly Trp Leu

        435                 440                 445

Ser His Asn Cys Gln His Ser Glu Asp Ala Gly Val Ile Cys Ser Ala
    450                 455                 460

Ala His Ser Trp Ser Thr Pro Ser Pro Asp Thr Leu Pro Thr Ile Thr
465                 470                 475                 480

Leu Pro Ala Ser Thr Val Gly Ser Glu Ser Ser Leu Ala Leu Arg Leu
                485                 490                 495

Val Asn Gly Gly Asp Arg Cys Gln Gly Arg Val Glu Val Leu Tyr Arg
            500                 505                 510

Gly Ser Trp Gly Thr Val Cys Asp Asp Ser Trp Asp Thr Asn Asp Ala
        515                 520                 525

Asn Val Val Cys Arg Gln Leu Gly Cys Gly Trp Ala Met Leu Ala Pro
    530                 535                 540

Gly Asn Ala Arg Phe Gly Gln Gly Ser Gly Pro Ile Val Leu Asp Asp
545                 550                 555                 560

Val Arg Cys Ser Gly Asn Glu Ser Tyr Leu Trp Ser Cys Pro His Asn
                565                 570                 575

Gly Trp Leu Ser His Asn Cys Gly His Ser Glu Asp Ala Gly Val Ile
            580                 585                 590

Cys Ser Gly Pro Glu Ser Ser Leu Ala Leu Arg Leu Val Asn Gly Gly
        595                 600                 605

Asp Arg Cys Gln Gly Arg Val Glu Val Leu Tyr Arg Gly Ser Trp Gly
    610                 615                 620

Thr Val Cys Asp Asp Ser Trp Asp Thr Asn Asp Ala Asn Val Val Cys
625                 630                 635                 640

Arg Gln Leu Gly Cys Gly Trp Ala Met Ser Ala Pro Gly Asn Ala Arg
                645                 650                 655

Phe Gly Gln Gly Ser Gly Pro Ile Val Leu Asp Asp Val Arg Cys Ser
            660                 665                 670

Gly His Glu Ser Tyr Leu Trp Ser Cys Pro Asn Asn Gly Trp Leu Ser
        675                 680                 685

His Asn Cys Gly His His Glu Asp Ala Gly Val Ile Cys Ser Ala Ala
    690                 695                 700

Gln Ser Arg Ser Thr Pro Arg Pro Asp Thr Leu Ser Thr Ile Thr Leu
705                 710                 715                 720

Pro Pro Ser Thr Val Gly Ser Glu Ser Ser Leu Thr Leu Arg Leu Val
                725                 730                 735

Asn Gly Ser Asp Arg Cys Gln Gly Arg Val Glu Val Leu Tyr Arg Gly
            740                 745                 750

Ser Trp Gly Thr Val Cys Asp Asp Ser Trp Asp Thr Asn Asp Ala Asn
        755                 760                 765

Val Val Cys Arg Gln Leu Gly Cys Gly Trp Ala Thr Ser Ala Pro Gly
    770                 775                 780

Asn Ala Arg Phe Gly Gln Gly Ser Gly Pro Ile Val Leu Asp Asp Val
785                 790                 795                 800

Arg Cys Ser Gly His Glu Ser Tyr Leu Trp Ser Cys Pro His Asn Gly
                805                 810                 815

Trp Leu Ser His Asn Cys Gly His His Glu Asp Ala Gly Val Ile Cys
            820                 825                 830

Ser Val Ser Gln Ser Arg Pro Thr Pro Ser Pro Asp Thr Trp Pro Thr
        835                 840                 845

Ser His Ala Ser Thr Ala Gly Pro Glu Ser Ser Leu Ala Leu Arg Leu
    850                 855                 860

Val Asn Gly Gly Asp Arg Cys Gln Gly Arg Val Glu Val Leu Tyr Arg
865                 870                 875                 880

Gly Ser Trp Gly Thr Val Cys Asp Asp Ser Trp Asp Thr Ser Asp Ala
                885                 890                 895

Asn Val Val Cys Arg Gln Leu Gly Cys Gly Trp Ala Thr Ser Ala Pro
            900                 905                 910

Gly Asn Ala Arg Phe Gly Gln Gly Ser Gly Pro Ile Val Leu Asp Asp
        915                 920                 925

Val Arg Cys Ser Gly Tyr Glu Ser Tyr Leu Trp Ser Cys Pro His Asn
    930                 935                 940

Gly Trp Leu Ser His Asn Cys Gln His Ser Glu Asp Ala Gly Val Ile
945                 950                 955                 960

Cys Ser Ala Ala His Ser Trp Ser Thr Pro Ser Pro Asp Thr Leu Pro
                965                 970                 975

Thr Ile Thr Leu Pro Ala Ser Thr Val Gly Ser Glu Ser Ser Leu Ala
            980                 985                 990

Leu Arg Leu Val Asn Gly Gly Asp  Arg Cys Gln Gly Arg  Val Glu Val
        995                 1000                 1005

Leu Tyr  Gln Gly Ser Trp Gly  Thr Val Cys Asp Asp  Ser Trp Asp
    1010                 1015                 1020

Thr Asn  Asp Ala Asn Val Val  Cys Arg Gln Leu Gly  Cys Gly Trp
    1025                 1030                 1035

Ala Met  Ser Ala Pro Gly Asn  Ala Arg Phe Gly Gln  Gly Ser Gly
    1040                 1045                 1050

Pro Ile  Val Leu Asp Asp Val  Arg Cys Ser Gly His  Glu Ser Tyr
    1055                 1060                 1065

Leu Trp  Ser Cys Pro His Asn  Gly Trp Leu Ser His  Asn Cys Gly
    1070                 1075                 1080

His Ser  Glu Asp Ala Gly Val  Ile Cys Ser Ala Ser  Gln Ser Arg
    1085                 1090                 1095

Pro Thr  Pro Ser Pro Asp Thr  Trp Pro Thr Ser His  Ala Ser Thr
    1100                 1105                 1110

Ala Gly  Ser Glu Ser Ser Leu  Ala Leu Arg Leu Val  Asn Gly Gly
    1115                 1120                 1125

Asp Arg  Cys Gln Gly Arg Val  Glu Val Leu Tyr Arg  Gly Ser Trp
    1130                 1135                 1140

Gly Thr  Val Cys Asp Asp Tyr  Trp Asp Thr Asn Asp  Ala Asn Val
    1145                 1150                 1155

Val Cys  Arg Gln Leu Gly Cys  Gly Trp Ala Met Ser  Ala Pro Gly
    1160                 1165                 1170

Asn Ala  Arg Phe Gly Gln Gly  Ser Gly Pro Ile Val  Leu Asp Asp
    1175                 1180                 1185

Val Arg Cys Ser Gly His Glu Ser Tyr Leu Trp Ser Cys Pro His
    1190                1195                1200

Asn Gly Trp Leu Ser His Asn Cys Gly His His Glu Asp Ala Gly
    1205                1210                1215

Val Ile Cys Ser Ala Ser Gln Ser Gln Pro Thr Pro Ser Pro Asp
    1220                1225                1230

Thr Trp Pro Thr Ser His Ala Ser Thr Ala Gly Ser Glu Ser Ser
    1235                1240                1245

Leu Ala Leu Arg Leu Val Asn Gly Gly Asp Arg Cys Gln Gly Arg
    1250                1255                1260

Val Glu Val Leu Tyr Arg Gly Ser Trp Gly Thr Val Cys Asp Asp
    1265                1270                1275

Tyr Trp Asp Thr Asn Asp Ala Asn Val Val Cys Arg Gln Leu Gly
    1280                1285                1290

Cys Ser Trp Ala Thr Ser Ala Pro Gly Asn Ala Arg Phe Gly Gln
    1295                1300                1305

Gly Ser Gly Pro Ile Val Leu Asp Asp Val Arg Cys Ser Gly His
    1310                1315                1320

Glu Ser Tyr Leu Trp Ser Cys Pro His Asn Gly Trp Phe Ser His
    1325                1330                1335

Asn Cys Gly His His Glu Asp Ala Gly Val Ile Cys Ser Ala Ser
    1340                1345                1350

Gln Ser Gln Pro Thr Pro Ser Pro Asp Thr Trp Pro Thr Ser His
    1355                1360                1365

Ala Ser Thr Ala Gly Ser Glu Ser Ser Leu Ala Leu Arg Leu Val
    1370                1375                1380

Asn Gly Gly Asp Arg Cys Gln Gly Arg Val Glu Val Leu Tyr Arg
    1385                1390                1395

Gly Ser Trp Gly Thr Val Cys Asp Asp Tyr Trp Asp Thr Asn Asp
    1400                1405                1410

Ala Asn Val Val Cys Arg Gln Leu Gly Cys Gly Trp Ala Thr Ser

1415                1420                1425

Ala Pro Gly Asn Ala Arg Phe Gly Gln Gly Ser Gly Pro Ile Val
    1430                1435                1440

Leu Asp Asp Val Arg Cys Ser Gly His Glu Ser Tyr Leu Trp Ser
    1445                1450                1455

Cys Pro His Asn Gly Trp Leu Ser His Asn Cys Gly His His Glu
    1460                1465                1470

Asp Ala Gly Val Ile Cys Ser Ala Ser Gln Ser Gln Pro Thr Pro
    1475                1480                1485

Ser Pro Asp Thr Trp Pro Thr Ser Arg Ala Ser Thr Ala Gly Ser
    1490                1495                1500

Glu Ser Thr Leu Ala Leu Arg Leu Val Asn Gly Gly Asp Arg Cys
    1505                1510                1515

Arg Gly Arg Val Glu Val Leu Tyr Gln Gly Ser Trp Gly Thr Val
    1520                1525                1530

Cys Asp Asp Tyr Trp Asp Thr Asn Asp Ala Asn Val Val Cys Arg
    1535                1540                1545

Gln Leu Gly Cys Gly Trp Ala Met Ser Ala Pro Gly Asn Ala Gln
    1550                1555                1560

Phe Gly Gln Gly Ser Gly Pro Ile Val Leu Asp Asp Val Arg Cys
    1565                1570                1575

Ser Gly His Glu Ser Tyr Leu Trp Ser Cys Pro His Asn Gly Trp
    1580                1585                1590

Leu Ser His Asn Cys Gly His His Glu Asp Ala Gly Val Ile Cys
    1595                1600                1605

Ser Ala Ala Gln Ser Gln Ser Thr Pro Arg Pro Asp Thr Trp Leu
    1610                1615                1620

Thr Thr Asn Leu Pro Ala Leu Thr Val Gly Ser Glu Ser Ser Leu
    1625                1630                1635

Ala Leu Arg Leu Val Asn Gly Gly Asp Arg Cys Arg Gly Arg Val
    1640                1645                1650

Glu Val Leu Tyr Arg Gly Ser Trp Gly Thr Val Cys Asp Asp Ser
    1655                1660                1665

Trp Asp Thr Asn Asp Ala Asn Val Val Cys Arg Gln Leu Gly Cys
    1670                1675                1680

Gly Trp Ala Met Ser Ala Pro Gly Asn Ala Arg Phe Gly Gln Gly
    1685                1690                1695

Ser Gly Pro Ile Val Leu Asp Asp Val Arg Cys Ser Gly Asn Glu
    1700                1705                1710

Ser Tyr Leu Trp Ser Cys Pro His Lys Gly Trp Leu Thr His Asn
    1715                1720                1725

Cys Gly His His Glu Asp Ala Gly Val Ile Cys Ser Ala Thr Gln
    1730                1735                1740

Ile Asn Ser Thr Thr Thr Asp Trp Trp His Pro Thr Thr Thr Thr
    1745                1750                1755

Thr Ala Arg Pro Ser Ser Asn Cys Gly Gly Phe Leu Phe Tyr Ala
    1760                1765                1770

Ser Gly Thr Phe Ser Ser Pro Ser Tyr Pro Ala Tyr Tyr Pro Asn
    1775                1780                1785

Asn Ala Lys Cys Val Trp Glu Ile Glu Val Asn Ser Gly Tyr Arg
    1790                1795                1800

Ile Asn Leu Gly Phe Ser Asn Leu Lys Leu Glu Ala His His Asn
    1805                1810                1815

Cys Ser Phe Asp Tyr Val Glu Ile Phe Asp Gly Ser Leu Asn Ser
    1820                1825                1830

Ser Leu Leu Leu Gly Lys Ile Cys Asn Asp Thr Arg Gln Ile Phe
    1835                1840                1845

Thr Ser Ser Tyr Asn Arg Met Thr Ile His Phe Arg Ser Asp Ile
    1850                1855                1860

Ser Phe Gln Asn Thr Gly Phe Leu Ala Trp Tyr Asn Ser Phe Pro
    1865                1870                1875

Ser Asp Ala Thr Leu Arg Leu Val Asn Leu Asn Ser Ser Tyr Gly
    1880                1885                1890

Leu Cys Ala Gly Arg Val Glu Ile Tyr His Gly Gly Thr Trp Gly
    1895                1900                1905

Thr Val Cys Asp Asp Ser Trp Thr Ile Gln Glu Ala Glu Val Val
    1910                1915                1920

Cys Arg Gln Leu Gly Cys Gly Arg Ala Val Ser Ala Leu Gly Asn
    1925                1930                1935

Ala Tyr Phe Gly Ser Gly Ser Gly Pro Ile Thr Leu Asp Asp Val
    1940                1945                1950

Glu Cys Ser Gly Thr Glu Ser Thr Leu Trp Gln Cys Arg Asn Arg
    1955                1960                1965

Gly Trp Phe Ser His Asn Cys Asn His Arg Glu Asp Ala Gly Val
    1970                1975                1980

Ile Cys Ser Gly Asn His Leu Ser Thr Pro Ala Pro Phe Leu Asn
    1985                1990                1995

Ile Thr Arg Pro Asn Thr Asp Tyr Ser Cys Gly Gly Phe Leu Ser
    2000                2005                2010

Gln Pro Ser Gly Asp Phe Ser Ser Pro Phe Tyr Pro Gly Asn Tyr
    2015                2020                2025

Pro Asn Asn Ala Lys Cys Val Trp Asp Ile Glu Val Gln Asn Asn
    2030                2035                2040

Tyr Arg Val Thr Val Ile Phe Arg Asp Val Gln Leu Glu Gly Gly
    2045                2050                2055

Cys Asn Tyr Asp Tyr Ile Glu Val Phe Asp Gly Pro Tyr Arg Ser
    2060                2065                2070

Ser Pro Leu Ile Ala Arg Val Cys Asp Gly Ala Arg Gly Ser Phe
    2075                2080                2085

Thr Ser Ser Ser Asn Phe Met Ser Ile Arg Phe Ile Ser Asp His
    2090                2095                2100

Ser Ile Thr Arg Arg Gly Phe Arg Ala Glu Tyr Tyr Ser Ser Pro
    2105                2110                2115

Ser Asn Asp Ser Thr Asn Leu Leu Cys Leu Pro Asn His Met Gln
    2120                2125                2130

Ala Ser Val Ser Arg Ser Tyr Leu Gln Ser Leu Gly Phe Ser Ala
    2135                2140                2145

Ser Asp Leu Val Ile Ser Thr Trp Asn Gly Tyr Tyr Glu Cys Arg
    2150                2155                2160

Pro Gln Ile Thr Pro Asn Leu Val Ile Phe Thr Ile Pro Tyr Ser
    2165                2170                2175

Gly Cys Gly Thr Phe Lys Gln Ala Asp Asn Asp Thr Ile Asp Tyr
    2180                2185                2190

Ser Asn Phe Leu Thr Ala Ala Val Ser Gly Gly Ile Ile Lys Arg
    2195                2200                2205

Arg Thr Asp Leu Arg Ile His Val Ser Cys Arg Met Leu Gln Asn
    2210                2215                2220

Thr Trp Val Asp Thr Met Tyr Ile Ala Asn Asp Thr Ile His Val
    2225                2230                2235

Ala Asn Asn Thr Ile Gln Val Glu Glu Val Gln Tyr Gly Asn Phe
    2240                2245                2250

Asp Val Asn Ile Ser Phe Tyr Thr Ser Ser Ser Phe Leu Tyr Pro
    2255                2260                2265

Val Thr Ser Arg Pro Tyr Tyr Val Asp Leu Asn Gln Asp Leu Tyr
    2270                2275                2280

Val Gln Ala Glu Ile Leu His Ser Asp Ala Val Leu Thr Leu Phe
    2285                2290                2295

Val Asp Thr Cys Val Ala Ser Pro Tyr Ser Asn Asp Phe Thr Ser
    2300                2305                2310

Leu Thr Tyr Asp Leu Ile Arg Ser Gly Cys Val Arg Asp Asp Thr
    2315                2320                2325

Tyr Gly Pro Tyr Ser Ser Pro Ser Leu Arg Ile Ala Arg Phe Arg
    2330                2335                2340

Phe Arg Ala Phe His Phe Leu Asn Arg Phe Pro Ser Val Tyr Leu
    2345                2350                2355

Arg Cys Lys Met Val Val Cys Arg Ala Tyr Asp Pro Ser Ser Arg

2360                    2365                    2370

Cys Tyr Arg Gly Cys Val Leu Arg Ser Lys Arg Asp Val Gly Ser
    2375                    2380                    2385

Tyr Gln Glu Lys Val Asp Val Val Leu Gly Pro Ile Gln Leu Gln
    2390                    2395                    2400

Thr Pro Pro Arg Arg Glu Glu Glu Pro Arg
    2405                    2410

<210>  5
<211>  9
<212>  PRT
<213>  Artificial sequence

<220>
<223>  synthetic peptide

<220>
<221>  misc_feature
<222>  (5)..(8)
<223>  Xaa can be any naturally occurring amino acid

<400>  5

Val Glu Val Leu Xaa Xaa Xaa Xaa Trp
1               5

<210>  6
<211>  9
<212>  PRT
<213>  Artificial sequence

<220>
<223>  synthetic peptide

<220>
<221>  misc_feature
<222>  (5)..(8)
<223>  Xaa can be any naturally occurring amino acid

<400>  6

Val Glu Ile Leu Xaa Xaa Xaa Xaa Trp
1               5

<210>  7
<211>  9
<212>  PRT
<213>  Artificial Sequence

<220>
<223>  synthetic peptide

<220>
<221>  misc_feature
<222>  (5)..(8)
<223>  Xaa can be any naturally occurring amino acid

<400>  7

Val Glu Ile Tyr Xaa Xaa Xaa Xaa Trp
1               5


<210>  8
<211>  9
<212>  PRT
<213>  Artificial Sequence

<220>
<223>  synthetic peptide


<220>
<221>  misc_feature
<222>  (5)..(8)
<223>  Xaa can be any naturally occurring amino acid

<400>  8

Val Glu Val Tyr Xaa Xaa Xaa Xaa Trp
1               5


<210>  9
<211>  5
<212>  PRT
<213>  Artificial Sequence

<220>
<223>  synthetic peptide

<400>  9

Arg Cys Gln Gly Arg
1               5


<210>  10
<211>  4
<212>  PRT
<213>  Artificial sequence

<220>
<223>  synthetic peptide

<400>  10

Tyr Arg Gly Ser
1


<210>  11
<211>  100
<212>  PRT

<213>  Homo sapiens

<400>  11

Arg Leu Val Asn Gly Ser Asp Arg Cys Gln Gly Arg Val Glu Val Leu
1               5                   10                  15

Tyr Arg Gly Ser Trp Gly Thr Val Cys Asp Asp Ser Trp Asp Thr Asn
            20                  25                  30

Asp Ala Asn Val Val Cys Arg Gln Leu Gly Cys Gly Trp Ala Thr Ser
        35                  40                  45

Ala Pro Gly Asn Ala Arg Phe Gly Gln Gly Ser Gly Pro Ile Val Leu
    50                  55                  60

Asp Asp Val Arg Cys Ser Gly His Glu Ser Tyr Leu Trp Ser Cys Pro
65                  70                  75                  80

His Asn Gly Trp Leu Ser His Asn Cys Gly His His Glu Asp Ala Gly
                85                  90                  95

Val Ile Cys Ser
            100

<210>  12
<211>  18
<212>  PRT
<213>  Homo sapiens

<400>  12

Arg Cys Gln Gly Arg Val Glu Val Leu Tyr Arg Gly Ser Trp Gly Thr
1               5                   10                  15

Val Cys

<210>  13
<211>  600
<212>  PRT
<213>  Bos taurus

<400>  13

Ala Leu Arg Leu Val Asn Gly Ser Asp Arg Cys Gln Gly Arg Val Glu
1               5                   10                  15

Val Leu Tyr Gly Gly Ser Trp Gly Thr Val Cys Asp Asp Ser Trp Asp
            20                  25                  30

Thr Asn Asp Ala Asn Val Val Cys Arg Gln Leu Gly Cys Gly Trp Ala

35                  40                  45

Ile Ser Ala Pro Gly Asp Ala Arg Phe Gly Gln Gly Ser Gly Pro Ile
    50                  55                  60

Val Leu Asp Asp Val Gly Cys Ser Gly Tyr Glu Thr Tyr Leu Trp Ser
65                  70                  75                  80

Cys Ser His Ser Pro Trp Asn Thr His Asn Cys Gly His Ser Glu Asp
                85                  90                  95

Ala Ser Val Ile Cys Ser Ala Ser Gln Thr Gln Ser Thr Val Val Pro
            100                 105                 110

Asp Trp Leu Tyr Pro Thr Thr Asp Tyr Gly Thr Glu Ser Gly Leu Ala
        115                 120                 125

Leu Arg Leu Val Asn Gly Gly Asp Arg Cys Gln Gly Arg Val Glu Val
    130                 135                 140

Leu Tyr Arg Gly Ser Trp Gly Thr Val Cys Asp Asp Ser Trp Asp Thr
145                 150                 155                 160

Asn Asp Ser Asn Val Val Cys Arg Gln Leu Gly Cys Gly Trp Ala Ile
                165                 170                 175

Ser Ala Pro Gly Asn Ala Arg Phe Gly Gln Gly Ser Gly Pro Ile Val
            180                 185                 190

Leu Asp Asp Val Gly Cys Ser Gly Tyr Glu Thr Tyr Leu Trp Ser Cys
        195                 200                 205

Ser His Asn Pro Trp Asn Thr His Asn Cys Gly His Ser Glu Asp Ala
    210                 215                 220

Ser Val Ile Cys Ser Ala Ser Gln Thr Gln Ser Thr Val Val Pro Asp
225                 230                 235                 240

Leu Trp Tyr Pro Thr Thr Asp Tyr Gly Thr Glu Ser Gly Leu Ala Leu
                245                 250                 255

Arg Leu Val Asn Gly Ser Asp Arg Cys Gln Gly Arg Val Glu Val Leu
            260                 265                 270

Tyr Arg Gly Ser Trp Gly Thr Val Cys Asp Asp Ser Trp Asp Thr Asn
        275                 280                 285

Asp Ala Asn Val Val Cys Arg Gln Leu Gly Cys Gly Trp Gly Ile Ser
    290                 295                 300

Ala Pro Gly Asp Ala Arg Phe Gly Gln Gly Ser Gly Pro Ile Val Leu
305                 310                 315                 320

Asp Asp Val Gly Cys Ser Gly Tyr Glu Thr Tyr Leu Trp Ser Cys Ser
                325                 330                 335

His Asn Pro Trp Asn Thr His Asn Cys Gly His Ser Glu Asp Ala Ser
            340                 345                 350

Val Ile Cys Ser Ala Ser Gln Thr Gln Ser Thr Val Val Pro Asp Leu
        355                 360                 365

Trp Tyr Pro Thr Thr Asp Tyr Gly Thr Glu Ser Gly Leu Ala Leu Arg
    370                 375                 380

Leu Val Asn Gly Ser Asp Arg Cys Gln Gly Arg Val Glu Val Leu Tyr
385                 390                 395                 400

Gly Gly Ser Trp Gly Thr Val Cys Asp Asp Ser Trp Asp Thr Asn Asp
                405                 410                 415

Ala Asn Val Val Cys Arg Gln Leu Gly Cys Gly Ser Gly Ile Ser Ala
            420                 425                 430

Pro Gly Asp Ala Arg Phe Gly Gln Gly Ser Gly Pro Ile Leu Leu Asp
        435                 440                 445

Asp Val Gly Cys Ser Gly Tyr Glu Thr Tyr Leu Trp Ser Cys Ser His
    450                 455                 460

Ser Pro Trp Asn Ser His Asn Cys Gly His Ser Lys Asp Ala Ser Val
465                 470                 475                 480

Ile Cys Ser Ala Ala Gln Ile Asn Ser Ser Thr Pro Gly Trp Gln Pro
                485                 490                 495

Pro Gln Thr Thr Thr Thr Gln Thr Pro Gly Val Asn Phe Ser Thr Pro
            500                 505                 510

Asp Trp Leu Ser Pro Thr Thr Thr Pro Thr Gln Thr Pro Gly Val Asn
        515                 520                 525

Phe Ser Thr Pro Asp Trp Leu Ser Pro Thr Thr Thr Pro Thr Gln Thr
    530                 535                 540

Pro Gly Val Asn Phe Ser Thr Pro Asp Trp Leu Ser Pro Thr Thr Thr
545                 550                 555                 560

Pro Thr Gln Thr Pro Gly Val Asn Phe Ser Thr Pro Asp Trp Leu Ser
                565                 570                 575

Pro Thr Thr Thr Pro Thr Gln Thr Pro Gly Val Asn Phe Ser Thr Pro
            580                 585                 590

Asp Trp Leu Ser Pro Thr Thr Thr
        595                 600

<210>  14
<211>  2085
<212>  PRT
<213>  Mus musculus

<400>  14

Met Gly Ile Ser Thr Val Ile Phe Glu Ile Cys Leu Leu Trp Gly Gln
1               5                   10                  15

Ile Leu Ser Thr Ala Ser Gln Thr Ala Val Pro Thr Asp Gly Thr Asp
            20                  25                  30

Ser Gly Leu Ala Val Arg Leu Val Asn Gly Gly Asp Arg Cys Gln Gly
        35                  40                  45

Arg Val Glu Ile Leu Tyr Gln Gly Ser Trp Gly Thr Val Cys Asp Asp
    50                  55                  60

Ser Trp Asp Leu Asn Asp Ala Asn Val Val Cys Arg Gln Leu Gly Cys
65                  70                  75                  80

Gly Leu Ala Val Ser Ala Pro Gly Asn Ala Arg Phe Gly Gln Gly Ser
                85                  90                  95

Gly Pro Ile Val Met Asp Asp Val Ala Cys Gly Gly Tyr Glu Asp Tyr
            100                 105                 110

Leu Trp Arg Cys Ser His Arg Gly Trp Leu Ser His Asn Cys Gly His
        115                 120                 125

Gln Glu Asp Ala Gly Val Ile Cys Ser Asp Ser Gln Thr Ser Ser Pro
    130                 135                 140

Thr Pro Gly Trp Trp Asn Pro Gly Gly Thr Asn Asn Asp Val Phe Tyr
145                 150                 155                 160

Pro Thr Glu Gln Thr Thr Ala Glu Gln Thr Thr Ile Pro Asp Tyr Thr
                165                 170                 175

Pro Ile Gly Thr Asp Ser Gly Leu Ala Val Arg Leu Val Asn Gly Gly
            180                 185                 190

Asp Arg Cys Gln Gly Arg Val Glu Ile Leu Tyr Gln Gly Ser Trp Gly
        195                 200                 205

Thr Val Cys Asp Asp Ser Trp Asp Val Ser Asp Ala Asn Val Val Cys
    210                 215                 220

Arg Gln Leu Gly Cys Gly Trp Ala Val Ser Ala Pro Gly Asn Ala Tyr
225                 230                 235                 240

Phe Gly Gln Gly Gln Gly Pro Ile Val Leu Asp Asp Val Ala Cys Gly
                245                 250                 255

Gly Tyr Glu Asn Tyr Leu Trp Ser Cys Ser His Gln Gly Trp Leu Ser
            260                 265                 270

His Asn Cys Gly His Gln Glu Asp Ala Gly Val Ile Cys Ser Ala Ser
        275                 280                 285

Gln Ser Ser Ser Pro Thr Pro Gly Trp Trp Asn Pro Gly Gly Thr Asn
    290                 295                 300

Asn Asp Val Phe Tyr Pro Thr Glu Gln Thr Thr Ala Gly Thr Asp Ser
305                 310                 315                 320

Gly Leu Ala Val Arg Leu Val Asn Gly Gly Asp Arg Cys Gln Gly Arg
                325                 330                 335

Val Glu Ile Leu Tyr Gln Gly Ser Trp Gly Thr Val Cys Asp Asp Ser
            340                 345                 350

Trp Asp Thr Asn Asp Ala Asn Val Val Cys Arg Gln Leu Gly Cys Gly
        355                 360                 365

Trp Ala Val Ser Ala Pro Gly Asn Ala Tyr Phe Gly Pro Gly Ser Gly
    370                 375                 380

Ser Ile Val Leu Asp Asp Val Ala Cys Thr Gly His Glu Asp Tyr Leu
385                 390                 395                 400

Trp Arg Cys Ser His Arg Gly Trp Leu Ser His Asn Cys Gly His His
                405                 410                 415

Glu Asp Ala Gly Val Ile Cys Ser Ala Ser Gln Ser Ser Ser Pro Thr
            420                 425                 430

Pro Asp Val Phe Tyr Pro Thr Asp Gln Thr Thr Ala Glu Gln Thr Thr
        435                 440                 445

Val Pro Asp Tyr Thr Pro Ile Gly Thr Asp Ser Gly Leu Ala Val Arg
    450                 455                 460

Leu Val Asn Gly Gly Asp Arg Cys Gln Gly Arg Val Glu Ile Leu Tyr
465                 470                 475                 480

Gln Gly Ser Trp Gly Thr Val Cys Asp Asp Ser Trp Asp Leu Asn Asp
                485                 490                 495

Ala Asn Val Val Cys Arg Gln Leu Gly Cys Gly Leu Ala Val Ser Ala
            500                 505                 510

Pro Gly Ser Ala Arg Phe Gly Gln Gly Thr Gly Pro Ile Val Met Asp
        515                 520                 525

Asp Val Ala Cys Gly Gly Tyr Glu Asp Tyr Leu Trp Arg Cys Ser His
    530                 535                 540

Arg Gly Trp Leu Ser His Asn Cys Gly His His Glu Asp Ala Gly Val
545                 550                 555                 560

Ile Cys Ser Ala Ser Gln Ser Ser Ser Pro Thr Pro Asp Val Phe Tyr
                565                 570                 575

Pro Thr Asp Gln Thr Thr Ala Glu Gln Thr Thr Val Pro Asp Tyr Thr
            580                 585                 590

Pro Ile Gly Thr Asp Ser Gly Leu Ala Val Arg Leu Val Asn Gly Gly
        595                 600                 605

Asp Arg Cys Gln Gly Arg Val Glu Ile Leu Tyr Gln Gly Ser Trp Gly
    610                 615                 620

Thr Val Cys Asp Asp Ser Trp Asp Leu Asn Asp Ala Asn Val Val Cys
625                 630                 635                 640

Arg Gln Leu Gly Cys Gly Leu Ala Val Ser Ala Pro Gly Ser Ala Arg
                645                 650                 655

Phe Gly Gln Gly Thr Gly Pro Ile Val Met Asp Asp Val Ala Cys Gly

            660                 665                 670

Gly Tyr Glu Asp Tyr Leu Trp Arg Cys Ser His Arg Gly Trp Leu Ser
        675                 680                 685

His Asn Cys Gly His His Glu Asp Ala Gly Val Ile Cys Ser Ala Ser
    690                 695                 700

Gln Ser Ser Ser Pro Thr Pro Asp Val Phe Tyr Pro Thr Asp Gln Thr
705                 710                 715                 720

Thr Ala Glu Gln Thr Thr Val Pro Asp Tyr Thr Thr Ile Gly Thr Glu
                725                 730                 735

Asn Ser Leu Ala Val Arg Leu Glu Asn Gly Gly Asp Arg Cys Gln Gly
            740                 745                 750

Arg Val Glu Ile Leu Tyr Gln Gly Ser Trp Gly Thr Val Cys Asp Asp
        755                 760                 765

Ser Trp Asp Leu Asn Asp Ala Asn Val Val Cys Arg Gln Leu Gly Cys
    770                 775                 780

Gly Leu Ala Val Ser Ala Pro Gly Ser Ala Arg Phe Gly Gln Gly Thr
785                 790                 795                 800

Gly Pro Ile Val Met Asp Asp Val Ala Cys Gly Gly Tyr Glu Asp Tyr
                805                 810                 815

Leu Trp Arg Cys Ser His Arg Gly Trp Leu Ser His Asn Cys Gly His
            820                 825                 830

His Glu Asp Ala Gly Val Ile Cys Ser Ala Ser Gln Ser Ser Ser Pro
        835                 840                 845

Thr Pro Asp Val Phe Tyr Pro Thr Asp Gln Thr Thr Val Glu Gln Thr
    850                 855                 860

Thr Val Pro Asp Tyr Thr Pro Ile Gly Thr Glu Asn Ser Leu Ala Val
865                 870                 875                 880

Arg Leu Glu Asn Gly Gly Asp Arg Cys Gln Gly Arg Val Glu Ile Leu
                885                 890                 895

Tyr Gln Gly Ser Trp Gly Thr Val Cys Asp Asp Ser Trp Asp Thr Lys
            900                 905                 910

Asp Ala Asn Val Val Cys Arg Gln Leu Gly Cys Gly Trp Ala Val Ser
        915                 920                 925

Ala Pro Gly Asn Ala Tyr Phe Gly Pro Gly Ser Gly Ser Ile Val Leu
    930                 935                 940

Asp Asp Val Ala Cys Thr Gly His Glu Asp Tyr Leu Trp Ser Cys Ser
945                 950                 955                 960

His Arg Gly Trp Leu Ser His Asn Cys Gly His His Glu Asp Ala Gly
                965                 970                 975

Val Ile Cys Ser Asp Ala Gln Ile Gln Ser Thr Thr Arg Pro Asp Leu
            980                 985                 990

Trp Pro Thr Thr Thr Thr Pro Glu Thr Thr Thr Glu Leu Leu Thr Thr
        995                 1000                1005

Thr Pro Tyr Phe Asp Trp Trp Thr Thr Thr Ser Asp Tyr Ser Cys
    1010                1015                1020

Gly Gly Leu Leu Thr Gln Pro Ser Gly Gln Phe Ser Ser Pro Tyr
    1025                1030                1035

Tyr Pro Ser Asn Tyr Pro Asn Asn Ala Arg Cys Ser Trp Lys Ile
    1040                1045                1050

Val Leu Pro Asn Met Asn Arg Val Thr Val Val Phe Thr Asp Val
    1055                1060                1065

Gln Leu Glu Gly Gly Cys Asn Tyr Asp Tyr Ile Leu Val Tyr Asp
    1070                1075                1080

Gly Pro Glu Tyr Asn Ser Ser Leu Ile Ala Arg Val Cys Asp Gly
    1085                1090                1095

Ser Asn Gly Ser Phe Thr Ser Thr Gly Asn Phe Met Ser Val Val
    1100                1105                1110

Phe Ile Thr Asp Gly Ser Val Thr Arg Arg Gly Phe Gln Ala His
    1115                1120                1125

Tyr Tyr Ser Thr Val Ser Thr Asn Tyr Ser Cys Gly Gly Leu Leu
    1130                1135                1140

Thr Gln Pro Ser Gly Gln Phe Ser Ser Pro Tyr Tyr Pro Ser Asn
    1145                1150                1155

Tyr Pro Asn Asn Ala Arg Cys Ser Trp Glu Ile Leu Val Pro Asn
    1160                1165                1170

Met Asn Arg Val Thr Val Val Phe Thr Asp Val Gln Leu Glu Gly
    1175                1180                1185

Gly Cys Asn Tyr Asp Tyr Ile Leu Val Tyr Asp Gly Pro Gln Tyr
    1190                1195                1200

Asn Ser Ser Leu Ile Ala Arg Val Cys Asp Gly Ser Asn Gly Ser
    1205                1210                1215

Phe Thr Ser Thr Gly Asn Phe Met Ser Val Val Phe Ile Thr Asp
    1220                1225                1230

Gly Ser Val Thr Arg Arg Gly Phe Gln Ala His Tyr Tyr Ser Thr
    1235                1240                1245

Val Ser Thr Thr Pro Pro Val Pro Ile Pro Thr Thr Asp Asp Tyr
    1250                1255                1260

Ser Cys Gly Gly Leu Leu Thr Leu Pro Ser Gly Gln Phe Ser Ser
    1265                1270                1275

Pro His Tyr Pro Ser Asn Tyr Pro Asn Asn Ala Arg Cys Ser Trp
    1280                1285                1290

Glu Ile Leu Val Pro Asn Met Asn Arg Val Thr Val Ala Phe Thr
    1295                1300                1305

Asp Val Gln Leu Glu Gly Gly Cys Asn Tyr Asp Tyr Ile Leu Val
    1310                1315                1320

Tyr Asp Gly Pro Glu Tyr Asn Ser Ser Leu Ile Ala Arg Val Cys
    1325                1330                1335

Asp Gly Ser Asn Gly Ser Phe Thr Ser Thr Gly Asn Phe Met Ser
    1340                1345                1350

Val Val Phe Ile Thr Asp Gly Ser Val Thr Arg Arg Gly Phe Gln
    1355                1360                1365

Ala His Tyr Tyr Ser Thr Val Ser Thr Asn Tyr Ser Cys Gly Gly
    1370                1375                1380

Leu Leu Thr Gln Pro Ser Gly Gln Phe Ser Ser Pro His Tyr Pro
    1385                1390                1395

Ser Asn Tyr Pro Asn Asn Val Arg Cys Ser Trp Glu Ile Leu Val
    1400                1405                1410

Pro Ser Met Asn Arg Val Thr Val Ala Phe Thr Asp Val Gln Leu
    1415                1420                1425

Glu Gly Gly Cys Ser Phe Asp Tyr Ile Leu Val Tyr Asp Gly Pro
    1430                1435                1440

Glu Tyr Asn Ser Ser Leu Ile Ala Pro Val Cys Asp Gly Phe Asn
    1445                1450                1455

Gly Ser Phe Thr Ser Thr Gly Asn Phe Met Ser Val Val Phe Ile
    1460                1465                1470

Thr Asp Gly Ser Val Thr Arg Arg Gly Phe Gln Ala Tyr Tyr Tyr
    1475                1480                1485

Ser Thr Val Ser Thr Pro Pro Ser Phe His Pro Asn Ile Thr Gly
    1490                1495                1500

Asn Asp Ser Ser Leu Ala Leu Arg Leu Val Asn Gly Ser Asn Arg
    1505                1510                1515

Cys Glu Gly Arg Val Glu Ile Leu Tyr Arg Gly Ser Trp Gly Thr
    1520                1525                1530

Val Cys Asp Asp Ser Trp Gly Ile Ser Asp Ala Asn Val Val Cys
    1535                1540                1545

Arg Gln Leu Gly Cys Gly Ser Ala Leu Ser Ala Pro Gly Asn Ala
    1550                1555                1560

Trp Phe Gly Gln Gly Ser Gly Leu Ile Val Leu Asp Asp Val Ser
    1565                1570                1575

Cys Ser Gly Tyr Glu Ser His Leu Trp Asn Cys His His Pro Gly
    1580                1585                1590

Trp Leu Val His Asn Cys Arg His Ser Glu Asp Ala Gly Val Ile
    1595                1600                1605

Cys Ala Leu Pro Glu Val Thr Ser Pro Ser Pro Gly Trp Trp Thr
    1610                1615                1620

Thr Ser Pro Ser Tyr Val Asn Tyr Thr Cys Gly Gly Phe Leu Thr

1625                1630                1635

Gln Pro Ser Gly Gln Phe Ser Ser Pro Phe Tyr Pro Gly Asn Tyr
    1640                1645                1650

Pro Asn Asn Ala Arg Cys Leu Trp Asn Ile Glu Val Pro Asn Asn
    1655                1660                1665

Tyr Arg Val Thr Val Val Phe Arg Asp Leu Gln Leu Glu Arg Gly
    1670                1675                1680

Cys Ser Tyr Asp Tyr Ile Glu Ile Phe Asp Gly Pro His His Ser
    1685                1690                1695

Ser Pro Leu Ile Ala Arg Val Cys Asp Gly Ser Leu Gly Ser Phe
    1700                1705                1710

Thr Ser Thr Ser Asn Phe Met Ser Ile Arg Phe Ile Thr Asp His
    1715                1720                1725

Ser Ile Thr Ala Arg Gly Phe Gln Ala His Tyr Tyr Ser Asp Phe
    1730                1735                1740

Asp Asn Asn Thr Thr Asn Leu Leu Cys Gln Ser Asn His Met Gln
    1745                1750                1755

Ala Ser Val Ser Arg Ser Tyr Leu Gln Ser Met Gly Tyr Ser Ala
    1760                1765                1770

Arg Asp Leu Val Ile Pro Gly Trp Asn Ser Ser Tyr His Cys Gln
    1775                1780                1785

Pro Gln Ile Thr Gln Arg Glu Val Ile Phe Thr Ile Pro Tyr Thr
    1790                1795                1800

Gly Cys Gly Thr Ile Lys Gln Ala Asp Asn Glu Thr Ile Asn Tyr
    1805                1810                1815

Ser Asn Phe Leu Arg Ala Val Val Ser Asn Gly Ile Ile Lys Arg
    1820                1825                1830

Arg Lys Asp Leu Asn Ile His Val Ser Cys Lys Met Leu Gln Asn
    1835                1840                1845

Thr Trp Val Asn Thr Met Tyr Ile Thr Asn Asn Thr Val Glu Ile
    1850                1855                1860

Gln Glu Val Gln Tyr Gly Asn Phe Asp Val Asn Ile Ser Phe Tyr
    1865                1870                1875

Thr Ser Ser Ser Phe Leu Phe Pro Val Thr Ser Ser Pro Tyr Tyr
    1880                1885                1890

Val Asp Leu Asp Gln Asn Leu Tyr Leu Gln Ala Glu Ile Leu His
    1895                1900                1905

Ser Asp Ala Ser Leu Ala Leu Phe Val Asp Thr Cys Val Ala Ser
    1910                1915                1920

Pro His Pro Asn Asp Phe Ser Ser Leu Thr Tyr Asp Leu Ile Arg
    1925                1930                1935

Ser Gly Cys Val Arg Asp Asp Thr Tyr Gln Ser Tyr Ser Ser Pro
    1940                1945                1950

Ser Pro Arg Val Ser Arg Phe Lys Phe Ser Ser Phe His Phe Leu
    1955                1960                1965

Asn Arg Phe Pro Ser Val Tyr Leu Gln Cys Lys Leu Val Val Cys
    1970                1975                1980

Arg Ala Tyr Asp Thr Ser Ser Arg Cys Tyr Arg Gly Cys Val Val
    1985                1990                1995

Arg Ser Lys Arg Asp Val Gly Ser Tyr Gln Glu Lys Val Asp Val
    2000                2005                2010

Val Leu Gly Pro Ile Gln Leu Gln Ser Pro Ser Lys Glu Lys Arg
    2015                2020                2025

Ser Leu Asp Leu Ala Val Glu Asp Val Lys Lys Pro Ala Ser Ser
    2030                2035                2040

Gln Ala Val Tyr Pro Thr Ala Ala Ile Phe Gly Gly Val Phe Leu
    2045                2050                2055

Ala Met Val Leu Ala Val Ala Ala Phe Thr Leu Gly Arg Arg Thr
    2060                2065                2070

His Ile Asp Arg Gly Gln Pro Pro Ser Thr Lys Leu
    2075                2080                2085

<210>  15
<211>  1418
<212>  PRT

<213>  Rattus norvegicus

<400>  15

Met Gly Ile Ser Ile Val Ile Phe Glu Ile Cys Leu Leu Trp Gly Gln
1               5                   10                  15

Ile Leu Ser Thr Ala Ser Gln Ser Arg Ser Ser Thr Pro Asp Trp Trp
            20                  25                  30

Asn His Gly Gly Thr Ile Asn Asp Val Ile Tyr Asp Thr Gln Glu Thr
        35                  40                  45

Pro Glu Val Thr Thr Thr Gln Val Pro Asp Ser Thr Ser Ile Gly Thr
    50                  55                  60

Asp Ser Gly Leu Ala Val Arg Leu Val Asn Gly Gly Asp Arg Cys Arg
65                  70                  75                  80

Gly Arg Val Glu Ile Leu Tyr Gln Gly Ser Trp Gly Thr Met Cys Asp
                85                  90                  95

Asp Gly Thr Asp Ser Gly Leu Ala Val Arg Leu Val Asn Gly Gly Asp
            100                 105                 110

Arg Cys Arg Gly Arg Val Glu Ile Leu Tyr Gln Gly Ser Trp Gly Thr
        115                 120                 125

Met Cys Asp Asp Ser Trp Asp Ile Asn Asp Ala Asn Val Val Cys Arg
    130                 135                 140

Gln Leu Gly Cys Gly Trp Ala Leu Ser Ala Pro Gly Ser Ala Gln Phe
145                 150                 155                 160

Gly Gln Gly Leu Gly Pro Ile Val Leu Asp Asp Val Ala Cys Arg Gly
                165                 170                 175

His Glu Ala Tyr Leu Trp Ser Cys Ser His Arg Gly Trp Leu Ser His
            180                 185                 190

Asn Cys Gly His Gln Glu Asp Ala Gly Val Ile Cys Ser Asp Ser Gln
        195                 200                 205

Thr Ser Ser Pro Thr Pro Gly Trp Trp Asn Pro Gly Gly Thr Asn Asn
    210                 215                 220

Asp Val Ile Tyr Asp Thr Gln Glu Thr Thr Glu Thr Ser Gln Thr Ser
225                 230                 235                 240

Ser Pro Thr Pro Asp Trp Trp Asn His Gly Gly Thr Ile Asn Asp Val
                245                 250                 255

Ile Tyr Asp Thr Gln Glu Thr Thr Glu Gly Thr Asp Ser Gly Leu Ala
            260                 265                 270

Val Arg Leu Val Asn Gly Gly Asp Arg Cys Arg Gly Arg Val Glu Ile
        275                 280                 285

Leu Tyr Gln Gly Ser Trp Gly Thr Val Cys Asp Asp Ser Trp Asp Ile
    290                 295                 300

Asn Asp Ala Asn Val Val Cys Arg Gln Leu Gly Cys Gly Trp Ala Leu
305                 310                 315                 320

Ser Ala Pro Gly Ser Ala Gln Phe Gly Gln Gly Ser Gly Ser Ile Val
                325                 330                 335

Leu Asp Asp Val Ala Cys Arg Gly His Glu Ala Tyr Leu Trp Ser Cys
            340                 345                 350

Ser His Arg Gly Trp Leu Ser His Asn Cys Gly His Gln Glu Asp Ala
        355                 360                 365

Gly Val Ile Cys Ser Tyr Ser Gln Thr Ser Ser Pro Thr Pro Asp Ser
    370                 375                 380

Gln Thr Ser Ser Pro Thr Pro Gly Trp Trp Asn Pro Gly Gly Thr Asn
385                 390                 395                 400

Asn Asp Val Ser Tyr Gly Pro Glu Gln Thr Thr Asp Ala Thr Asp Ser
                405                 410                 415

Gly Leu Ala Val Arg Leu Val Asn Gly Gly Asp Arg Cys Gln Gly Arg
            420                 425                 430

Val Glu Ile Leu Tyr Gln Gly Ser Trp Gly Thr Val Cys Asp Asp Ser
        435                 440                 445

Trp Asp Thr Lys Asp Ala Asn Val Val Cys Arg Gln Leu Val Cys Gly
    450                 455                 460

Trp Ala Leu Ser Ala Pro Gly Ser Ala His Phe Gly Gln Gly Ser Gly
465                 470                 475                 480

Ser Ile Val Leu Asp Asp Val Ala Cys Thr Gly His Glu Ala Tyr Leu
                485                 490                 495

Trp Ser Cys Ser His Arg Gly Trp Leu Ser His Asn Cys Gly His His
            500                 505                 510

Glu Asp Ala Gly Val Ile Cys Ser Asp Ala Gln Thr Gln Ser Thr Thr
        515                 520                 525

Trp Pro Asp Met Trp Pro Thr Thr Thr Pro Glu Thr Thr Thr Asp Trp
    530                 535                 540

Trp Thr Thr Lys Tyr Ser Ser Ser Val Pro Thr Thr Gln Phe Pro Thr
545                 550                 555                 560

Ile Ala Asp Trp Trp Thr Thr Pro Ser Pro Glu Tyr Thr Cys Gly Gly
                565                 570                 575

Leu Leu Thr Leu Pro Tyr Gly Gln Phe Ser Ser Pro Tyr Tyr Pro Gly
            580                 585                 590

Ser Tyr Pro Asn Asn Ala Arg Cys Leu Trp Lys Ile Phe Val Ser Ser
        595                 600                 605

Met Asn Arg Val Thr Val Val Phe Thr Asp Val Gln Leu Glu Gly Gly
    610                 615                 620

Cys Asn Tyr Asp Tyr Ile Leu Val Phe Asp Gly Pro Glu Asn Asn Ser
625                 630                 635                 640

Ser Leu Ile Ala Arg Val Cys Asp Gly Phe Asn Gly Ser Phe Thr Ser
                645                 650                 655

Thr Gln Asn Phe Met Ser Val Val Phe Ile Thr Asp Gly Ser Val Thr
            660                 665                 670

Arg Arg Gly Phe Gln Ala Asp Tyr Tyr Ser Thr Pro Ile Ser Thr Ser
        675                 680                 685

Thr Thr Ser Pro Thr Thr Phe Pro Ile Val Thr Asp Trp Trp Thr Thr
    690                 695                 700

Pro Ser Pro Glu Tyr Thr Cys Gly Gly Leu Leu Thr Leu Pro Tyr Gly
705                 710                 715                 720

Gln Phe Ser Ser Pro Tyr Tyr Pro Gly Ser Tyr Pro Asn Asn Ala Arg
                725                 730                 735

Cys Leu Trp Lys Ile Phe Val Pro Ser Met Asn Arg Val Thr Val Val

740                 745                 750

Phe Thr Asp Val Gln Leu Glu Gly Gly Cys Asn Tyr Asp Tyr Ile Leu
        755                 760                 765

Gly Phe Asp Gly Pro Glu Tyr Asn Ser Ser Leu Ile Ala Arg Val Cys
    770                 775                 780

Asp Gly Ser Asn Gly Ser Phe Thr Ser Thr Gln Asn Phe Met Ser Val
785                 790                 795                 800

Val Phe Ile Thr Asp Gly Ser Val Thr Arg Arg Gly Phe Gln Ala Asp
                805                 810                 815

Tyr Tyr Ser Thr Pro Ile Arg Thr Ser Thr Thr Pro Pro Thr Thr Phe
            820                 825                 830

Pro Ile Ile Thr Gly Asn Asp Ser Ser Leu Val Leu Arg Leu Val Asn
        835                 840                 845

Gly Thr Asn Arg Cys Glu Gly Arg Val Glu Ile Leu Tyr Arg Gly Ser
    850                 855                 860

Trp Val Pro Cys Ala Asp Asp Ser Trp Asp Ile Asn Asp Ala Asn Val
865                 870                 875                 880

Val Cys Arg Gln Leu Gly Cys Gly Ser Ala Leu Ser Ala Pro Gly Asn
                885                 890                 895

Ala Trp Phe Gly Gln Gly Ser Gly Leu Ile Val Leu Asp Asp Val Ser
            900                 905                 910

Cys Ser Gly Tyr Glu Ser His Leu Trp Asn Cys Arg His Pro Gly Trp
        915                 920                 925

Leu Val His Asn Cys Arg His Val Glu Asp Ala Gly Val Ile Cys Ser
    930                 935                 940

Leu Pro Asp Pro Thr Pro Ser Pro Gly Pro Val Trp Thr Ser Pro Pro
945                 950                 955                 960

Phe Val Asn Tyr Thr Cys Gly Gly Phe Leu Thr Gly Leu Ser Gly Gln
                965                 970                 975

Phe Ser Ser Pro Tyr Tyr Pro Gly Ser Tyr Pro Asn Asn Ala Arg Cys
            980                 985                 990

Leu Trp Asn Ile Glu Val Pro Asn Asn Tyr Arg Val Thr Val Val Phe
        995                 1000                1005

Arg Asp Val Gln Leu Glu Gly Gly Cys Asn Tyr Asp Tyr Ile Glu
    1010                1015                1020

Ile Phe Asp Gly Pro His His Ser Ser Pro Leu Ile Ala Arg Val
    1025                1030                1035

Cys Asp Gly Ala Met Gly Ser Phe Thr Ser Thr Ser Asn Phe Met
    1040                1045                1050

Ser Val Arg Phe Thr Thr Asp His Ser Val Thr Arg Arg Gly Phe
    1055                1060                1065

Arg Ala Asp Tyr Tyr Ser Asp Phe Asp Asn Asn Thr Thr Asn Leu
    1070                1075                1080

Leu Cys Leu Ser Asn His Met Arg Ala Ser Val Ser Arg Ser Tyr
    1085                1090                1095

Leu Gln Ser Met Gly Tyr Ser Ser Arg Asp Leu Val Ile Pro Gly
    1100                1105                1110

Trp Asn Val Ser Tyr Gln Cys Gln Pro Gln Ile Thr Gln Arg Glu
    1115                1120                1125

Val Ile Phe Thr Ile Pro Tyr Thr Gly Cys Gly Thr Thr Lys Gln
    1130                1135                1140

Ala Asp Asn Glu Thr Ile Asn Tyr Ser Asn Phe Leu Lys Ala Ala
    1145                1150                1155

Val Ser Asn Gly Ile Ile Lys Arg Arg Lys Asp Leu His Ile His
    1160                1165                1170

Val Ser Cys Lys Met Leu Gln Asn Thr Trp Val Asn Thr Met Tyr
    1175                1180                1185

Ile Thr Asn Asn Thr Val Glu Ile Gln Glu Val Gln Tyr Gly Asn
    1190                1195                1200

Phe Asp Val Asn Ile Ser Phe Tyr Thr Ser Ser Ser Phe Leu Tyr
    1205                1210                1215

Pro Val Thr Ser Ser Pro Tyr Tyr Val Asp Leu Asp Gln Asn Leu
    1220                1225                1230

Tyr Leu Gln Ala Glu Val Leu His Ser Asp Thr Ser Leu Ala Leu
    1235                1240                1245

Phe Val Asp Thr Cys Val Ala Ser Pro His Pro Asn Asp Phe Ser
    1250                1255                1260

Ser Leu Thr Tyr Asp Leu Ile Arg Ser Gly Cys Ile Arg Asp Glu
    1265                1270                1275

Thr Tyr Gln Ser Tyr Ser Ser Pro Ser Pro Arg Ile Thr Arg Phe
    1280                1285                1290

Lys Phe Ser Ser Phe His Phe Leu Asn Arg Phe Pro Ser Val Tyr
    1295                1300                1305

Leu Gln Cys Lys Leu Val Val Cys Arg Ala Asn Asp Val Ser Ser
    1310                1315                1320

Arg Cys Tyr Arg Gly Cys Val Val Arg Ser Lys Arg Asp Val Gly
    1325                1330                1335

Ser Tyr Gln Glu Lys Val Asp Val Val Leu Gly Pro Ile Gln Leu
    1340                1345                1350

Gln Ser Pro Ser Lys Glu Lys Arg Ser Leu Asp Leu Ala Val Ala
    1355                1360                1365

Asp Val Glu Lys Pro Ala Ser Ser Gln Glu Val Tyr Pro Thr Ala
    1370                1375                1380

Ala Ile Phe Gly Gly Val Phe Leu Ala Leu Val Val Ala Val Ala
    1385                1390                1395

Ala Phe Thr Leu Gly Arg Lys Thr Arg Thr Ala Arg Gly Gln Pro
    1400                1405                1410

Pro Ser Thr Lys Met
    1415

<210> 16
<211> 1396
<212> PRT
<213> Rattus norvegicus

<400> 16

Met Gly Ile Ser Ile Val Ile Phe Glu Ile Cys Leu Leu Trp Gly Gln
1               5                   10                  15

Ile Leu Ser Thr Ala Ser Gln Ser Arg Ser Ser Thr Pro Asp Trp Trp
                20                  25                  30

Asn His Gly Gly Thr Ile Asn Asp Val Ile Tyr Asp Thr Gln Glu Thr
        35                  40                  45

Pro Glu Val Thr Thr Thr Gln Val Pro Asp Ser Thr Ser Ile Gly Thr
    50                  55                  60

Asp Ser Gly Leu Ala Val Arg Leu Val Asn Gly Gly Asp Arg Cys Arg
65                  70                  75                  80

Gly Arg Val Glu Ile Leu Tyr Gln Gly Ser Trp Gly Thr Met Cys Asp
                85                  90                  95

Asp Ser Trp Asp Ile Asn Asp Ala Asn Val Val Cys Arg Gln Leu Gly
            100                 105                 110

Cys Gly Trp Ala Leu Ser Ala Pro Gly Ser Ala Gln Phe Gly Gln Gly
        115                 120                 125

Leu Gly Pro Ile Val Leu Asp Asp Val Ala Cys Arg Gly His Glu Ala
    130                 135                 140

Tyr Leu Trp Ser Cys Ser His Arg Gly Trp Leu Ser His Asn Cys Gly
145                 150                 155                 160

His Gln Glu Asp Ala Gly Val Ile Cys Ser Asp Ser Gln Thr Ser Ser
                165                 170                 175

Pro Thr Pro Gly Trp Trp Asn Pro Gly Gly Thr Asn Asn Asp Val Ile
            180                 185                 190

Tyr Asp Thr Gln Glu Thr Thr Glu Thr Ser Gln Thr Ser Ser Pro Thr
        195                 200                 205

Pro Asp Trp Trp Asn His Gly Gly Thr Ile Asn Asp Val Ile Tyr Asp
    210                 215                 220

Thr Gln Glu Thr Thr Glu Gly Thr Asp Ser Gly Leu Ala Val Arg Leu
225                 230                 235                 240

Val Asn Gly Gly Asp Arg Cys Arg Gly Arg Val Glu Ile Leu Tyr Gln
                245                 250                 255

Gly Ser Trp Gly Thr Val Cys Asp Asp Ser Trp Asp Ile Asn Asp Ala
            260                 265                 270

Asn Val Val Cys Arg Gln Leu Gly Cys Gly Trp Ala Leu Ser Ala Pro
        275                 280                 285

Gly Ser Ala Gln Phe Gly Gln Gly Ser Gly Ser Ile Val Leu Asp Asp
    290                 295                 300

Val Ala Cys Arg Gly His Glu Ala Tyr Leu Trp Ser Cys Ser His Arg
305                 310                 315                 320

Gly Trp Leu Ser His Asn Cys Gly His Gln Glu Asp Ala Gly Val Ile
                325                 330                 335

Cys Ser Tyr Ser Pro Thr Ser Ser Pro Thr Pro Gly Trp Trp Asn Pro
            340                 345                 350

Gly Phe Thr Asn Ser Asp Val Ser Tyr Arg Thr Glu Leu Pro Thr Asp
        355                 360                 365

Asp Ser Gln Thr Ser Ser Pro Thr Pro Asp Ser Gln Thr Ser Ser Pro
    370                 375                 380

Thr Pro Gly Trp Trp Asn Pro Gly Gly Thr Asn Asn Asp Val Ser Tyr
385                 390                 395                 400

Gly Pro Glu Gln Thr Thr Asp Ala Thr Asp Ser Gly Leu Ala Val Arg
                405                 410                 415

Leu Val Asn Gly Gly Asp Arg Cys Gln Gly Arg Val Glu Ile Leu Tyr
            420                 425                 430

Gln Gly Ser Trp Gly Thr Val Cys Asp Asp Ser Trp Asp Thr Lys Asp
        435                 440                 445

Ala Asn Val Val Cys Arg Gln Leu Val Cys Gly Trp Ala Leu Ser Ala
    450                 455                 460

Pro Gly Ser Ala His Phe Gly Gln Gly Ser Gly Ser Ile Val Leu Asp
465                 470                 475                 480

Asp Val Ala Cys Thr Gly His Glu Ala Tyr Leu Trp Ser Cys Ser His
                485                 490                 495

Arg Gly Trp Leu Ser His Asn Cys Gly His His Glu Asp Ala Gly Val
            500                 505                 510

Ile Cys Ser Asp Ala Gln Thr Gln Ser Thr Thr Trp Pro Asp Met Trp

        515                 520                 525

Pro Thr Thr Thr Pro Glu Thr Thr Thr Asp Trp Trp Thr Thr Lys Tyr
    530                 535                 540

Ser Ser Ser Val Pro Thr Thr Gln Phe Pro Thr Ile Ala Asp Trp Trp
545                 550                 555                 560

Thr Thr Pro Ser Pro Glu Tyr Thr Cys Gly Gly Leu Leu Thr Leu Pro
                565                 570                 575

Tyr Gly Gln Phe Ser Ser Pro Tyr Tyr Pro Gly Ser Tyr Pro Asn Asn
            580                 585                 590

Ala Arg Cys Leu Trp Lys Ile Phe Val Ser Ser Met Asn Arg Val Thr
        595                 600                 605

Val Val Phe Thr Asp Val Gln Leu Glu Gly Gly Cys Asn Tyr Asp Tyr
    610                 615                 620

Ile Leu Val Phe Asp Gly Pro Glu Asn Asn Ser Ser Leu Ile Ala Arg
625                 630                 635                 640

Val Cys Asp Gly Phe Asn Gly Ser Phe Thr Ser Thr Gln Asn Phe Met
                645                 650                 655

Ser Val Val Phe Ile Thr Asp Gly Ser Val Thr Arg Arg Gly Phe Gln
            660                 665                 670

Ala Asp Tyr Tyr Ser Thr Pro Ile Ser Thr Ser Thr Thr Ser Pro Thr
        675                 680                 685

Thr Phe Pro Ile Val Thr Asp Trp Trp Thr Thr Pro Ser Pro Glu Tyr
    690                 695                 700

Thr Cys Gly Gly Leu Leu Thr Leu Pro Tyr Gly Gln Phe Ser Ser Pro
705                 710                 715                 720

Tyr Tyr Pro Gly Ser Tyr Pro Asn Asn Ala Arg Cys Leu Trp Lys Ile
                725                 730                 735

Phe Val Pro Ser Met Asn Arg Val Thr Val Val Phe Thr Asp Val Gln
            740                 745                 750

Leu Glu Gly Gly Cys Asn Tyr Asp Tyr Ile Leu Gly Phe Asp Gly Pro
        755                 760                 765

Glu Tyr Asn Ser Ser Leu Ile Ala Arg Val Cys Asp Gly Ser Asn Gly
    770                 775                 780

Ser Phe Thr Ser Thr Gln Asn Phe Met Ser Val Val Phe Ile Thr Asp
785                 790                 795                 800

Gly Ser Val Thr Arg Arg Gly Phe Gln Ala Asp Tyr Tyr Ser Thr Pro
                805                 810                 815

Ile Arg Thr Ser Thr Arg Asn Asp Ser Ser Leu Val Leu Arg Leu Val
            820                 825                 830

Asn Gly Thr Asn Arg Cys Glu Gly Arg Val Glu Ile Leu Tyr Arg Gly
        835                 840                 845

Ser Trp Gly Thr Val Cys Asp Asn Ser Trp Asp Ile Asn Asp Ala Asn
    850                 855                 860

Val Val Cys Arg Gln Leu Gly Cys Gly Ser Ala Leu Ser Ala Pro Gly
865                 870                 875                 880

Asn Ala Trp Phe Gly Gln Gly Ser Gly Leu Ile Val Leu Asp Asp Val
                885                 890                 895

Ser Cys Ser Gly Tyr Glu Ser His Leu Trp Asn Cys Arg His Pro Gly
            900                 905                 910

Trp Leu Val His Asn Cys Arg His Val Glu Asp Ala Gly Val Ile Cys
        915                 920                 925

Ser Leu Pro Asp Pro Thr Pro Ser Pro Gly Pro Val Asn Tyr Thr Cys
    930                 935                 940

Gly Gly Phe Leu Thr Gly Leu Ser Gly Gln Phe Ser Ser Pro Tyr Tyr
945                 950                 955                 960

Pro Gly Ser Tyr Pro Asn Asn Ala Arg Cys Leu Trp Asn Ile Glu Val
                965                 970                 975

Pro Asn Asn Tyr Arg Val Thr Val Val Phe Arg Asp Val Gln Leu Glu
            980                 985                 990

Gly Gly Cys Asn Tyr Asp Tyr Ile  Glu Ile Phe Asp Gly  Pro His His
        995                 1000                 1005

Ser Ser  Pro Leu Ile Ala Arg  Val Cys Asp Gly Ala  Met Gly Ser
    1010                 1015                 1020

Phe Thr Ser Thr Ser Asn Phe Met Ser Val Arg Phe Thr Thr Asp
    1025                1030                1035

His Ser Val Thr Arg Arg Gly Phe Arg Ala Asp Tyr Tyr Ser Asp
    1040                1045                1050

Phe Asp Asn Asn Thr Thr Asn Leu Leu Cys Leu Ser Asn His Met
    1055                1060                1065

Arg Ala Ser Val Ser Arg Ser Tyr Leu Gln Ser Met Gly Tyr Ser
    1070                1075                1080

Ser Arg Asp Leu Val Ile Pro Gly Trp Asn Val Ser Tyr Gln Cys
    1085                1090                1095

Gln Pro Gln Ile Thr Gln Arg Glu Val Ile Phe Thr Ile Pro Tyr
    1100                1105                1110

Thr Gly Cys Gly Thr Thr Lys Gln Ala Asp Asn Glu Thr Ile Asn
    1115                1120                1125

Tyr Ser Asn Phe Leu Lys Ala Ala Val Ser Asn Gly Ile Ile Lys
    1130                1135                1140

Arg Arg Lys Asp Leu His Ile His Val Ser Cys Lys Met Leu Gln
    1145                1150                1155

Asn Thr Trp Val Asn Thr Met Tyr Ile Thr Asn Asn Thr Val Glu
    1160                1165                1170

Ile Gln Glu Val Gln Tyr Gly Asn Phe Asp Val Asn Ile Ser Phe
    1175                1180                1185

Tyr Thr Ser Ser Ser Phe Leu Tyr Pro Val Thr Ser Ser Pro Tyr
    1190                1195                1200

Tyr Val Asp Leu Asp Gln Asn Leu Tyr Leu Gln Ala Glu Val Leu
    1205                1210                1215

His Ser Asp Thr Ser Leu Ala Leu Phe Val Asp Thr Cys Val Ala
    1220                1225                1230

Ser Pro His Pro Asn Asp Phe Ser Ser Leu Thr Tyr Asp Leu Ile
    1235                1240                1245

Arg Ser Gly Cys Ile Arg Asp Glu Thr Tyr Gln Ser Tyr Ser Ser
    1250                1255                1260

Pro Ser Pro Arg Ile Thr Arg Phe Lys Phe Ser Ser Phe His Phe
    1265                1270                1275

Leu Asn Arg Phe Pro Ser Val Tyr Leu Gln Cys Lys Leu Val Val
    1280                1285                1290

Cys Arg Ala Asn Asp Val Ser Ser Arg Cys Tyr Arg Gly Cys Val
    1295                1300                1305

Val Arg Ser Lys Arg Asp Val Gly Ser Tyr Gln Glu Lys Val Asp
    1310                1315                1320

Val Val Leu Gly Pro Ile Gln Leu Gln Ser Pro Ser Lys Glu Lys
    1325                1330                1335

Arg Ser Leu Asp Leu Ala Val Ala Asp Val Glu Lys Pro Ala Ser
    1340                1345                1350

Ser Gln Glu Val Tyr Pro Thr Ala Ala Ile Phe Gly Gly Val Phe
    1355                1360                1365

Leu Ala Leu Val Val Ala Val Ala Ala Phe Thr Leu Gly Arg Lys
    1370                1375                1380

Thr Arg Thr Ala Arg Gly Gln Pro Pro Ser Thr Lys Met
    1385                1390                1395

<210> 17
<211> 1204
<212> PRT
<213> Sus scrofa

<400> 17

Met Gly Thr Ser Ala Val Ile Leu Glu Ile Cys Leu Leu Leu Ser Gln
1               5                   10                  15

Val Leu Thr Thr Val Ser Ser Thr Thr Gln Thr Glu Ser Thr Thr Glu
            20                  25                  30

Asp Arg Thr Gln Ile Thr Glu Thr Ala Phe Trp Glu Thr Gln Thr Ile
        35                  40                  45

Asn Ser Val Ser Glu Ser Asp Leu Pro Gly Thr His Ala Ser Ser Phe
    50                  55                  60

His Thr Glu Glu Pro Leu Thr Thr Ile Ala Ala Glu Gly Thr Glu Trp
65                  70                  75                  80

Asp Leu Ala Leu Arg Leu Val Asn Gly Gly Asp Arg Cys Gln Gly Arg
                85                  90                  95

Val Glu Ile Leu Tyr Gln Gly Ser Trp Gly Thr Val Cys Asp Asp Ser
            100                 105                 110

Trp Asp Thr Asn Asp Ala Asn Val Val Cys Arg Gln Leu Gly Cys Gly
        115                 120                 125

Trp Ala Val Ser Ala Pro Gly Ser Ala Arg Phe Gly Gln Gly Leu Gly
    130                 135                 140

Pro Ile Leu Leu Asp Asp Leu Arg Cys Ser Gly His Glu Thr Tyr Leu
145                 150                 155                 160

Trp Ser Cys Pro His Ser Gly Trp Lys Thr His Asn Cys Gly His Gln
                165                 170                 175

Glu Asp Ala Gly Val Ile Cys Ser Gly Ala Gln Arg Ser Ser Thr Val
            180                 185                 190

Ile Pro Asp Trp Trp Tyr Thr Thr Thr Arg Ser Gln Thr Ala His Ile
        195                 200                 205

Arg Ser Thr Ile Pro Ala Trp Trp His Pro Thr Thr Thr Thr Ala Ala
    210                 215                 220

Arg Thr Glu Trp Asp Leu Ala Leu Arg Leu Val Asn Gly Gly Asp Arg
225                 230                 235                 240

Cys Gln Gly Arg Val Glu Val Leu Tyr Gln Gly Ser Trp Gly Thr Val
                245                 250                 255

Cys Asp Asp Ser Trp Asp Thr Asn Asp Ala Asn Val Val Cys Arg Gln
            260                 265                 270

Leu Gly Cys Gly Trp Ala Val Ser Ala Pro Gly Ser Ala Arg Phe Gly
        275                 280                 285

Gln Gly Ser Gly Pro Ile Leu Leu Asp Asp Leu Arg Cys Ser Gly His
    290                 295                 300

Glu Thr Tyr Leu Trp Ser Cys Pro His Ser Gly Trp Asn Thr His Asn
305                 310                 315                 320

Cys Gly His His Glu Asp Ala Gly Val Ile Cys Ser Asp Ala Gln Arg

                325                 330                 335

Ser Ser Thr Val Ile Pro Asp Trp Trp Tyr Thr Thr Thr Pro Ser Gln
            340                 345                 350

Thr Ala His Ile Arg Ser Thr Ile Pro Ala Trp Trp His Pro Thr Thr
        355                 360                 365

Thr Thr Ala Ala Arg Thr Glu Trp Asp Leu Ala Leu Arg Leu Val Asn
    370                 375                 380

Gly Gly Asp Arg Cys Gln Gly Arg Val Glu Val Leu Tyr Gln Gly Ser
385                 390                 395                 400

Trp Gly Thr Val Cys Asp Asp Ser Trp Asp Thr Asn Asp Ala Asn Val
                405                 410                 415

Val Cys Arg Gln Leu Gly Cys Gly Trp Ala Val Ser Ala Pro Gly Ser
            420                 425                 430

Ala Arg Phe Gly Gln Gly Ser Gly Pro Ile Leu Leu Asp Asp Leu Arg
        435                 440                 445

Cys Ser Gly His Glu Thr Tyr Leu Trp Ser Cys Pro His Ser Gly Trp
    450                 455                 460

Asn Thr His Asn Cys Gly His His Glu Asp Ala Gly Val Ile Cys Ser
465                 470                 475                 480

Asp Ala Gln Arg Ser Ser Thr Val Ile Pro Asp Trp Trp Tyr Thr Thr
                485                 490                 495

Thr Pro Ser Gln Thr Trp Trp His Pro Thr Thr Thr Thr Ala Ala Ser
            500                 505                 510

Pro Ser Ser Pro Cys Gly Gly Phe Leu Thr Ser Ala Ser Gly Thr Phe
        515                 520                 525

Ser Ser Pro Ser Tyr Pro Gly Leu Tyr Pro Asn Asn Ala Asn Cys Val
    530                 535                 540

Trp Glu Ile Glu Val Asn Ser Gly Tyr Arg Ile Asn Leu Gly Phe Asn
545                 550                 555                 560

Asn Leu Gln Leu Glu Val His Ile Asn Cys Ile Tyr Asp Tyr Ile Glu
                565                 570                 575

Ile Phe Asp Glu Ser Pro Gly Ser Asn Thr Ser Leu Gly Lys Ile Cys
            580                 585                 590

Asn His Thr Ser Gln Ile Phe Thr Ser Ser Tyr Asn Arg Met Thr Val
        595                 600                 605

Arg Phe Arg Ser Asp Gly Ser Val Gln Lys Pro Gly Phe Ser Ala Trp
    610                 615                 620

Tyr Asn Ser Phe Pro Arg Asp Ala Ser Leu Arg Leu Val Asn Ser Asn
625                 630                 635                 640

Ser Ser Tyr Pro Ser Cys Ala Gly Arg Val Glu Ile Tyr Gln Gly Gly
                645                 650                 655

Arg Trp Gly Thr Val Cys Asp Asp Gly Trp Asp Ile Gln Asp Ala Gln
            660                 665                 670

Val Val Cys Arg Gln Leu Gly Cys Gly Asn Ala Val Ser Ala Pro Gly
        675                 680                 685

Asn Ala Tyr Phe Gly Pro Gly Ser Gly Pro Ile Thr Leu Asp Asp Val
    690                 695                 700

Ala Cys Ser Gly Thr Glu Ser Thr Leu Trp Gln Cys Arg Asn Arg Gly
705                 710                 715                 720

Trp Phe Ser His Asn Cys Gly His Ser Glu Asp Ala Gly Val Ile Cys
                725                 730                 735

Ser Val Pro Ala Phe Thr Thr Thr Pro Pro Ala Thr Asn Tyr Ser Cys
            740                 745                 750

Gly Gly Phe Leu Ser Gln Ala Ala Gly Gly Phe Asn Ser Pro Phe Tyr
        755                 760                 765

Pro Gly Asn Tyr Pro Asn Asn Ala Asn Cys Val Trp Asp Ile Glu Val
    770                 775                 780

Gln Asn Asn Tyr Arg Val Thr Val Val Phe Arg Asp Val Gln Leu Glu
785                 790                 795                 800

Ser Gly Cys Asn Phe Asp Tyr Ile Glu Val Phe Asp Gly Pro Tyr Arg
                805                 810                 815

Ser Ser Pro Leu Leu Ala Arg Val Cys Asn Gly Ala Ser Gly Ser Phe
            820                 825                 830

Thr Ser Ser Ser Asn Phe Met Ser Ile Arg Phe Ile Ser Asp Val Ser
        835                 840                 845

Val Thr Arg Ala Gly Phe Arg Ala Asn Tyr Tyr Ser Ser Pro Ser Ser
    850                 855                 860

Gly Ser Thr Arg Leu His Cys Leu Gln Asn His Met Gln Ala Ser Val
865                 870                 875                 880

Ser Thr Ser Tyr Leu Gln Ser Leu Gly Tyr Ser Ala Arg Asp Leu Val
                885                 890                 895

Ile Pro Gly Trp Glu Trp Ser Tyr Gln Cys Gln Pro Gln Ile Thr Ser
            900                 905                 910

Thr Gln Val Thr Phe Thr Ile Pro Tyr Ser Ser Cys Gly Thr Ile Gln
        915                 920                 925

Arg Val Asp Asn Asp Thr Ile Thr Tyr Ser Asn Ser Leu Arg Ala Ala
    930                 935                 940

Val Ser Ser Gly Ile Ile Lys Arg Lys Lys Asp Leu Asn Met Tyr Val
945                 950                 955                 960

Ser Cys Arg Met Leu Gln Asn Thr Trp Val Asn Thr Val Tyr Ile Ala
                965                 970                 975

Asn Asp Thr Leu Glu Val Gln Asn Val Gln Tyr Gly Asn Phe Asp Val
            980                 985                 990

Asn Ile Ser Phe Phe Thr Ser Ser Ser Phe Leu Tyr Pro Val Arg Ser
        995                 1000                1005

Ser Pro Tyr Tyr Val Asp Leu Asn Gln Asn Leu Tyr Leu Gln Ala
    1010                1015                1020

Glu Leu Leu His Ala Asn Ala Ser Leu Ala Leu Phe Val Asp Thr
    1025                1030                1035

Cys Val Ala Ser Pro Tyr Pro Asn Asp Phe Thr Thr Leu Thr Tyr
    1040                1045                1050

Asp Leu Ile Arg Ser Gly Cys Val Lys Asp Glu Thr Tyr Gln Ser
    1055                1060                1065

Tyr Ser Gln Pro Ser Pro Arg Ile Val Arg Phe Lys Phe Ser Ser
    1070                1075                1080

Phe His Phe Leu Ser Arg Phe Pro Ser Val Tyr Leu Gln Cys Lys
    1085                1090                1095

Met Val Val Cys Arg Ala Phe Asp Ser Ser Ser Arg Cys Arg Arg
    1100                1105                1110

Gly Cys Val Val Arg Ser Lys Arg Asp Val Gly Ser Tyr Gln Glu
    1115                1120                1125

Lys Val Asp Val Val Leu Gly Pro Ile Gln Leu Gln Thr Leu His
    1130                1135                1140

Ala Glu Lys Arg Ser Leu Asp Gln Pro Ala Val Asp Leu Glu Glu
    1145                1150                1155

Lys Ala Ser Ala Gln Gly Ser Tyr Asp Gly Ala Ala Ile Ser Ala
    1160                1165                1170

Gly Ile Phe Leu Val Val Val Leu Ala Val Ala Ala Phe Thr Leu
    1175                1180                1185

Gly Arg Arg Gly Arg Ala Ala Asp Gly Gln Pro Leu Ile Ser Lys
    1190                1195                1200

Thr

<210> 18
<211> 1349
<212> PRT
<213> Sus scrofa

<400> 18

Met Gly Thr Ser Ala Val Ile Leu Glu Ile Cys Leu Leu Leu Ser Gln
1               5                   10                  15

Val Leu Thr Thr Val Ser Ser Thr Thr Gln Thr Glu Ser Thr Thr Glu
            20                  25                  30

Asp Arg Thr Gln Ile Thr Glu Thr Ala Phe Trp Glu Thr Gln Thr Ile
        35                  40                  45

Asn Ser Val Ser Glu Ser Asp Leu Pro Gly Thr His Ala Ser Ser Phe
    50                  55                  60

His Thr Glu Glu Pro Leu Thr Thr Ile Ala Ala Glu Gly Thr Glu Trp
65                  70                  75                  80

Asp Leu Ala Leu Arg Leu Val Asn Gly Gly Asp Arg Cys Gln Gly Arg
                85                  90                  95

Val Glu Ile Leu Tyr Gln Gly Ser Trp Gly Thr Val Cys Asp Asp Ser
            100                 105                 110

Trp Asp Thr Asn Asp Ala Asn Val Val Cys Arg Gln Leu Gly Cys Gly
        115                 120                 125

Trp Ala Ile Ser Ala Pro Gly Ser Ala Arg Phe Gly Gln Gly Ser Gly
    130                 135                 140

Pro Ile Leu Leu Asp Asp Leu Arg Cys Ser Gly His Glu Thr Tyr Leu
145                 150                 155                 160

Trp Ser Cys Pro His Ser Gly Trp Lys Thr His Asn Cys Gly His Gln
                165                 170                 175

Glu Asp Ala Gly Val Ile Cys Ser Gly Ala Gln Arg Ser Ser Thr Val
            180                 185                 190

Ile Pro Ala Trp Trp His Pro Thr Thr Thr Thr Thr Ala Thr Ser Ser
        195                 200                 205

Phe His Thr Glu Glu Pro Leu Thr Thr Ile Ala Ala Glu Gly Thr Glu
    210                 215                 220

Trp Asp Leu Ala Leu Arg Leu Val Asn Gly Gly Asp Arg Cys Gln Gly
225                 230                 235                 240

Arg Val Glu Ile Leu Tyr Gln Gly Ser Trp Gly Thr Val Cys Asp Asp
                245                 250                 255

Ser Trp Asp Thr Asn Asp Ala Asn Val Val Cys Arg Gln Leu Gly Cys
            260                 265                 270

Gly Trp Ala Val Ser Ala Pro Gly Ser Ala Arg Phe Gly Gln Gly Leu
        275                 280                 285

Gly Pro Ile Leu Leu Asp Asp Leu Arg Cys Ser Gly His Glu Thr Tyr
    290                 295                 300

Leu Trp Ser Cys Pro His Ser Gly Trp Lys Thr His Asn Cys Gly His
305                 310                 315                 320

Gln Glu Asp Ala Gly Val Ile Cys Ser Gly Ala Gln Arg Ser Ser Thr

325                 330                 335

Val Ile Pro Asp Trp Trp Tyr Thr Thr Thr Arg Ser Gln Thr Ala His
            340                 345                 350

Ile Arg Ser Thr Ile Pro Ala Trp Trp His Pro Thr Thr Thr Thr Ala
        355                 360                 365

Ala Arg Thr Glu Trp Asp Leu Ala Leu Arg Leu Val Asn Gly Gly Asp
    370                 375                 380

Arg Cys Gln Gly Arg Val Glu Val Leu Tyr Gln Gly Ser Trp Gly Thr
385                 390                 395                 400

Val Cys Asp Asp Ser Trp Asp Thr Asn Asp Ala Asn Val Val Cys Arg
                405                 410                 415

Gln Leu Gly Cys Gly Trp Ala Val Ser Ala Pro Gly Ser Ala Arg Phe
            420                 425                 430

Gly Gln Gly Ser Gly Pro Ile Leu Leu Asp Asp Leu Arg Cys Ser Gly
        435                 440                 445

His Glu Thr Tyr Leu Trp Ser Cys Pro His Ser Gly Trp Asn Thr His
    450                 455                 460

Asn Cys Gly His His Glu Asp Ala Gly Val Ile Cys Ser Asp Ala Gln
465                 470                 475                 480

Arg Ser Ser Thr Val Ile Pro Asp Trp Trp Tyr Thr Thr Thr Pro Ser
                485                 490                 495

Gln Thr Ala His Ile Arg Ser Thr Ile Pro Ala Trp Trp His Pro Thr
            500                 505                 510

Thr Thr Thr Ala Ala Arg Thr Glu Trp Asp Leu Ala Leu Arg Leu Val
        515                 520                 525

Asn Gly Gly Asp Arg Cys Gln Gly Arg Val Glu Val Leu Tyr Gln Gly
    530                 535                 540

Ser Trp Gly Thr Val Cys Asp Asp Ser Trp Asp Thr Asn Asp Ala Asn
545                 550                 555                 560

Val Val Cys Arg Gln Leu Gly Cys Gly Trp Ala Val Ser Ala Pro Gly
                565                 570                 575

Ser Ala Arg Phe Gly Gln Gly Ser Gly Pro Ile Leu Leu Asp Asp Leu
            580                 585                 590

Arg Cys Ser Gly His Glu Thr Tyr Leu Trp Ser Cys Pro His Ser Gly
        595                 600                 605

Trp Asn Thr His Asn Cys Gly His His Glu Asp Ala Gly Val Ile Cys
    610                 615                 620

Ser Asp Ala Gln Arg Ser Ser Thr Val Ile Pro Asp Trp Trp Tyr Thr
625                 630                 635                 640

Thr Thr Pro Ser Gln Thr Trp Trp His Pro Thr Thr Thr Thr Ala Ala
                645                 650                 655

Ser Pro Ser Ser Pro Cys Gly Gly Phe Leu Thr Ser Ala Ser Gly Thr
            660                 665                 670

Phe Ser Ser Pro Ser Tyr Pro Gly Leu Tyr Pro Asn Asn Ala Asn Cys
        675                 680                 685

Val Trp Glu Ile Glu Val Asn Ser Gly Tyr Arg Ile Asn Leu Gly Phe
    690                 695                 700

Asn Asn Leu Gln Leu Glu Val His Ile Asn Cys Ile Tyr Asp Tyr Ile
705                 710                 715                 720

Glu Ile Phe Asp Glu Ser Pro Gly Ser Asn Thr Ser Leu Gly Lys Ile
                725                 730                 735

Cys Asn His Thr Ser Gln Ile Phe Thr Ser Ser Tyr Asn Arg Met Thr
            740                 745                 750

Val Arg Phe Arg Ser Asp Gly Ser Val Gln Lys Pro Gly Phe Ser Ala
        755                 760                 765

Trp Tyr Asn Ser Phe Pro Arg Asp Ala Ser Leu Arg Leu Val Asn Ser
    770                 775                 780

Asn Ser Ser Tyr Pro Ser Cys Ala Gly Arg Val Glu Ile Tyr Gln Gly
785                 790                 795                 800

Gly Arg Trp Gly Thr Val Cys Asp Asp Gly Trp Asp Ile Gln Asp Ala
                805                 810                 815

Gln Val Val Cys Arg Gln Leu Gly Cys Gly Asn Ala Val Ser Ala Pro
            820                 825                 830

Gly Asn Ala Tyr Phe Gly Pro Gly Ser Gly Pro Ile Thr Leu Asp Asp
        835                 840                 845

Val Ala Cys Ser Gly Thr Glu Ser Thr Leu Trp Gln Cys Arg Asn Arg
    850                 855                 860

Gly Trp Phe Ser His Asn Cys Gly His Ser Glu Asp Ala Gly Val Ile
865                 870                 875                 880

Cys Ser Val Pro Ala Phe Thr Thr Thr Pro Pro Ala Thr Asn Tyr Ser
                885                 890                 895

Cys Gly Gly Phe Leu Ser Gln Ala Ala Gly Gly Phe Asn Ser Pro Phe
            900                 905                 910

Tyr Pro Gly Asn Tyr Pro Asn Asn Ala Asn Cys Val Trp Asp Ile Glu
        915                 920                 925

Val Gln Asn Asn Tyr Arg Val Thr Val Val Phe Arg Asp Val Gln Leu
    930                 935                 940

Glu Ser Gly Cys Asn Phe Asp Tyr Ile Glu Val Phe Asp Gly Pro Tyr
945                 950                 955                 960

Arg Ser Ser Pro Leu Leu Ala Arg Val Cys Asn Gly Ala Ser Gly Ser
                965                 970                 975

Phe Thr Ser Ser Ser Asn Phe Met Ser Ile Arg Phe Ile Ser Asp Val
            980                 985                 990

Ser Val Thr Arg Ala Gly Phe Arg Ala Asn Tyr Tyr Ser Ser Pro Ser
        995                 1000                1005

Ser Gly Ser Thr Arg Leu His Cys Leu Gln Asn His Met Gln Ala
    1010                1015                1020

Ser Val Ser Thr Ser Tyr Leu Gln Ser Leu Gly Tyr Ser Ala Arg
    1025                1030                1035

Asp Leu Val Ile Pro Gly Trp Glu Trp Ser Tyr Gln Cys Gln Pro
    1040                1045                1050

Gln Ile Thr Ser Thr Gln Val Thr Phe Thr Ile Pro Tyr Ser Ser
    1055                1060                1065

Cys Gly Thr Ile Gln Arg Val Asp Asn Asp Thr Ile Thr Tyr Ser
    1070                1075                1080

Asn Ser Leu Arg Ala Ala Val Ser Ser Gly Ile Ile Lys Arg Lys
    1085                1090                1095

Lys Asp Leu Asn Met Tyr Val Ser Cys Arg Met Leu Gln Asn Thr
    1100                1105                1110

Trp Val Asn Thr Val Tyr Ile Ala Asn Asp Thr Leu Glu Val Gln
    1115                1120                1125

Asn Val Gln Tyr Gly Asn Phe Asp Val Asn Ile Ser Phe Phe Thr
    1130                1135                1140

Ser Ser Ser Phe Leu Tyr Pro Val Arg Ser Ser Pro Tyr Tyr Val
    1145                1150                1155

Asp Leu Asn Gln Asn Leu Tyr Leu Gln Ala Glu Leu Leu His Ala
    1160                1165                1170

Asn Ala Ser Leu Ala Leu Phe Val Asp Thr Cys Val Ala Ser Pro
    1175                1180                1185

Tyr Pro Asn Asp Phe Thr Thr Leu Thr Tyr Asp Leu Ile Arg Ser
    1190                1195                1200

Gly Cys Val Lys Asp Glu Thr Tyr Gln Ser Tyr Ser Gln Pro Ser
    1205                1210                1215

Pro Arg Ile Val Arg Phe Lys Phe Ser Ser Phe His Phe Leu Ser
    1220                1225                1230

Arg Phe Pro Ser Val Tyr Leu Gln Cys Lys Met Val Val Cys Arg
    1235                1240                1245

Ala Phe Asp Ser Ser Ser Arg Cys Arg Arg Gly Cys Val Val Arg
    1250                1255                1260

Ser Lys Arg Asp Val Gly Ser Tyr Gln Glu Lys Val Asp Val Val
    1265                1270                1275

Leu Gly Pro Ile Gln Leu Gln Thr Leu His Ala Glu Lys Arg Ser
    1280                1285                1290

Leu Asp Gln Pro Ala Val Asp Leu Glu Glu Lys Ala Ser Ala Gln
    1295                1300                1305

Gly Ser Tyr Asp Gly Ala Ala Ile Ser Ala Gly Ile Phe Leu Val

1310                1315                1320

Val Val  Leu Ala Val Ala Ala  Phe Thr Leu Gly Arg  Arg Gly Arg
    1325                1330                1335

Ala Ala  Asp Gly Gln Pro Leu  Ile Ser Lys Thr
    1340                1345

<210>  19
<211>  16
<212>  PRT
<213>  artificial sequence

<220>
<223>  synthetic peptide

<400>  19

Gln Gly Arg Val Glu Val Leu Tyr Arg Gly Ser Trp Gly Thr Val Cys
1               5                   10                  15

<210>  20
<211>  15
<212>  PRT
<213>  Artificial sequence

<220>
<223>  synthetic peptide

<220>
<221>  misc_feature
<222>  (1)..(1)
<223>  Xaa can be any naturally occurring amino acid

<220>
<221>  misc_feature
<222>  (8)..(9)
<223>  Xaa can be any naturally occurring amino acid

<220>
<221>  misc_feature
<222>  (12)..(15)
<223>  Xaa can be any naturally occurring amino acid

<400>  20

Xaa Arg Val Glu Val Leu Tyr Xaa Xaa Ser Trp Xaa Xaa Xaa Xaa
1               5                   10                  15

<210>  21
<211>  12
<212>  PRT
<213>  Artificial sequence

<220>
<223>  synthetic peptide

<220>
<221>  misc_feature
<222>  (5)..(8)
<223>  Xaa can be any naturally occurring amino acid

<400>  21

Val Glu Ile Leu Xaa Xaa Xaa Xaa Trp Gly Thr Val
1               5                   10


<210>  22
<211>  12
<212>  PRT
<213>  artificial sequence

<220>
<223>  synthetic peptide


<220>
<221>  misc_feature
<222>  (5)..(8)
<223>  Xaa can be any naturally occurring amino acid

<400>  22

Val Glu Val Leu Xaa Xaa Xaa Xaa Trp Gly Thr Val
1               5                   10


<210>  23
<211>  12
<212>  PRT
<213>  artificial sequence

<220>
<223>  synthetic peptide


<220>
<221>  misc_feature
<222>  (5)..(8)
<223>  Xaa can be any naturally occurring amino acid

<400>  23

Val Glu Ile Tyr Xaa Xaa Xaa Xaa Trp Gly Thr Val
1               5                   10


<210>  24
<211>  12
<212>  PRT
<213>  artificial sequence

<220>
<223>  synthetic peptide

<400>  24

Val Glu Ile Leu Tyr Arg Gly Ser Trp Gly Thr Val

1               5                   10


<210>  25
<211>  12
<212>  PRT
<213>  artificial sequence

<220>
<223>  synthetic peptide

<400>  25

Val Glu Val Leu Tyr Arg Gly Ser Trp Gly Thr Val
1               5                   10


<210>  26
<211>  12
<212>  PRT
<213>  artificial sequence

<220>
<223>  synthetic peptide

<400>  26

Val Glu Ile Tyr His Gly Gly Thr Trp Gly Thr Val
1               5                   10


<210>  27
<211>  6
<212>  PRT
<213>  artificial sequence

<220>
<223>  synthetic peptide


<220>
<221>  misc_feature
<222>  (5)..(5)
<223>  Xaa can be any naturally occurring amino acid

<400>  27

Val Glu Val Leu Xaa Gln
1               5


<210>  28
<211>  12
<212>  PRT
<213>  artificial sequence

<220>
<223>  synthetic peptide


<220>
<221>  misc_feature
<222>  (5)..(8)

<223>  Xaa can be any naturally occurring amino acid

<400>  28

Val Glu Ile Phe Xaa Xaa Xaa Xaa Asp Ser Ser Leu
1               5                   10


<210>  29
<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  29

Val Val Val Leu Xaa Xaa Xaa Xaa Trp
1               5


<210>  30
<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  30

Val Glu Val Arg Xaa Xaa Xaa Xaa Trp
1               5


<210>  31
<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  31

Val Glu Ile Ser Xaa Xaa Xaa Xaa Trp
1               5

<210>  32
<211>  9
<212>  PRT
<213>  artificial sequence

<220>
<223>  synthetic peptide

<220>
<221>  misc_feature
<222>  (5)..(8)
<223>  Xaa can be any naturally occurring amino acid

<400>  32

Val Gly Val Leu Xaa Xaa Xaa Xaa Trp
1               5

<210>  33
<211>  9
<212>  PRT
<213>  artificial sequence

<220>
<223>  synthetic peptide

<220>
<221>  misc_feature
<222>  (5)..(8)
<223>  Xaa can be any naturally occurring amino acid

<400>  33

Val Glu Val Ser Xaa Xaa Xaa Xaa Trp
1               5

<210>  34
<211>  9
<212>  PRT
<213>  artificial sequence

<220>
<223>  synthetic peptide

<220>
<221>  misc_feature
<222>  (5)..(8)
<223>  Xaa can be any naturally occurring amino acid

<400>  34

Val Glu Val Phe Xaa Xaa Xaa Xaa Trp
1               5

<210>  35
<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  35

Val Glu Leu Arg Xaa Xaa Xaa Xaa Trp
1               5


<210>  36
<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  36

Val Glu Leu Phe Xaa Xaa Xaa Xaa Trp
1               5


<210>  37
<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  37

Val Glu Val His Xaa Xaa Xaa Xaa Trp
1               5


<210>  38

<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  38

Val Glu Leu Phe Xaa Xaa Xaa Xaa Trp
1               5


<210>  39
<211>  9
<212>  PRT
<213>  Artificial sequence

<220>
<223>  synthetic peptide


<220>
<221>  misc_feature
<222>  (5)..(8)
<223>  Xaa can be any naturally occurring amino acid

<400>  39

Val Glu Ile Leu Xaa Xaa Xaa Xaa Trp
1               5


<210>  40
<211>  9
<212>  PRT
<213>  artificial sequence

<220>
<223>  synthetic peptide


<220>
<221>  misc_feature
<222>  (5)..(8)
<223>  Xaa can be any naturally occurring amino acid

<400>  40

Val Glu Ile Phe Xaa Xaa Xaa Xaa Trp
1               5


<210>  41
<211>  9
<212>  PRT
<213>  artificial sequence

<220>
<223>  synthetic peptide

<220>
<221>  misc_feature
<222>  (5)..(8)
<223>  Xaa can be any naturally occurring amino acid

<400>  41

Val Glu Phe Phe Xaa Xaa Xaa Xaa Trp
1               5

<210>  42
<211>  9
<212>  PRT
<213>  artificial sequence

<220>
<223>  synthetic peptide

<220>
<221>  misc_feature
<222>  (5)..(8)
<223>  Xaa can be any naturally occurring amino acid

<400>  42

Val Glu Leu His Xaa Xaa Xaa Xaa Trp
1               5

<210>  43
<211>  9
<212>  PRT
<213>  artificial sequence

<220>
<223>  synthetic peptide

<220>
<221>  misc_feature
<222>  (5)..(8)
<223>  Xaa can be any naturally occurring amino acid

<400>  43

Leu Glu Val Arg Xaa Xaa Xaa Xaa Trp
1               5

<210>  44
<211>  9
<212>  PRT
<213>  artificial sequence

<220>
<223>  syntehtic peptide

<220>
<221>  misc_feature
<222>  (5)..(8)
<223>  Xaa can be any naturally occurring amino acid

<400>  44

Val Glu Val Gln Xaa Xaa Xaa Xaa Trp
1               5


<210>  45
<211>  9
<212>  PRT
<213>  artificial sequence

<220>
<223>  syntehtic peptide


<220>
<221>  misc_feature
<222>  (5)..(8)
<223>  Xaa can be any naturally occurring amino acid

<400>  45

Val Glu Leu Leu Xaa Xaa Xaa Xaa Trp
1               5


<210>  46
<211>  12
<212>  PRT
<213>  artificial sequence

<220>
<223>  synthetic peptide


<220>
<221>  misc_feature
<222>  (5)..(8)
<223>  Xaa can be any naturally occurring amino acid

<400>  46

Val Glu Leu Tyr Xaa Xaa Xaa Xaa Pro Pro Ala Leu
1               5                   10


<210>  47
<211>  9
<212>  PRT
<213>  artificial sequence

<220>
<223>  synthetic peptide


<220>

<221>  misc_feature
<222>  (5)..(8)
<223>  Xaa can be any naturally occurring amino acid

<400>  47

Leu Glu Val Phe Xaa Xaa Xaa Xaa Trp
1               5

## Description

### Background of Invention

Purifying contaminated solutions is a pivotal challenge in many aspect. For example, contaminated water is a huge challenge in various parts of the world. Furthermore, infected blood leads to sepsis in human patients. Sepsis is one of the most common diseases worldwide and is associated with a very high death rate. Infections leading to pyrogenic reactions or even sepsis are usually caused by bacteria and in some cases also by fungal or viral pathogens. Pathogenic microorganisms like gram negative-bacteria cause sepsis not only by their direct presence but especially through pathogen components such as lipopolysaccharides (LPS) that are located in their outer cell membrane. Sepsis can be either caused by the pathogens themselves and/or by pathogen components such as endotoxins (e.g. LPS). In response to the pathogenic insult, a huge amount of cytokines are released possibly resulting in multiple organ dysfunctions and death despite treatment. It is often not possible to manage the cytokine and the coagulation cascade to a safe level without lethal outcome. Also in products like food, contaminated water or therapeutic agents, pathogens and/or pathogen components are present. This contamination can also influence the medical condition of healthy people and can worsen the condition of ill people. Thus, the reduction of pathogen and/or pathogen component concentration in aqueous solutions and body fluid samples is a real challenge at present and in future.

The current treatment, of e.g. sepsis, consists in the administration of a combination of broad-spectrum antibiotics and simultaneous fluid therapy, vasoactive drugs, respiratory and metabolic support, as well as the administration of corticoids, blood transfusions and dialysis to assist kidney function. Besides a few new approaches, classic treatment options like fluid therapy and hemodynamic support of antibiotics application are still the gold standard. The application of antibiotics has been widely discussed over the last years as more and more resistances occur. Nevertheless, one of the biggest issues delaying a proper identification of the pathogen causing a sepsis is the often very low pathogen concentration in the blood of the patient. Due to this low titer, the pathogens have to be amplified *in vitro.* This is time consuming but currently the only way to identify securely identify the pathogen itself but a prerequisite to be able to identify possible antibiotic resistances. Additionally, many patients are already under antibiotic treatment, when the sepsis diagnosis is intended. This is another obstacle for the rapid identification of the sepsis causing pathogen so that even new techniques like nanopore sequencing, often struggle to identify the pathogens, when present in the solution at a low concentration (Derrington et al. 2010). However, a fast identification of the pathogen is pivotal for a tailored treatment (e.g. antibiotic treatment of the patient).

New systems implemented in dialysis systems using special adsorbent filters are trying to filter cytokines out of the patients' blood to stop the uncontrolled reaction of the body (Zhang et al. 2005). At the moment, the effect of different inflammatory and anti-inflammatory substances or in what amount they are needed to fight the infection are discussed. However, for example LPS is known to play an important role, besides starting the cytokine cascade, in the patients' survival rate as it can also influence blood clotting. To detect LPS or other causes of sepsis, new biomarkers are currently being developed to improve diagnoses, but are very expensive and are specific to only one type of pathogen. Recent clearance methods like particle based endotoxin reduction using antibodies bound to the particles are too selective in most cases, as sepsis is caused by multiple and often unidentified, disease causing factors. Peptides that are similar to the ones found in human saliva, have been shown to absorb a broad spectrum of pathogens.

In addition, extracorporeal filtration systems have also been used, which are aimed at lowering various inflammation mediators such as cytokines. Less frequently used but clinically approved are specific filtrations of pathogens using polymyxin B columns (Toraymyxin^{™}, Kunitomo et al. 2001). However, these columns have various limitations in a clinical setup. There are research approaches to use particles for the filtration of pathogens using e.g. specific antibodies. However, antibody-based systems have several disadvantages as they are often highly specific - possibly too specific - for particular pathogen components such as LPS and also very expensive (Kang et al. 2014). With regard to unspecific extracorporeal adsorbers, there are systems with polymer coatings that are able to bind LPS. However, these unspecific adsorbers merely bind LPS in a concentration range, which is considerably higher than the clinically relevant range of less than 10 EU/ml (EU=endotoxin unit).

In a previous pilot study, the use of iron oxide particles functionalized with a peptide to reduce the amount of LPS was shown (Karawacka et al. 2018). Hydroxyapatite and iron oxide coated particles have gained interest as they can be used in many biomedical fields and are also considered to be used in the reduction of pathogenic biofilms.

Thus, there is a need in the art to improve the purification of aqueous solutions and human body fluids. In this way, the concentration of pathogens and/or pathogen components in an aqueous or body fluid sample would be reduced.

### Summary of Invention

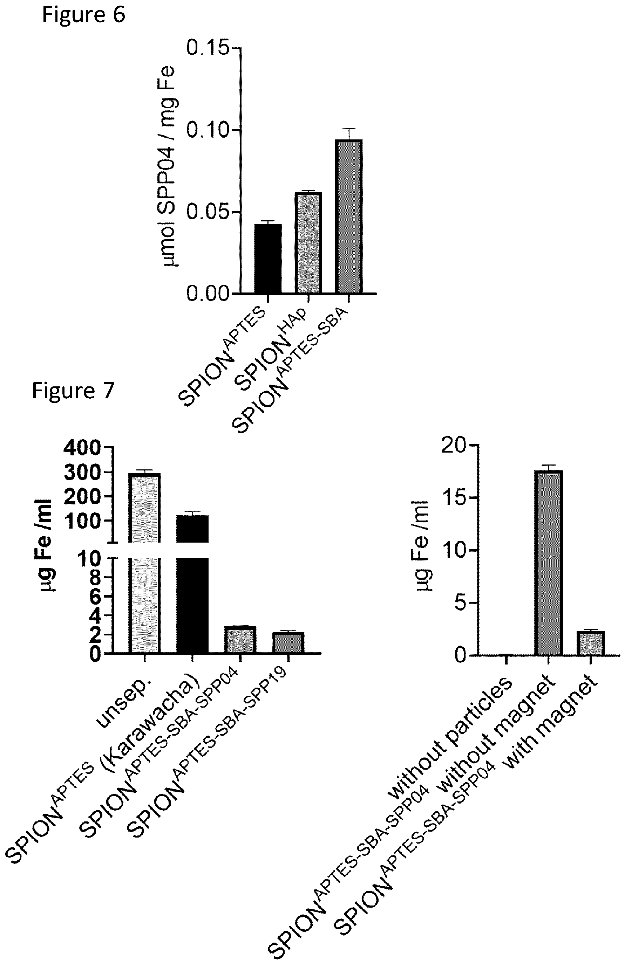

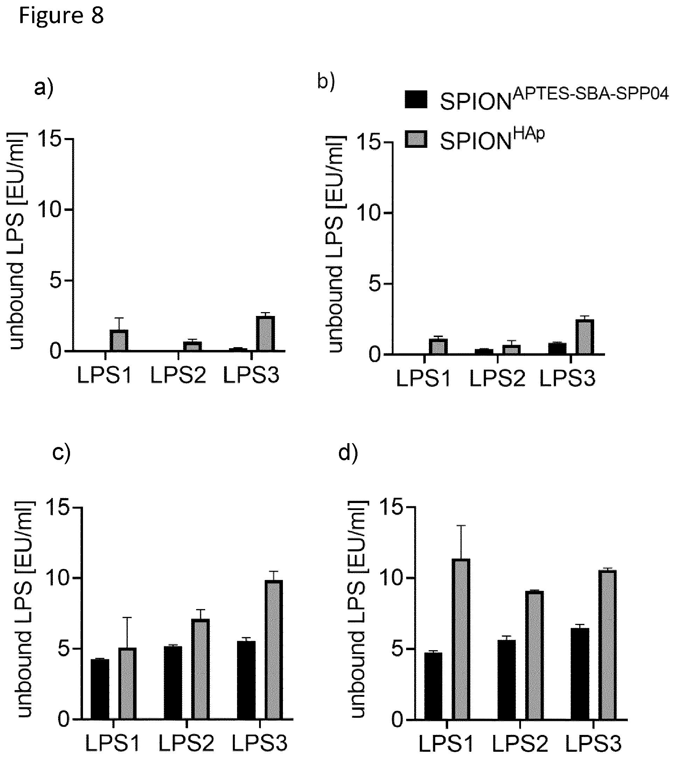

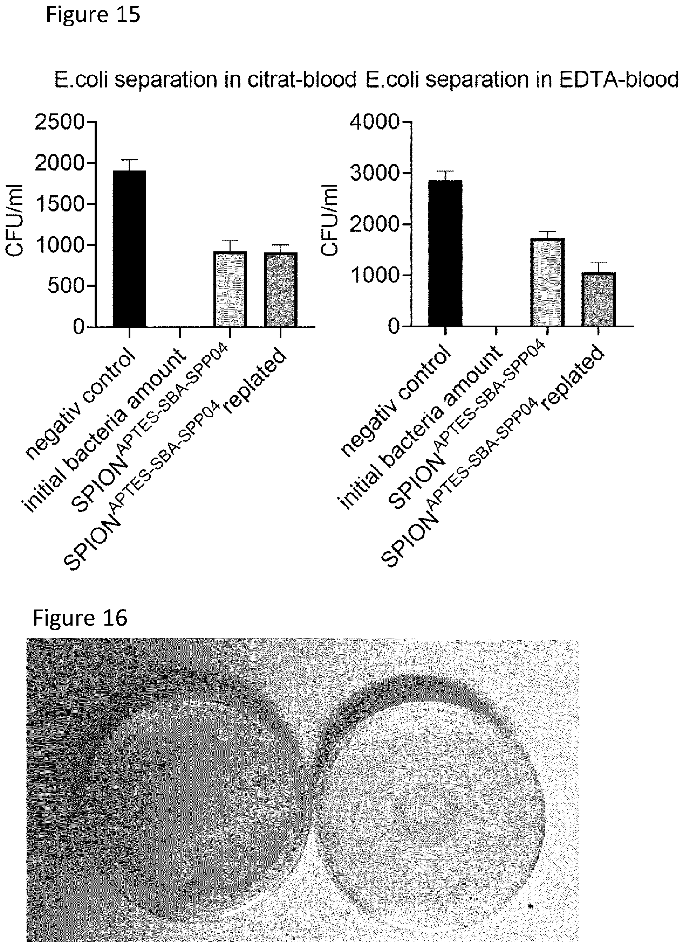

The approach of the present invention uses superparamagnetic iron-based particles for reducing the concentration of pathogens and/or pathogen components in an aqueous or body fluid sample. The present disclosure provides several key advantages over the prior art. Compared to Karawacka et al. (2018), which first described iron-based particles linked to a pathogen binding peptide, the superparamagnetic iron-based particles as disclosed herein have been substantially improved. Specifically, the superparamagnetic iron-based particles as disclosed are magnetically attractable so that at least 65% of the particles can be immobilized when a magnetic field, of e.g. 310 mT, is applied. As compared and contrasted in **Example 2 (****Figure 7****)** herein, the particles of Karawacka et al. (2018) could not be efficiently attracted. However, magnetic attractability is pivotal for using the superparamagnetic iron-based particles for reducing the concentration of pathogens and/or pathogen components in an aqueous or body fluid sample. Apart from substantial improvement of the superparamagnetic iron-based particle magnetability characteristic, the present disclosure also demonstrates with experimental evidence that linked target binding peptides are able to reduce the concentration of pathogens such as *E.coli, P.aeruginosa* and *Serratia* as well as pathogen components such as LPS and LTA (**Example 3-5,** **Figures 8****,** **9****,** **11-14**). Even though the target binding peptides have been described in general previously in the art, it is surprising that the linked target binding peptides to iron-based particles significantly reduce the concentration of pathogens and/or pathogen components, e.g. LPS, LTA, *E.coli, P.aeruginosa* and *Serratia.* In addition, the general applicability of the target binding peptide in the context of reducing the concentration of pathogens and/or pathogen components is also demonstrated in different aqueous solutions. The reduction of bacteria has not only been demonstrated in Ringer solution but also in human blood (**Example 6,** **Figure 15**). Thus, the superparamagnetic iron-based particles as disclosed herein can be used in aqueous solutions such as drinking water as well as body fluid samples such as blood in an *ex vivo* setup. Furthermore, the iron-based particles as disclosed herein can also be used in a method to identify pathogens in a sample. The identification is of pathogens is aided as the particles concentrate the pathogens and, surprisingly, the pathogens are still viable after immobilization (**Example 7,** **Figure 16**). Thus, the pathogen may not need to be amplified, which saves time and effort when trying to identify pathogens. This acceleration in the identifying process can for example be valuable time for a septic patient, who can potentially be treated more quickly.

In particular, a method for reducing the concentration of pathogens and/or pathogen components in an aqueous or body fluid sample is described herein, wherein the method comprises the steps of:
a. providing the aqueous or body fluid sample;
b. incubating the sample with superparamagnetic iron-based particles, wherein the superparamagnetic iron-based particles are linked to a target binding peptide wherein the target is a pathogen or a pathogen component; and
c. immobilising the superparamagnetic iron-based particles with a magnetic field and thereby separating the pathogen-bound and/or pathogen component-bound superparamagnetic iron-based particles from the sample;
   whereby a reduced concentration of pathogens, and/or pathogen components in the sample is obtained, as further defined in the claims.

Furthermore, a method for identifying pathogens in an aqueous or body fluid sample is disclosed, the method comprising the steps of
a. providing the aqueous or body fluid sample;
b. incubating the sample with superparamagnetic iron-based particles, wherein the superparamagnetic iron-based particles are linked to a pathogen binding peptide;
c. immobilising the superparamagnetic iron-based particles with a magnetic field and thereby separating the pathogen-bound superparamagnetic iron-based particles from the sample; and
d. identifying the separated pathogens, as further defined in the claims.

Specifically, the magnetic attractability of the superparamagnetic iron-based particles may be characterized by a reduction of the superparamagnetic iron-based particle concentration in Ringer solution by 65% to 99.95% when applying a magnetic field of 0.31 Tesla for three minutes in static condition, as defined by the claims.

In another aspect, a use of superparamagnetic iron-based particles for reducing the concentration of pathogens and/or pathogen components in an aqueous or body fluid sample is disclosed, as defined in the claims. Furthermore, a use of superparamagnetic iron-based particles for identifying pathogens in an aqueous or body fluid sample is disclosed, wherein the superparamagnetic iron-based particles are linked to a target binding peptide, wherein the target is a pathogen, as defined in the claims.

Finally, superparamagnetic ironoxide nanoparticles (SPIONs) are disclosed, wherein the SPIONs are linked to a target binding peptide, wherein the target is a pathogen, and/or a pathogen component, as further defined in the claims.

### Detailed Description of Invention

In a first aspect, a method for reducing the concentration of pathogens and/or pathogen components in an aqueous or body fluid sample is described herein, wherein the method comprises the steps of:
a. providing the aqueous or body fluid sample;
b. incubating the sample with superparamagnetic iron-based particles, wherein the superparamagnetic iron-based particles are linked to a target binding peptide wherein the target is a pathogen or a pathogen component; and
c. immobilising the superparamagnetic iron-based particles with a magnetic field and thereby separating the pathogen-bound and/or pathogen component-bound superparamagnetic iron-based particles from the sample;
whereby a reduced concentration of pathogens, and/or pathogen components in the sample is obtained, as further defined in the claims.

In general, "superparamagnetic iron-based particles" as used herein are particles, which are magnetically attractable. The skilled person is aware that the magnetic behaviour of materials can be classified into five major groups: ferro-, ferri-, para-, antiferro-, and diamagnetism. Ferro- and ferrimagnetism are the basic mechanisms in permanent magnets. Permanent magnets can be magnetized by external magnetic fields and remain magnetized after the removal of the fields. Materials such as Fe (iron), Co, Ni, and their alloys, and some compounds of rare earth elements are ferromagnetic (Wu et al. 2019). Superparamagnetism is a form of magnetism, which appears in small ferromagnetic or ferrimagnetic particles. "Particles" as used herein are defined as particles of matter that are from 1 nm (nanometer) to 2 micrometers (µm) in diameter. The inventors contemplate that a particle as used herein can be formed of several individual crystallites forming an agglomerate while retaining superparamagnetism. Furthermore, the skilled person is aware of iron (Fe) based particles, such as ironoxide based particles, which have been used in biological applications (Wu et al. 2019). The skilled person is also aware of various iron-based particles. For example, the particles may be iron oxide, iron (Fe), iron-cobalt, alnico or permalloy particles. In other words, the superparamagnetic iron-based particles may be selected from iron oxide, iron (Fe), iron-cobalt, alnico, and permalloy particles.

The superparamagnetic iron-based particles may be superparamagnetic ironoxide nanoparticles (SPIONs). A "nanoparticle", as used herein, is a particle of matter that is between 1 nm and 1 µm in diameter. Furthermore, the iron oxide may be selected from Fe₃O₄, γ-Fe₂O₃, CoFe₂O₄ or a mixture thereof. In a strongly preferred embodiment, the iron oxide is Fe₃O₄.

The "magnetic attractability" of the superparamagnetic iron-based particles, as disclosed herein, can be measured. Specifically, the magnetic attractability of the particles as disclosed herein may be characterized by a reduction of the superparamagnetic iron-based particle concentration in Ringer solution by at least 65% when applying a magnetic field of 310 Millitesla for three minutes in static condition. In other words, the skilled person can compare the iron content, in e.g. Ringer solution, before and after applying a magnetic field in the supernatant. Upon magnetic field application, the superparamagnetic iron-based particles are immobilised and the iron concentration in the supernatant is thereby reduced. The iron content in a solution may be for example determined with Atomic Emission Spectroscopy (AES). The skilled person is also aware of other methods in order to determine the iron content in a sample. For example, the concentration of iron in a sample can also be determined photometrically. However, a determination of the iron concentration by AES is preferred. In **Example 1**, exemplary AES results are presented herein.

Furthermore, **Example 2** specifically compares magnetically attractable particles as disclosed herein with the particles as obtained by following the protocol of Karawacka et al. (2018). As exemplified by **Figure 7**, the iron concentration is reduced by 99% for iron-based particles as disclosed herein. In contrast, the particles as described in the art by Karawacka et al. (2018) are far less magnetically attractable and only show a reduction by around 60%. Based on Karawacka et al. (2018), a substantial improvement of the magnetic attractability of the particles was required, so that they can be used e.g. for reducing the pathogen concentration in septic patient blood *ex vivo* or for diagnostic purposes. Surprisingly, by increasing the amount of educts during preparation, the speed of mixing and/or by changing the clean-up of the particles volume, the magnetic attractability of the particles could be substantially improved. **Example 1** describes the manufacture of exemplary superparamagnetic iron-based particles and **Example 2** describes the substantial improvement over the particles as described in Karawacka et al. (2018).

Similarly to the static condition, the magnetic attractability may also be assessed in a flowing sample. In a preferred embodiment, the magnetic attractability is characterized by a reduction of the superparamagnetic iron-based particle concentration in water by at least 65% when applying a magnetic field of 0.8 Tesla at a flow speed of 0.057 m/s. The skilled person is able to assess the iron content in a supernatant solution, e.g. by AES, as described above. In a particularly preferred embodiment, the concentration of the superparamagnetic iron-based particles is reduced by 65% to 99.95%, preferably 70% to 99.95%, more preferably 75% to 99.95%, more preferably 80% to 99.95%, more preferably 90% to 99.95%, more preferably 91% to 99.95%, more preferably 92% to 99.95%, more preferably 93% to 99.95%, more preferably 94% to 99.95%, more preferably 95% to 99.95%, more preferably 96% to 99.95%, more preferably 97% to 99.95%, more preferably 98% to 99.95%, more preferably 99% to 99.95%, more preferably 99.1% to 99.95%, and even more preferably 99.2% to 99.95%. Said especially preferred ranges apply to the magnetic attractability when assessed in static condition and/or in flow condition. The exact ranges can be independently selected for the static condition and the flow condition.

The superparamagnetic iron-based particles may be superparamagnetic ironoxide nanoparticles (SPIONs). Of course, the skilled person is aware that iron oxides are chemical compounds composed of iron and oxygen. There are several known iron oxides and oxyhydroxides. In a strongly preferred embodiment, the iron oxide is selected from Fe3O4, γ-Fe2O3, CoFe2O4 or a mixture thereof, and even more preferably wherein the iron oxide is Fe3O4. Exemplary SPIONs and a method for the production of SPIONs is described in **Example 1** and **2**.

The magnetic attractability of such SPIONs may be characterized as a reduction of the SPION concentration to a range of 0.01 to 12 ug Fe/ml when incubating the SPIONs at a concentration of 300 ug Fe/ml in water and applying 0.31 Tesla for three minutes in static condition. In particular, the magnetic attractability may be characterized by a reduction of the SPION concentration to a range of 0.01 to 12 ug Fe/ml when incubating the SPIONs at a concentration of 17.5 ug Fe/ml in water and applying 0.8 Tesla at a flow speed of 0.057 m/s. Especially, the SPION concentration may be reduced to a concentration from 0.01 to 10 ug Fe/ml, preferably from 0.01 to 8 ug Fe/ml, preferably from 0.01 to 7 ug Fe/ml, more preferably from 0.01 to 6 ug Fe/ml, preferably from 0.01 to 5 ug Fe/ml, more preferably from 0.01 to 4 ug Fe/ml, and even more preferably from 0.01 to 3 ug Fe/ml.

The "sample", as used herein is an aqueous of body fluid sample. In general, an "aqueous solution" is a solution in which the solvent is water. In other words, an aqueous solution is water soluble and the skilled person is aware of aqueous solutions. In addition, the method as disclosed herein reduces the concentration of pathogens or pathogen components in said samples. Exemplary aqueous solutions are waste water, ground water, or drinking water. In aqueous solutions such as waste water, the reduction of the concentration of pathogens or pathogen components, may support the regeneration of waste water. In aqueous solutions such as ground water or drinking water, the reduction of the concentration of pathogens or pathogen components may make the ground water or drinking water suitable for human consumption.

In general, a reduction of pathogens or pathogen components in aqueous of "body fluid sample" may have the advantage that the immune system of a human may be supported e.g. in fighting a pathogen infection. Furthermore, a "body fluid" is any fluid, which occurs in or is excrete from a body. Especially preferred are samples from a human body. In particular, the sample may be a body fluid sample, which may be selected from blood, serum, plasma, lymph, urine, liquor, saliva and sputum, even more preferably wherein the human body fluid is blood or serum, and even more preferably wherein the human body fluid is blood. In an especially preferred embodiment, the blood is from a septic patient. If the sample is blood of a body, it is preferred that the blood sample is a mammalian blood sample, more preferably a primate blood sample, and even more preferably a human blood sample.

According to the method as disclosed herein, the concentration of pathogens, and/or pathogen components in the sample of step (c) may be reduced by at least 20%, preferably at least 25%, more preferably at least 30%, more preferably at least 35%, even more preferably at least 40%, even more preferably at least 45%, even more preferably at least 50%, even more preferably at least 55%, even more preferably at least 60%, even more preferably at least 65%, even more preferably at least 70%, and even more preferably at least 75% compared to the sample of step (a). The skilled person is able to determine the concentration of pathogens by standard experimentation. For example, the skilled person may measure the concentration of bacteria by counting the colony forming units (CFUs) on a growth plate. Alternatively, the skilled person may quantify the concentration of a pathogen by quantitative PCR, genome sequencing, ELISA, or mass spectrometry, which is also known in the art. The concentration of pathogen components, such as LPS or LTA, may be assessed as described in **Example 3**. However, the skilled person may also use other techniques in order to assess the concentration of pathogens and/or pathogen components.

In a preferred embodiment, the sample is incubated in step (b) with a concentration from 0.002 mg Fe/ml to 5 mg Fe/ml superparamagnetic iron-based particles per volume of sample, preferably from 0.004 mg Fe/ml to 3 mg Fe/ml superparamagnetic iron-based particles, more preferably from 0.007 mg Fe/ml to 2 mg Fe/ml superparamagnetic iron-based particles, even more preferably from 0.01 mg Fe/ml to 1 mg Fe/ml superparamagnetic iron-based particles, and even more preferably from 0.01 mg Fe/ml to 0.15625 mg Fe/ml superparamagnetic iron-based particles. As described above, the skilled person may assess the concentration of Fe/ml by AES. Strongly preferred concentrations are also disclosed in the Example section.

In light of the present disclosure, the skilled person is able to determine suitable time periods for the duration of step (b) (see e.g. **Figures 8-15**). The sample may be incubated in step (b) for a time period of at least 0.5 seconds, preferably at least 1 second, more preferably at least 2 seconds, more preferably at least 3 seconds, more preferably at least 10 seconds, more preferably at least 30 seconds, more preferably at least 1 minute, more preferably at least 2 minutes, more preferably at least 3 minutes, even more preferably at least 4 minutes, and most preferably for about 5 minutes. In a further preferred embodiment, the sample is incubated in step (b) for a time period of at most 1 hour, preferably at most 45 minutes, more preferably at most 35 minutes, more preferably at most 30 minutes, more preferably at most 20 minutes, more preferably at most 15 minutes, more preferably at most 10 minutes, more preferably at most 7.5 minutes, and most preferably about 5 minutes. Of course, the skilled person can freely combine any of the above time limits. In an especially preferred embodiment, the sample is incubated in step (b) for a time period from 0.5 second to 45 minutes, preferably from 1 second to 30 minutes, and more preferably from 3 second to 30 minutes.

A "pathogen", as used herein, is any organism that can produce a disease. A pathogen may also be referred to as an infectious agent. In the context of the present invention, "pathogens" includes viruses, prokaryotes like gram-positive and gram-negative bacteria, cyanobacteria, Mollicutes and unicellular eukaryotes (protozoa) like certain genera of fungi, algae and parasites (e.g. sporozoa). Pathogens often possess on their surface sulphated and phosphorylated structures and molecules, which can be bound by a target binding peptide as described in more detail below. Examples sulphated and phosphorylated structures include e.g. lipoteichoic acid (LTA) or lipopolysaccharide.

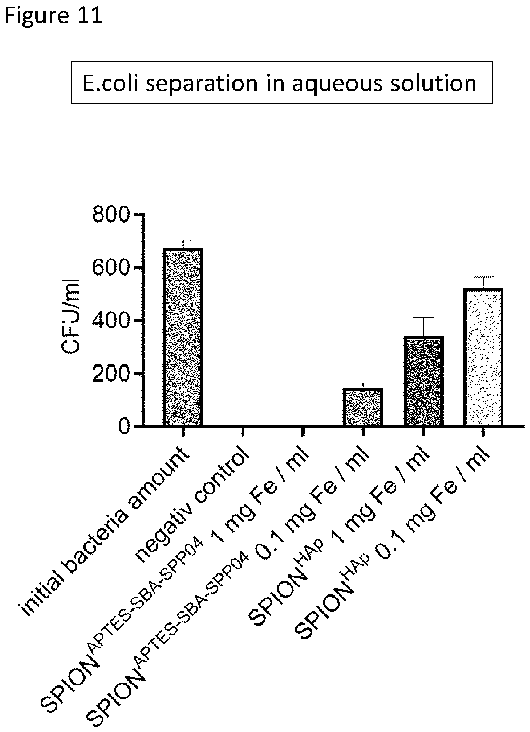

As the target binding peptide is not specific to a particular pathogen, as described below, the concentration of any pathogen can ideally be reduced. The present disclosure provides experimental data for several pathogens, such as gram-positive and gram-negative bacteria **(****Figures 11-15****).** Furthermore, it is known from common general knowledge that the target binding peptides as disclosed herein, are also capable of binding to fungi and viruses (Malamud et al. 2011 and Ligtenberg et al. 2010). Therefore, the superparamagnetic iron-based particles are linked to a target binding peptide as disclosed herein can be used for a wide range of pathogens. In a preferred embodiment, the pathogens may comprise bacteria, fungi and/or viruses, preferably the pathogens may comprise bacteria, even more preferably the pathogens may be bacteria. The skilled person considers that the sample can comprise a mixture of various pathogens, e.g. viruses and bacteria. Furthermore, several species of pathogens can be comprised within the sample. For example, *S. aureus and E.coli* can be comprised within in the sample. Of course, the sample can also contain various bacterial strain, such as different strains of *E.coli.* A preferred fungus, which may be comprised within the sample is *Candida spp.*

If the pathogen comprise bacteria, the pathogens may comprise gram-negative and/or a gram-positive bacteria. Specifically, the pathogen may consist of gram-negative and/or a gram-positive bacteria. The skilled person is aware of various gram-negative and gram-positive bacteria. Exemplary gram-negative bacteria are selected from one or more of *Escherichia coli, Pseudomonas spp., Klebsiella spp..* It is even more preferred that the gram-negative bacteria are selected from *Escherichia coli* (E.coli) and *Pseudomonas aeruginosa* (*P. aeruginosa*). It is further preferred that the gram-negative bacteria are *E.coli.* Exemplary gram-positive bacteria can be selected from one or more of *Staphylococcus aureus* (*S. aureus*), *Serratia, Streptococcus spp., Listeria monocytogenes, Clostridium difficile, Enterobacter,* and preferably the gram-positive bacteria may be *Staphylococcus aureus* or *Serratia,* and most preferably the gram-positive bacteria may be *Staphylococcus aureus.* Experimental data for the immobilisation of *S.aureus, E.coli, P.aeruginosa,* and *Serratia* is provided in the Example section, e.g. **Figures 11-14****.** As all tested bacteria showed a marked reduction of the pathogen concentration, it is expected that the reduction of other pathogens would be in the same range. Therefore, it is expected that the method as disclosed herein supports the reduction pathogens in general.

"Pathogen components" as used herein refers to any part of a pathogen. For example, a pathogen component may be a part of a cell wall or membrane or part of a viral envelope or capsid. For example, the pathogen components may comprise endotoxins and/or pathogenic cell wall components. Endotoxins are pathogen components of gram-negative bacteria and endotoxins are large molecules consisting of a lipid and a polysaccharide composed of O-antigen, outer core, and inner core joined by a covalent bond. Endotoxins are also referred to as lipopolysaccharides (LPSs). The O-antigen is a repetitive glycan polymer contained within an endotoxin. The O-antigen may also be referred to as an O-polysaccharide, or O-side-chain of the bacteria The outer core contains an oligosaccharide component that attaches directly to lipid A and commonly contains sugars such as heptose and 3-Deoxy-D-manno-oct-2-ulosonic acid. Lipid A is usually a phosphorylated glucosamine disaccharide decorated with multiple fatty acids. These hydrophobic fatty acid chains (Lipid A) anchor the LPS to the bacterial membrane, and the rest of the LPS projects from the cell surface. Put simply, LPS is typically found in the outer membrane of Gram-negative bacteria. However, LPS, as well as other pathogen components, are often remainders in an aqueous or body fluid sample. Such pathogen parts themselves can easily trigger an immune response in a human body. In patients, LPS can cause a huge immune response and can lead to septic shock. Consequently, a reduction of pathogen components, such as LPS, in body fluids has the advantage of cleaning the sample, which may reduce the immune response in the human body. Ideally, a patient can recover from the pathogenic attack more quickly, when then amount of pathogens and pathogen components is reduced. Furthermore, the reduction of pathogen components in aqueous samples can also lead to a reduction in immune response, when the aqueous sample is for example consumed as drinking water.

Typically the endotoxins are derived from gram-negative bacteria, more preferably wherein the endotoxins are derived from of one or more of *Klebsiella* spp., *Escherichia coli* (*E.coli*), and *Pseudomonas aeruginosa,* more preferably wherein the endotoxins is LPS, preferably wherein the endotoxins comprise E.coli LPS, and more preferably wherein LPS is selected from one or more of LPS O55:B5, LPS O26:B6, and LPS O111:B4. **Figure 8** provides experimental data for the reduction of three exemplary endotoxins by using the superparamagnetic iron-based particles as disclosed herein. As the concentration of all three types of LPS was reduced significantly, it is assumed that the concentration of endotoxins can be reduced by carrying out the method as disclosed herein.

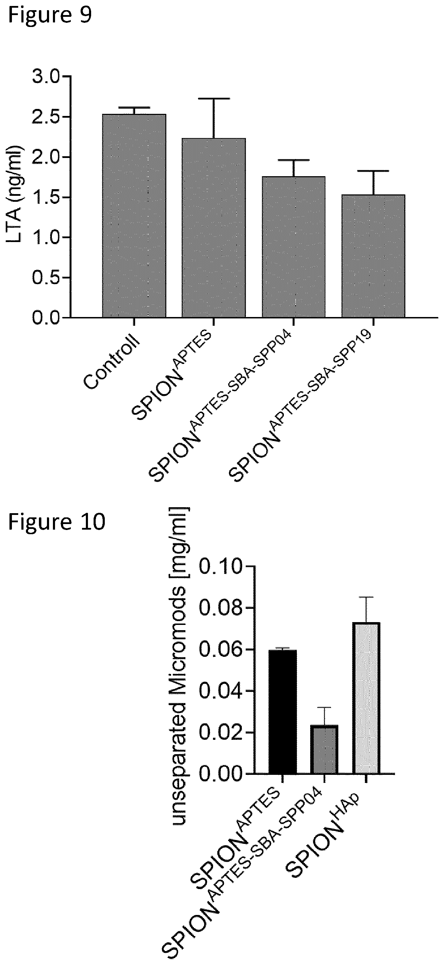

In general, "pathogenic cell wall components" are parts the cell wall of any pathogen having a cell wall. Preferably the cell wall is the outer layer of the pathogen. For example, in gram-positive bacteria and in fungi the cell wall forms the outer layer of the pathogen. In a preferred embodiments, the pathogenic cell wall component is derived from one or more of gram-positive bacteria and fungi, preferably wherein the cell wall component is derived from gram-positive bacteria, more preferably wherein the cell wall component is Lipoteichoic acid (LTA), teichoic acid and/or a peptidoglycan. Lipoteichoic acid (LTA) is a major constituent of the cell wall of gram-positive bacteria. Gram-positive bacteria have an inner (or cytoplasmic) membrane and, external to it, a thick (up to 80 nanometer) peptidoglycan layer. The structure of LTA varies between the different species of Gram-positive bacteria and may contain long chains of ribitol or glycerol phosphate. LTA is anchored to the cell membrane via a diacylglycerol. Teichoic acids are bacterial copolymers of glycerol phosphate or ribitol phosphate and carbohydrates linked via phosphodiester bonds. Teichoic acids are found within the cell wall of most Gram-positive bacteria such as species in the genera *Staphylococcus, Streptococcus, Bacillus, Clostridium, Corynebacterium,* and *Listeria,* and appear to extend to the surface of the peptidoglycan layer. They can be covalently linked to N-acetylmuramic acid or a terminal D-alanine in the tetrapeptide crosslinkage between N-acetylmuramic acid units of the peptidoglycan layer, or they can be anchored in the cytoplasmic membrane with a lipid anchor. Peptidoglycan (also called murein) is a polymer consisting of sugars and amino acids that forms a mesh-like layer outside the plasma membrane of most bacteria, forming the cell wall. The sugar component consists of alternating residues of β-(1,4) linked N-acetylglucosamine (NAG) and N-acetylmuramic acid (NAM). Peptidoglycan serves a structural role in the bacterial cell wall, giving structural strength, as well as counteracting the osmotic pressure of the cytoplasm. In a strongly preferred embodiment, the pathogenic cell wall component is LTA, even more preferably wherein the pathogenic cell wall component is LTA of *Staphylococcus aureus and*/or *Streptococcus pyogenes.* Experimental data for the reduction of LTA after immobilisation with superparamagnetic iron-based particles is provided in **Figure 9** herein. As demonstrated for two exemplary target binding peptide sequences, the concentration of LTA is markedly reduced when using the superparamagnetic iron-based particles as disclosed herein.

The "target binding peptide" as used herein is an amino acid sequence binding to a target, wherein the target is a pathogen or a pathogen component. A 'peptide' as used herein is an amino acid sequence of at most 100 amino acids, preferably at most 80 amino acids, more preferably at most 60 amino acids. Said amino acid sequence may have the more common L-configuration. However, the amino acids may also have the less common D-configuration. The D-configuration may have the advantage that natural proteases may digest the peptide less efficiently than the natural L-configuration. Binding of a peptide to a target can be measured by standard methods in the art such as an agglutination assay, ELISA, fluorescent polarization or FRET. In case the target comprises a nucleic acid, other electrophoretic shift assays may also be used for assessing the binding of target binding peptide to the target. Generally, the skilled person can use any target binding peptide, which binds to a pathogen or a pathogen component.

A "motif", as used herein refers to a short amino acid sequence motif. Specifically, a motif is an amino-acid sequence pattern. A sequence motif shall be distinguished from a structural motif, which is formed by a three-dimensional structure of amino acids. In the present case, that motif is typically important for the pathogen or pathogen component binding. In a preferred embodiment, the target binding peptide has a sequence comprising 12-30 amino acids having one motif selected from SEQ ID NO: 5 (VEVLxxxxW), SEQ ID NO: 6 (VEILxxxxW), SEQ ID NO: 7 (VEIYxxxxW) and SEQ ID NO: 8 (VEVYxxxxW). The motifs of SEQ ID Nos: 5-8 are pathogen or pathogen component binding motifs, wherein the first four amino acids and the tryptophan are key for binding. In an even more preferred embodiment, the target binding peptide has a sequence comprising 12-30 amino acids having one motif selected from SEQ ID NO: 21 (VEILxxxxWGTV), SEQ ID NO: 22 (VEVLxxxxWGTV), and SEQ ID NO: 23 (VEIYxxxxWGTV), even more preferably from SEQ ID NO: 24 (VEILYRGSWGTV), SEQ ID NO: 25 (VEVLYRGSWGTV), and SEQ ID NO: 26 (VEIYHGGTWGTV). In another embodiment, the target binding peptide has a sequence comprising 12-30 amino acids having one motif selected from SEQ ID NO: 27 (VEVLxQ), SEQ ID NO: 28 (VEIFxxxxDSSL), SEQ ID NO: 29 (VVVLxxxxW), SEQ ID NO: 30 (VEVRxxxxW), SEQ ID NO: 31 (VEISxxxxW), SEQ ID NO: 32 (VGVLxxxxW), SEQ ID NO: 33 (VEVSxxxxW), SEQ ID NO: 34 (VEVFxxxxW), SEQ ID NO 35 (VELRxxxxW), SEQ ID NO: 36 (VELFxxxxW), SEQ ID NO: 37 (VEVHxxxxW), SEQ ID NO: 38 (VELFxxxxW), SEQ ID NO: 39 (VEILxxxxW), SEQ ID NO: 40 (VEIFxxxxW, SEQ ID NO: 41 (VEFFxxxxW), SEQ ID NO: 42 (VELHxxxxW), SEQ ID NO: 43 (LEVRxxxxW), SEQ ID NO: 44 (VEVQxxxxW), SEQ ID NO: 45 (VELLxxxxW), SEQ ID NO: 46 (VELYxxxxPPAL), and SEQ ID NO: 47 (LEVFxxxxW).

For example, Leito et al. (2008) describes the motif VEVLxxxxW (SEQ ID NO:5) and its binding to various bacteria in detail. Briefly, various pathogens have been shown to bind to a 16-mer peptide (QGRVEVLYRGSWGTVC, SEQ ID NO: 19) located within the SRCR domains of e.g. DMBT1. An exemplary SRCR domain is disclosed herein as SEQ ID NO: 11. For different bacteria, there was a highly significant correlation between adhesion to DMBT1 and adhesion to SRCR peptide 2 suggesting that SRCR peptide 2 is the major bacteria binding site. Leito et al. (2008) also disclose that 8 amino acids were involved in binding (xRVEVLYxxSWxxxx) by performing an alanine substitution scan. Said motif is derived from DMBT1 (SEQ ID NO: 4), which is a member of the scavenger receptor cysteine-rich (SRCR) superfamily. DMBT1 is secreted protein mainly expressed by epithelia and glands. DMBT1 is also known as salivary agglutinin or glycoprotein-340 (gp340). DMBT1 is a member of the scavenger receptor cysteine-rich (SRCR) superfamily, i.e. proteins that have one or more SRCR domains. Furthermore, DMBT1 is known to selectively interact with some bacterial pathogens (e.g. Prakobphol et al., 2000,) and viruses (e.g. HIV (Wu et al., 2003,) and influenza A viruses (Hartshorn et al., 2003)), and thus inhibits the infectivity of these pathogens. For example, WO 2005/079834 also describes the key elements of the motif and derivatives thereof in detail. In various species the SRCR domains as well as the motifs have slightly varying motifs. As disclosed in SEQ ID NOs: 13-18, DMBT1 from mouse, rat and pig have slightly varying sequences. Variants of the motif that the skilled person might expect, on the basis of reviewed or predicted occurrence in animals such as large and small mammals living on land, in fresh or salt water, reptiles such as crocodiles, snakes and lizards, birds, fish and invertebrates such as the barnacle and liver fluke, will result in a similar binding behaviour to that of the human variants of the binding sequence. This refers to both previously published sequences as they are currently listed under protein sequence databases, such as Uniprot or Genebank with the query DMBT1 or its homologues, or sequences that are published or listed or can be derived from the genome sequencing of further species or other scientific methods. For example, the skilled person can access sequences of DMBT1 with the following identifiers:
1. Mus musculus (Mouse), sp|Q60997| DMBT1_MOUSE
2. Rattus norvegicus (Rat), sp|Q8CIZ5|DMBT1_RAT or sp|Q8CIZ5-4|DMBT1_RAT
3. Sus scrofa (Pig), Q4A3R3
4. Oryctolagus cuniculus (Rabbit), >sp|Q95218|DMBT1_RABIT or >sp|Q95218-2| DMBT1_RABIT
5. Mucin, partial [Bos taurus], GenBank: AAB35069.2
6. Canis lupus familiaris (Dog) (Canis familiaris), UniProtKB - F1PSK2 (F1PSK2_CANLF)
7. Equus caballus (Horse), UniProtKB - A0A650ABH4 (AOA650ABH4_HORSE)
8. Camelus dromedarius (Dromedary), UniProtKB - AOA5N4DIK3 (A0A5N4DIK3_CAMDR)
9. Pan paniscus (Pygmy chimpanzee), UniProtKB - AOA2R9A5Z0 (AOA2R9A5Z0_PANPA)
10. Pan troglodytes (Chimpanzee), UniProtKB - A0A2I3RQK0 (A0A2I3RQK0_PANTR), or UniProtKB - H2R1S1 (H2R1S1_PANTR)
11. Macaca fascicularis (Crab-eating macaque), UniProtKB - A0A2K5WDK7 (A0A2K5WDK7 _MACFA)
12. Papio anubis (Olive baboon) - Anubispavian, UniProtKB - A0A2I3LMY2 (A0A2I3LMY2_PAPAN)
13. Rhinopithecus roxellana (Golden snub-nosed monkey), UniProtKB - A0A2K6QI01 (A0A2K6QI01_RHIRO)
14. Colobus angolensis palliatus (Peters' Angolan colobus), UniProtKB - A0A2K5I9G6 (A0A2K5I9G6_COLAP)
15. Cebus capucinus imitator, UniProtKB - A0A2K5RTE1 (A0A2K5RTE1_CEBCA)
16. Cercocebus atys (Sooty mangabey), UniProtKB - A0A2K5P151 (A0A2K5P151_CERAT)
17. Tarsius syrichta (Philippine tarsier), UniProtKB - A0A3QOE684 (A0A3Q0E684_TARSY)
18. Aotus nancymaae (Ma's night monkey), UniProtKB - A0A2K5DY61 (A0A2K5DY61_AOTNA) or UniProtKB - A0A2K5DXZ5 (A0A2K5DXZ5_AOTNA)
19. Ursus arctos horribilis, UniProtKB - A0A3Q7VB66 (A0A3Q7VB66_URSAR)
20. Ursus maritimus (Thalarctos maritimus), UniProtKB - A0A384BSJ8 (A0A384BSJ8_URSMA)
21. Vulpes vulpes (Red fox), UniProtKB - A0A3Q7TZC5 (A0A3Q7TZC5_VULVU)
22. Erinaceus europaeus, UniProtKB - A0A1S3W6Y7 (A0A1S3W6Y7_ERIEU)
23. Dipodomys ordii (Ord's kangaroo rat), UniProtKB - A0A1S3FLZ2 (A0A1S3FLZ2_DIPOR)
24. Mesocricetus auratus (Golden hamster), UniProtKB - A0A1U7Q2M0 (A0A1U7Q2M0_MESAU)
25. Cricetulus griseus (Chinese hamster), UniProtKB - A0A061IHY8 (A0A061IHY8_CRIGR)
26. Heterocephalus glaber (Naked mole rat), UniProtKB - G5C0A8 (G5C0A8_HETGA)
27. Odobenus rosmarus divergens (Pacific walrus), UniProtKB - A0A2U3ZJA6 (A0A2U3ZJA6_ODORO)
28. Callorhinus ursinus (Northern fur seal), UniProtKB - A0A3Q7PQP7 (A0A3Q7PQP7_CALUR)
29. Ornithorhynchus anatinus (Duckbill platypus), UniProtKB - F6SXX1 (F6SXX1_ORNAN)
30. Balaenoptera acutorostrata scammoni (North Pacific minke whale), UniProtKB - A0A383ZEK5 (A0A383ZEK5_BALAS)
31. Physeter macrocephalus (Sperm whale) (Physeter catodon), UniProtKB - A0A455AME7 (A0A455AME7_PHYMC)
32. Delphinapterus leucas (Beluga whale), UniProtKB - A0A2Y9MTI0 (A0A2Y9MTI0_DELLE)
33. Lipotes vexillifer (Yangtze river dolphin), UniProtKB - A0A340WUA9 (A0A340WUA9_LIPVE)
34. Lonchura striata domestica (Bengalese finch), UniProtKB - A0A218UT58 (A0A218UT58_9PASE)
35. Alligator mississippiensis (American alligator), UniProtKB - A0A151NW93 (A0A151NW93_ALLMI)
36. Alligator sinensis (Chinese alligator), UniProtKB - A0A3Q0GPI6 (A0A3Q0GPI6_ALLSI)
37. Anolis carolinensis (Green anole) (American chameleon), UniProtKB - G1KTW4 (G1KTW4_ANOCA)
38. Pelodiscus sinensis (Chinese softshell turtle) (Trionyx sinensis), UniProtKB - K7FY50 (K7FY50_PELSI)
39. Ophiophagus hannah (King cobra) (Naja hannah), UniProtKB - V8NHZ2 (V8NHZ2_OPHHA)
40. Branchiostoma floridae (Florida lancelet), UniProtKB - C3XV77 (C3XV77_BRAFL)
41. Liparis tanakae (Tanaka's snailfish), UniProtKB - A0A4Z2IX58 (A0A4Z2IX58_9TELE)
42. Danio rerio (Zebrafish) (Brachydanio rerio), UniProtKB - Q5RI27 (Q5RI27_DANRE)
43. Poeciliopsis prolifica (blackstripe livebearer), UniProtKB - A0A0S7FBN9 (A0A0S7FBN9_9TELE)
44. Amphib Calanus amphitrite (Striped barnacle) (Balanus amphitrite), UniProtKB - A0A6A4VLU0 (A0A6A4VLU0_AMPAM)
45. Clonorchis sinensis (Chinese liver fluke), UniProtKB - A0A3R7CC15 (A0A3R7CC15_CLOSI)
46. Stylophora pistillata (Smooth cauliflower coral), UniProtKB - A0A2B4RDP7 (A0A2B4RDP7_STYPI)

In a preferred embodiment, the motif, which is important for the pathogen or pathogen component interaction, can be selected from one of SEQ ID NOs: 5-8 or 21-47, preferably SEQ ID NOs: 5-8.

The target binding peptide may have a sequence comprising 12-30 amino acids having one motif selected from SEQ ID NO: 5-8 or 27-47, preferably the target binding peptide may have a sequence comprising 12-30 amino acids having one motif selected from SEQ ID Nos:5-8, more preferably the target binding peptide may have a sequence comprising 15-25 amino acids having one motif selected from SEQ ID Nos:5-8, more preferably the target binding peptide may have a sequence comprising 16-20 amino acids having one motif selected from SEQ ID Nos: 5-8, more preferably the target binding peptide may have a sequence comprising 17-19 amino acids having one motif selected from SEQ ID Nos: 5-8, even more preferably the target binding peptide may have a sequence comprising 17-19 amino acids having the motif selected from SEQ ID NO:5, even more preferably the target binding peptide may have a sequence comprising 17-19 amino acids having the motif selected from SEQ ID NO:5 and the motif is N-terminally preceded by 4-6 amino acids, most preferably the target binding peptide may have a sequence comprising 17-19 amino acids having the motif selected from SEQ ID NO:5 and the motif is N-terminally preceded by SEQ ID NO:9 (RCQGR). For example, the target binding peptide may have a sequence consisting of 12-30 amino acids having one motif selected from SEQ ID NO: 5-8.

"SRCR domains" are found in eukaryotes and these domains are present on the cell membrane and have a role in binding to specific ligands. The structure contains a six stranded beta-sheet and one alpha-helix. In a preferred embodiment, the target binding peptide shows at least 70% sequence identity to a sequence comprised within a scavenger receptor cysteine-rich (SRCR) protein domain, preferably wherein the target binding peptide shows at least 70% sequence identity to a sequence comprised within a SRCR protein domain of DMBT-1 (GP340). The skilled person can readily assess the identity of amino acids sequence by alignment, e.g. by online sequence alignment tools such as ClustalW2.

The skilled person is able to identify SRCR domain e.g. in the protein data bank (PDB). An exemplary SRCR domain is disclosed in SEQ ID NO: 11. It is even more preferred that the target binding peptide is comprised within one of the SRCR protein domain of DMBT-1 (GP340). All 14 SRCR domains within DMBT1 are disclosed below:

| Domains of DMBT1 | SEQ ID NO: | sequence |
|---|---|---|
| SRCR1 | 21 | LRLVNGDGRCQGRVEILYRGSWGTVCDDSWDTNDANVVCRQLGCGWAMSAPGNAWFGQGSGPIA LDDVRCSGHESYLWSCPHNGWLSHNCGHGEDAGVICS |
| SRCR2 | 22 | LRLVNGGDRCRGRVEVLYRGSWGTVCDDYWDTNDANVVCRQLGCGWAMSAPGNAQFGQGSGPIV LDDVRCSGHESYLWSCPHNGWLTHNCGHSEDAGVICS |
| SRCR3 | 23 | LRLVNGGDRCQGRVEVLYRGSWGTVCDDSWDTSDANVVCRQLGCGWATSAPGNARFGQGSGPIV LDDVRCSGYESYLWSCPHNGWLSHNCQHSEDAGVICS |
| SRCR4 | 24 | LRLVNGGDRCQGRVEVLYRGSWGTVCDDSWDTNDANVVCRQLGCGWAMLAPGNARFGQGSGPIV LDDVRCSGNESYLWSCPHNGWLSHNCGHSEDAGVICS |
| SRCR5 | 25 | LRLVNGGDRCQGRVEVLYRGSWGTVCDDSWDTNDANVVCRQLGCGWATSAPGNARFGQGSGPIV LDDVRCSGHESYLWSCPNNGWLSHNCGHHEDAGVICS |
| SRCR6 | 11 | LRLVNGSDRCQGRVEVLYRGSWGTVCDDSWDTNDANVVCRQLGCGWATSAPGNARFGQGSGPIV LDDVRCSGHESYLWSCPHNGWLSHNCGHHEDAGVICS |
| SRCR7 | 26 | LRLVNGGDRCQGRVEVLYRGSWGTVCDDSWDTSDANVVCRQLGCGWATSAPGNARFGQGSGPIV LDDVRCSGYESYLWSCPHNGWLSHNCQHSEDAGVICS |
| SRCR8 | 27 | LRLVNGGDRCQGRVEVLYQGSWGTVCDDSWDTNDANVVCRQLGCGWAMSAPGNARFGQGSGPIV LDDVRCSGHESYLWSCPHNGWLSHNCGHSEDAGVICS |
| SRCR9 | 28 | LRLVNGGDRCQGRVEVLYRGSWGTVCDDYWDTNDANVVCRQLGCGWAMSAPGNARFGQGSGPIV LDDVRCSGHESYLWSCPHNGWLSHNCGHHEDAGVICS |
| SRCR10 | 29 | LRLVNGGDRCQGRVEVLYRGSWGTVCDDYWDTNDANVVCRQLGCGWATSAPGNARFGQGSGPIV LDDVRCSGHESYLWSCPHNGWLSHNCGHHEDAGVICS |
| SRCR11 | 30 | LRLVNGGDRCQGRVEVLYRGSWGTVCDDYWDTNDANVVCRQLGCGWATSAPGNARFGQGSGPIV LDDVRCSGHESYLWSCPHNGWLSHNCGHHEDAGVICS |
| SRCR12 | 31 | LRLVNGGDRCRGRVEVLYQGSWGTVCDDYWDTNDANVVCRQLGCGWAMSAPGNAQFGQGSGPIVL DDVRCSGHESYLWSCPHNGWLSHNCGHHEDAGVICS |
| SRCR13 | 32 | LRLVNGGDRCRGRVEVLYRGSWGTVCDDSWDTNDANVVCRQLGCGWAMSAPGNARFGQGSGPIVL DDVRCSGNESYLWSCPHKGWLTHNCGHHEDAGVICS |
| SRCR14 | 33 | LRLVNLNSSYGLCAGRVEIYHGGTWGTVCDDSWTIQEAEVVCRQLGCGRAVSALGNAYFGSGSGPITL DDVECSGTESTLWQCRNRGWFSHNCNHREDAGVICS |

In the above table, the individual peptide sequences binding to the pathogen or pathogen binding component are underlined. Furthermore, the binding motif is shaded in grey. Based on these sequences as well as the gene identifiers of the 46 exemplary animals above, the skilled person will readily identify suitable sequences, which are pathogen or pathogen component binding.

It is preferred that the target binding peptide may have a sequence comprising one motif selected from SEQ ID Nos: 5-8 or 27-47 and wherein the target binding peptide comprises a sequence having at least 60% identity to the sequence of SEQ ID NO:1, preferably target binding peptide may have a sequence comprising one motif selected from SEQ ID Nos: 5-8 or 27-47 and the target binding peptide comprises a sequence having at least 65% identity to the sequence of SEQ ID NO:1, more preferably target binding peptide may have a sequence comprising one motif selected from SEQ ID Nos: 5-8 or 27-47and the target binding peptide comprises a sequence having at least 70% identity to the sequence of SEQ ID NO:1, more preferably target binding peptide may have a sequence comprising one motif selected from SEQ ID Nos: 5-8 or 27-47and the target binding peptide comprises a sequence having at least 75% identity to the sequence of SEQ ID NO:1, more preferably target binding peptide may have a sequence comprising one motif selected from SEQ ID Nos: 5-8 or 27-47 and the target binding peptide comprises a sequence having at least 80% identity to the sequence of SEQ ID NO:1, more preferably target binding peptide may have a sequence comprising one motif selected from SEQ ID Nos: 5-8 or 27-47 and the target binding peptide comprises a sequence having at least 85% identity to the sequence of SEQ ID NO:1, more preferably target binding peptide may have a sequence comprising one motif selected from SEQ ID Nos: 5-8 or 27-47 and the target binding peptide comprises a sequence having at least 90% identity to the sequence of SEQ ID NO:1, and even more preferably target binding peptide may have a sequence comprising one motif selected from SEQ ID Nos: 5-8 or 27-47 and the target binding peptide comprises a sequence having at least 95% identity to the sequence of SEQ ID NO:1.

Moreover, the target binding peptide may have a sequence comprising one motif selected from SEQ ID Nos: 5-8 or 27-47 and the target binding peptide comprises a sequence having at least 60% identity to the sequence of SEQ ID NO:2, preferably target binding peptide may have a sequence comprising one motif selected from SEQ ID Nos: 5-8 or 27-47 and the target binding peptide comprises a sequence having at least 65% identity to the sequence of SEQ ID NO:2, more preferably target binding peptide may have a sequence comprising one motif selected from SEQ ID Nos: 5-8 or 27-47 and the target binding peptide comprises a sequence having at least 70% identity to the sequence of SEQ ID NO:2, more preferably target binding peptide may have a sequence comprising one motif selected from SEQ ID Nos: 5-8 or 27-47 and the target binding peptide comprises a sequence having at least 75% identity to the sequence of SEQ ID NO:2, more preferably target binding peptide may have a sequence comprising one motif selected from SEQ ID Nos: 5-8 or 27-47 and the target binding peptide comprises a sequence having at least 80% identity to the sequence of SEQ ID NO:2, more preferably target binding peptide may have a sequence comprising one motif selected from SEQ ID Nos: 5-8 or 27-47 and the target binding peptide comprises a sequence having at least 85% identity to the sequence of SEQ ID NO:2, more preferably target binding peptide may have a sequence comprising one motif selected from SEQ ID Nos: 5-8 or 27-47 and the target binding peptide comprises a sequence having at least 90% identity to the sequence of SEQ ID NO:2, and even more preferably target binding peptide may have a sequence comprising one motif selected from SEQ ID Nos: 5-8 or 27-47 and the target binding peptide comprises a sequence having at least 95% identity to the sequence of SEQ ID NO:2.

In particular, the target binding peptide may have a sequence comprising one motif selected from SEQ ID Nos: 5-8 and wherein the target binding peptide comprises a sequence having at least 60% identity to the sequence of SEQ ID NO:1, preferably target binding peptide may have a sequence comprising one motif selected from SEQ ID Nos: 5-8 and the target binding peptide comprises a sequence having at least 65% identity to the sequence of SEQ ID NO:1, more preferably target binding peptide may have a sequence comprising one motif selected from SEQ ID Nos: 5-8 and the target binding peptide comprises a sequence having at least 70% identity to the sequence of SEQ ID NO:1, more preferably target binding peptide may have a sequence comprising one motif selected from SEQ ID Nos: 5-8 and the target binding peptide comprises a sequence having at least 75% identity to the sequence of SEQ ID NO:1, more preferably target binding peptide may have a sequence comprising one motif selected from SEQ ID Nos: 5-8 and the target binding peptide comprises a sequence having at least 80% identity to the sequence of SEQ ID NO:1, more preferably target binding peptide may have a sequence comprising one motif selected from SEQ ID Nos: 5-8 and the target binding peptide comprises a sequence having at least 85% identity to the sequence of SEQ ID NO:1, more preferably target binding peptide may have a sequence comprising one motif selected from SEQ ID Nos: 5-8 and the target binding peptide comprises a sequence having at least 90% identity to the sequence of SEQ ID NO:1, and even more preferably target binding peptide may have a sequence comprising one motif selected from SEQ ID Nos: 5-8 and the target binding peptide comprises a sequence having at least 95% identity to the sequence of SEQ ID NO:1.

Furthermore, the target binding peptide may have a sequence comprising one motif selected from SEQ ID Nos: 5-8 and the target binding peptide comprises a sequence having at least 60% identity to the sequence of SEQ ID NO:2, preferably target binding peptide may have a sequence comprising one motif selected from SEQ ID Nos: 5-8 and the target binding peptide comprises a sequence having at least 65% identity to the sequence of SEQ ID NO:2, more preferably target binding peptide may have a sequence comprising one motif selected from SEQ ID Nos: 5-8 and the target binding peptide comprises a sequence having at least 70% identity to the sequence of SEQ ID NO:2, more preferably target binding peptide may have a sequence comprising one motif selected from SEQ ID Nos: 5-8 and the target binding peptide comprises a sequence having at least 75% identity to the sequence of SEQ ID NO:2, more preferably target binding peptide may have a sequence comprising one motif selected from SEQ ID Nos: 5-8 and the target binding peptide comprises a sequence having at least 80% identity to the sequence of SEQ ID NO:2, more preferably target binding peptide may have a sequence comprising one motif selected from SEQ ID Nos: 5-8 and the target binding peptide comprises a sequence having at least 85% identity to the sequence of SEQ ID NO:2, more preferably target binding peptide may have a sequence comprising one motif selected from SEQ ID Nos: 5-8 and the target binding peptide comprises a sequence having at least 90% identity to the sequence of SEQ ID NO:2, and even more preferably target binding peptide may have a sequence comprising one motif selected from SEQ ID Nos: 5-8 and the target binding peptide comprises a sequence having at least 95% identity to the sequence of SEQ ID NO:2.

For example, the target binding protein may have a sequence from 12 to 30 amino acids, preferably the target binding protein may have a sequence from 13 to 25 amino acids, more preferably the target binding protein may have a sequence from 14 to 20 amino acids, more preferably the target binding protein may have a sequence from 15 to 19 amino acids, even more preferably the target binding protein may have a sequence from 16 to 18 amino acids.

The target binding peptide may be a pathogen component binding peptide, preferably the target binding peptide binds to a poly-sulfated and/or poly-phosphorylated ligand located on the surface of the pathogen. For example, End et al. (2009) demonstrates with competition experiments and ELISA studies that the SRCR domains of DMBT1 specifically bind poly-sulfated and poly-phosphorylated structures, such as heparansulfate, LPS, and lipoteichoic acid. As demonstrated in End et al. (2009), the skilled person is able to determine whether a peptide sequence binds to poly-sulfated and poly-phosphorylated structures such as LPS. According to the present disclosure, the target binding peptide may be linked to superparamagnetic iron-based particles and a marked reduction of LPS may be measured upon immobilisation of the LPS-bound particles (**Figure 8**). In light of the present disclosure, the skilled person is also equipped with several experiments in order to assess whether a linked target binding peptide is capable of binding to a pathogen or pathogen component (**Figures 8-15**) and thereby reduce the concentration of pathogens and/or pathogen components. In a strongly preferred embodiment, the target binding peptide binds to a lipopolysaccharide (LPS), lipoteichoic acid (LTA) and/or heparansulfate, even more preferably wherein the target binding peptide binds to LPS and/or LTA.

The superparamagnetic iron-based particles and the target binding peptide are linked. Said linkage is sufficiently stable throughout the performance of the method to not break when the particles are used to immobilise e.g. a pathogen. The superparamagnetic iron-based particles and the target binding peptide may be linked by any suitable linkage to stably connect the superparamagnetic iron-based particles and the target binding peptide. Numerous suitable linkages are known in the art. The skilled person can also test whether the linkage is suitably stable by assessing the amount of linked peptide per iron-based particles using UV spectrometer absorption (280 nm). For example, the amount of peptide linked to the superparamagnetic iron-based particles was assessed in **Example 8.** Specifically, the superparamagnetic iron-based particles and the target binding peptide may be suitably linked when the concentration of target binding peptide before and after carrying out a method as disclosed herein reduces by at most 20%, preferably at most 17.5%, more preferably at most 15%, even more preferably at most 12.5%, even more preferably at most 10%. The target binding peptide concentration can, for example, be assessed by UV absorption. However, any other methods known in the art to assess a protein concentration may be used.

Furthermore, the linkage between the superparamagnetic iron-based particles and the target binding peptide may be covalent or non-covalent. In case the linkage is non-covalent, the linkage is preferred to be electrostatic. Said electrostatic interaction may be an ionic interaction, arising from an electrostatic attraction between two groups of opposite charge. For example, Zainol Abidin et al. (2019) shows that iron oxide-based nanoparticles bind phosphate from aqueous solutions with high affinity and most probably due to electrostatic interaction. Furthermore, said electrostatic interaction is pH-insensitive in a range from pH 2-13 making such electrostatic interactions equally suitable for linkage of the target binding peptide to the superparamagnetic iron-based particle as covalent linkages. In a particularly preferred embodiment, the superparamagnetic iron-based particles are bound to the target binding peptide via a bond between a phosphate group on the target binding peptide and the superparamagnetic iron-based particle, preferably the phosphate group is part of a phosphorylated C-terminal serine of the target binding peptide. Of course, other amino acids may also be phosphorylated. In particular, phosphorylated serines, threonines, tyrosines and histidines may be linked to the superparamagnetic iron-based nanoparticle. Phosphorylated amino acids are known in the art and can be commercially purchased (e.g. peptide SPP04-pS herein) has been purchased from GeneCast (or Proteogenix) . The inventors provide experimental data for said linkage of superparamagnetic iron-based particles to a target binding peptide in **Example 8.**

Advantageously, the superparamagnetic iron-based particles may be covalently linked to the target binding peptide via a connecting module. Said connecting module may be capable of covalently linking the superparamagnetic iron-based particles and the target binding peptide. For example, the connecting module may be selected from hydroxyapatite or a serine-derived aldehyde. In case the connecting module is a serine derived aldehyde, said serine-derived aldehyde may be at the terminus of the target binding peptide, preferably the serine-derived aldehyde may be at the C-terminus of the target binding peptide. In case the connecting module is hydroxyapatite, hydroxyapatite may link to an acidic amino acid (e.g. aspartic acid or glutamic acid) at a terminus of the target binding peptide, preferably the C-terminus.

The connecting module may consist of an 'anchor unit' and optionally a 'linker unit'. Thus, the connecting module may be formed of (i) an anchor unit or of (ii) an anchor and linker unit. It is preferred that the anchor unit is covalently linked to the superparamagnetic iron-based particle and to the target binding peptide or to the linker unit. In other words, the anchor unit is a molecule, which has been covalently anchored/linked to the superparamagnetic iron-based particle and the target binding peptide has been be covalently linked to the anchor unit - directly or via a linker unit. In case an anchor and a linker unit is present, the anchor and the linker unit are covalently linked, the anchor unit is covalently linked to the superparamagnetic iron-based particle, and the linker unit is covalently linked to the target binding peptide. In another preferred embodiment, the anchor unit is covalently linked to the superparamagnetic iron-based particle and the target binding peptide. A possible covalent interaction between the superparamagnetic iron-based particle and the anchor unit can be achieved by a silanization reaction. The present disclosure provides guidance on silanization reactions in **Example 1.** Furthermore, Liu et al. (2013) discloses a silanization reaction of superparamagnetic iron oxide nanoparticles and suitable reaction conditions. In a preferred embodiment, the anchor unit is a molecule comprising an amino group and a silane, which are covalently bound to the target binding peptide or linker unit and the superparamagnetic iron-based particles, respectively. Prior to anchoring the anchoring unit to the superparamagnetic iron-based particle, the anchoring unit is capable of covalently binding to the superparamagnetic iron-based particle and to the target binding peptide or the linker unit. It is even more preferred that the anchor unit is an aminosilane. Exemplary aminosilanes can be independently selected from (3-aminopropyl)-triethoxysilane (APTES), (3-aminopropyl)-diethoxy-methylsilane (APDEMS), (3-aminopropyl)-dimethyl-ethoxysilane (APDMES), and (3-aminopropyl)-trimethoxysilane (APTMS). In a strongly preferred embodiment, the aminosilane is (3-Aminopropyl)triethoxysilan (APTES). For example, Karawacka et al. 2018 discloses possible anchor and linker units.

In a strongly preferred embodiment, the connecting module is formed by an anchor unit and a linker unit. Said linker unit may be any linker unit suitable to enhance the covalent binding of target binding peptide to the anchor unit. In particular, said linker unit may be linked to the anchor unit and the target binding peptide by a covalent linkage. In an even stronger preferred embodiment, the linker unit is N-succinimidyl bromoacetate (SBA) or succinimidyl 3-(2-pyridyldithio)propionate) (SPDP). In an especially preferred embodiment linker unit is SBA. However, any suitable linker unit may be used to enhance the concentration of target binding peptide on the surface of superparamagnetic iron-based particles, e.g. thiol selective linkers such as thiol-maleimide (Northrop et al. 2015). The enhanced concentration of target binding peptide per amount of iron may be assess by UV absorption (280 nm). For example, **Figure 6** shows that linker unit, e.g. SBA, increases the concentration of peptide on the surface of superparamagnetic iron-based particles compared to superparamagnetic iron-based particles, which are linked to the target binding peptide via the anchor unit APTES or hydroxyapatite. In **Figure 6****,** it can be appreciated that the linker unit increased the concentration of peptide per amount of superparamagnetic iron-based particles. **Figure 6** shows that the concentration of target binding peptide bound to the surface of iron-based particles has been roughly tripled by introducing the linker unit. It is very strongly preferred that the connecting module comprises, and preferably consists of, the anchor unit APTES and the linker unit SBA.

"Immobilization", as used herein, means that something is made immobile. In the present case, immobilization occurs by the influence of a magnetic field. This means that the magnetically attractable particles are immobilised by the magnet field. In step (c), the superparamagnetic iron-based particles are immobilised in a magnetic field. A suitable magnetic field can be determined by the skilled person. For example, a suitable magnetic field is capable of immobilizing the superparamagnetic iron based particles. Immobilization can be assessed e.g. by assessing the iron concentration in the supernatant or flow through sample, e.g. by AES, as exemplary shown in **Figure 7**. Advantageously, the magnetic field applied in step (c) may be from 100 to 1200 mT, preferably from 150 to 800 mT, more preferably from 200 mT to 600 mT, even more preferably from 250 mT to 400 mT, and most preferably the magnetic field is around 310 mT.

Immobilization of the superparamagnetic iron-based particles increases with increasing time periods. In light of the present disclosure, and in particular **Figures 8-15**, the skilled person is able to determine suitable time periods for immobilization of the superparamagnetic iron-based particles. In a preferred embodiment, the superparamagnetic iron-based particles are immobilised in step (c) for a time period of at least 0.5 seconds, preferably at least 1 second, more preferably at least 2 seconds, even more preferably at least 3 seconds, more preferably at least 10 seconds, more preferably at least 20 seconds, more preferably at least 30 seconds, more preferably at least 45 seconds, more preferably at least 1 minute, even more preferably at least 2 minutes, and most preferably at least 3 minutes. It is also preferred that the superparamagnetic iron-based particles are immobilised in step (c) for a time period of at most 30 minutes, preferably at most 25 minutes, more preferably at most 20 minutes, more preferably at most 15 minutes, and even more preferably at most 10 minutes. Of course, the skilled person can freely combine the preferred time ranges for immobilization as disclosed herein. In a strongly preferred embodiment, the superparamagnetic iron-based particles are immobilised in step (c) for a time period from 15 seconds to 20 minutes, preferably from 30 seconds to 15 minutes, and more preferably from 1 minute to 10 minutes.

The method as disclosed herein may be carried out in a flow through set-up. A flow-through set-up may for example be set up like a dialysis system. It is preferred that the method is carried out using an extracorporeal blood-cleansing device. The general concept of extracorporeal blood-cleansing device has been disclosed in the art (e.g. Kang et al.(2014)). However, to the inventors knowledge, said extracorporeal blood-cleansing has not been carried out with the superparamagnetic iron-based particles linked to target binding peptides as disclosed herein and further defined in the claims. In a flow-through set up the flow speed may be at most 3 m/s, preferably at most 2.5 m/s, more preferably at most 2 m/s, even more preferably at most 1.5 m/s, even more preferably at most 1 m/s, even more preferably at most 0.9 m/s, even more preferably at most 0.8 m/s, even more preferably at most 0.7 m/s, even more preferably at most 0.6 m/s, even more preferably at most 0.5 m/s, even more preferably at most 0.4 m/s, even more preferably at most 0.35 m/s, even more preferably at most 0.3 m/s, even more preferably at most 0.25 m/s, even more preferably at most 0.2 m/s, even more preferably at most 0.15 m/s, even more preferably at most 0.1 m/s, even more preferably at most 0.09 m/s, even more preferably at most 0.08 m/s, even more preferably at most 0.07 m/s, and most preferably at most 0.06 m/s. Similarly, the flow speed may be at least 0.008 m/s, preferably at least 0.01 m/s, more preferably at least 0.02 m/s, even more preferably at least 0.025 m/s, even more preferably at least 0.03 m/s, even more preferably at least 0.035 m/s, even more preferably at least 0.04 m/s, even more preferably at least 0.045 m/s, even more preferably at least 0.05 m/s, and most preferably at least 0.055 m/s. Of course, the skilled person can freely combine any flow speed upper and/or lower limits freely. Flow speed can be assessed by standard methods in the art. For example, the flow speed can be assessed by determining volume output per time unit at a given tubing diameter.

The method as disclosed herein comprises superparamagnetic iron based particles linked to a target binding peptide. In a preferred embodiment, SPIONs are linked to a target binding peptide. Said SPIONs linked to a target binding peptide can show several characteristics when assessing the capability of reducing the concentration of pathogens and/or pathogen components in a sample. In a preferred embodiment, the SPIONs linked to a target binding peptide are capable of reducing the concentration of LPS in step (c) by at least 70%, preferably 80%, more preferably 90%, and even more preferably 95%, compared to the sample of step (a) comprising a LPS concentration of 10 EU/ml, wherein the reduction is determined after incubating the SPIONs linked to a target binding peptide at a concentration of 1 mg Fe/ml in Ringer solution followed by immobilising the SPIONs linked to a target binding peptide in a magnetic field of 0.31 Tesla for a time period of 30 seconds by using an assay based on the detection of recombinant factor C. For example, **Figure 8** demonstrates said characteristic with experimental data.

Furthermore, the SPIONs linked to a target binding peptide may be capable of reducing the concentration of LPS of step (c) by at least 60%, preferably at least 70%, more preferably at least 80%, and even more preferably at least 90%, compared to the sample of step (a) comprising a LPS concentration of 10 EU/ml, wherein the reduction is determined after incubating the SPIONs linked to a target binding peptide at a concentration of 1 mg Fe/ml in Ringer solution followed by immobilising the SPIONs linked to a target binding peptide in a magnetic field of 0.31 Tesla for a time period of 3 seconds by using an assay based on the detection of recombinant factor C. Exemplary **Figure 8** also supports this characteristic with experimental data.

In addition, the SPIONs linked to a target binding peptide may be capable of reducing the concentration of LPS of step (c) by at least 20%, preferably at least 30%, more preferably at least 35%, and even more preferably at least 40%, compared to the sample of step (a) comprising a LPS concentration of 10 EU/ml, wherein the reduction is determined after incubating the SPIONs linked to a target binding peptide at a concentration of 0.01 mg Fe/ml in Ringer solution followed by immobilising the SPIONs linked to a target binding peptide in a magnetic field of 0.31 Tesla for a time period of 30 seconds by using an assay based on the detection of recombinant factor C. **Figure 8** provides exemplary experimental data.

Equally, the SPIONs linked to a target binding peptide may be capable of reducing the concentration of LPS of step (c) by at least 20%, preferably at least 25%, even more preferably at least 30%, and even more preferably at least 35%, compared to the sample of step (a) comprising a LPS at a concentration of 10 EU/ml, wherein the reduction is determined after incubating the SPIONs linked to a target binding peptide at a concentration of 0.01 mg Fe/ml in Ringer solution followed by immobilising the SPIONs linked to a target binding peptide in a magnetic field of 0.31 Tesla for a time period of 3 seconds by using an assay based on the detection of recombinant factor C. **Figure 8** provides exemplary experimental data.

The SPIONs linked to a target binding peptide as disclosed herein may also be capable of reducing the concentration of pathogens, such as bacteria, viruses and/or fungi. In an embodiment, the SPIONs linked to a target binding peptide are capable of reducing the concentration of gram negative bacteria, e.g. *E.coli,* of step (c) by at least 20%, preferably at least 25%, even more preferably at least 30%, even more preferably at least 35% even more preferably at least 40%, even more preferably at least 45%, and even more preferably at least 50%, compared to the sample of step (a) comprising *Ecoli* at a concentration resulting in 650 colony forming units per ml of sample (CFU/ml), wherein the reduction is determined after incubating the SPIONs linked to a target binding peptide at a concentration of 0.1-1.0 mg Fe/ml in Ringer solution for a time period of 5 minutes followed by immobilising the SPIONs linked to a target binding peptide in a magnetic field of 0.31 Tesla for a time period of 3 min. For example, **Figure 11** provides experimental data with regard to said characteristic.

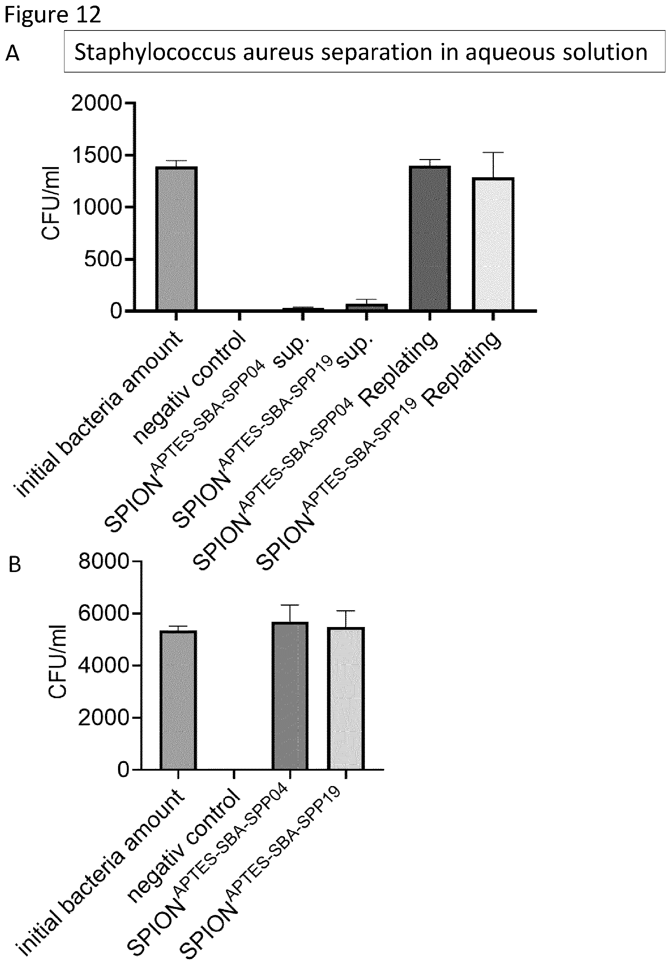

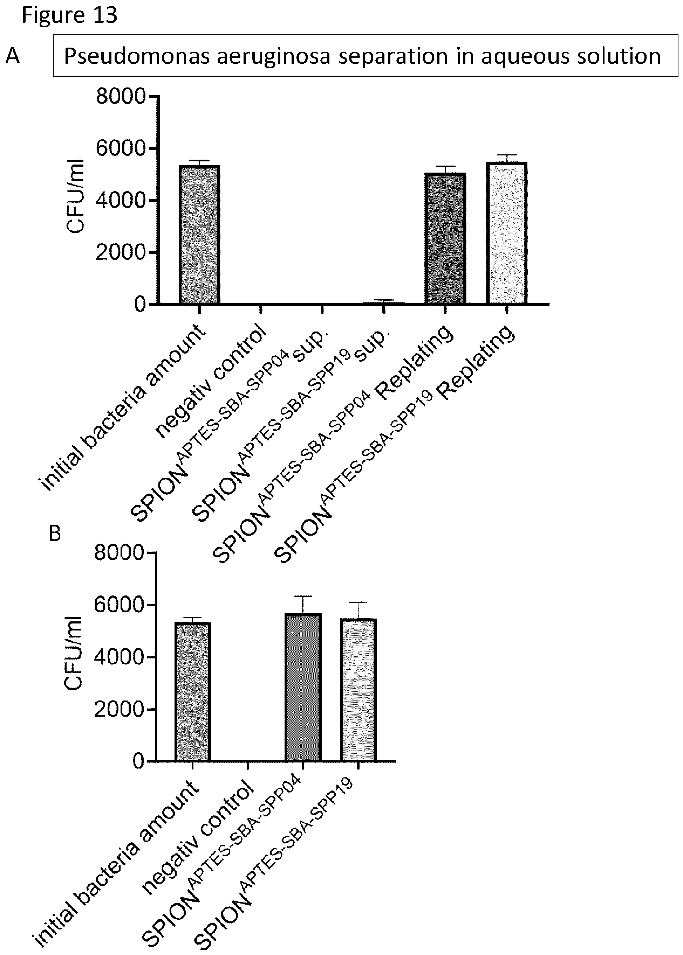

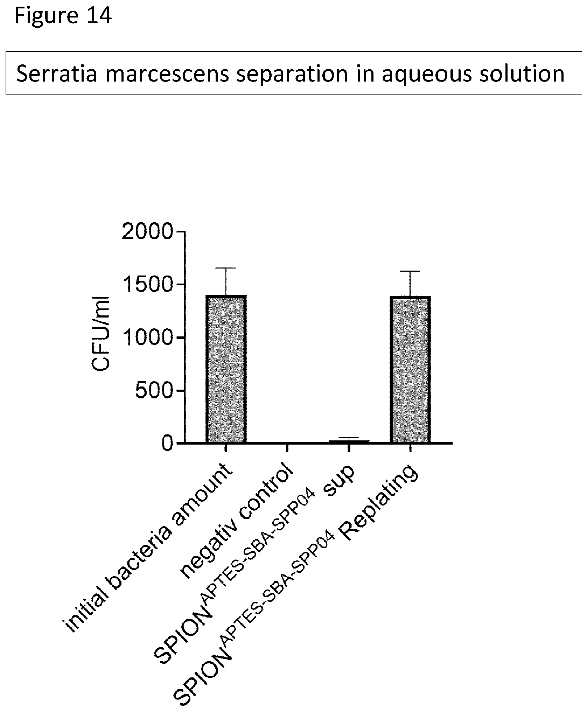

Advantageously, the SPIONs linked to a target binding peptide may be capable of reducing the concentration of gram positive bacteria, e.g. *S.aureus,* of step (c) by at least 50%, preferably at least 65%, even more preferably at least 70%, even more preferably at least 75% even more preferably at least 80%, even more preferably at least 85%, even more preferably at least 90%, and even more preferably at least 95%, compared to the sample of step (a) comprising *S.aureus* at a concentration resulting in around 1400 CFU/ml, wherein the reduction is determined after incubating the SPIONs linked to a target binding peptide at a concentration of 0.3 mg Fe/ml in Ringer solution for a time period of 5 minutes followed by immobilising the SPIONs linked to a target binding peptide in a magnetic field of 0.31 Tesla for a time period of 3 min. Exemplary **Figure 12** supports this characteristic of the SPIONs linked to a target binding peptide. Similarly, the SPIONs linked to a target binding peptide are also capable of markedly reducing the concentration of other pathogens, e.g. *Pseudomonas aeruginosa* or *Serratia marcescens,* as experimentally supported **Figures 13** and **14**, respectively.

Favourably, the SPIONs linked to a target binding peptide are capable of reducing the concentration of E.coli of step (c) by at least 20%, preferably at least 25%, even more preferably at least 30%, even more preferably at least 35% even more preferably at least 40%, even more preferably at least 45%, and even more preferably at least 50%, compared to the sample of step (a) comprising *E.coli* at a concentration resulting in around 1900 CFU/ml, wherein the reduction is determined after incubating the SPIONs linked to a target binding peptide at a concentration of 0.3 mg Fe/ml in human blood for a time period of 5 minutes followed by immobilising the SPIONs linked to a target binding peptide in a magnetic field of 0.31 Tesla for a time period of 3 min. Exemplary **Figure 15**, provides experimental data on said characteristic.

In another aspect, the present disclosure also describes a method for identifying pathogens in an aqueous or body fluid sample, the method comprising the steps of
a. providing the aqueous or body fluid sample;
b. incubating the sample with superparamagnetic iron-based particles, wherein the superparamagnetic iron-based particles are linked to a pathogen binding peptide;
c. immobilising the superparamagnetic iron-based particles with a magnetic field and thereby separating the pathogen-bound superparamagnetic iron-based particles from the sample; and
d. identifying the separated pathogens.

For the individual terms as used in said method, the definitions and embodiments as described above equally apply.

Specifically, the magnetic attractability of the particles as disclosed herein may be characterized by a reduction of the superparamagnetic iron-based particle concentration in Ringer solution by at least 65% when applying a magnetic field of 310 Millitesla for three minutes in static condition. Similarly to the static condition, the magnetic attractability can also be assessed in a flowing sample. In a preferred embodiment, the magnetic attractability is characterized by a reduction of the superparamagnetic iron-based particle concentration in water by at least 65% when applying a magnetic field of 0.8 Tesla at a flow speed of 0.057 m/s.

In a preferred embodiment the pathogens are concentrated in step (c) and thereby the identification of the separated pathogens is facilitated. For example, **Figure 16** shows the high degree of pathogen concentration demonstrated by a dense lawn of bacteria, when carrying out the method. Furthermore, the 're-plating experiments', as e.g. shown in **Figures 11-13****,** show that the pathogens can also be concentrated with the help of the superparamagnetic iron-based particles. Furthermore, said re-plating experiments show that the immobilised bacteria are still viable after binding and immobilisation. A concentration of the pathogens facilitates the identification of pathogens as a further amplification step of the pathogen may be omitted. Said amplification step would take time and also there are pathogens which cannot be easily amplified under laboratory conditions. Furthermore, this time saving may be valuable time for a patient. By identifying the pathogen faster, the patients may be able to be treated more quickly specifically, e.g. with a suitable specific anti-viral or a specific antibiotic. In other words, such a method has great diagnostic value.

Furthermore, an aqueous sample, such as drinking water, may be contaminated with a particular pathogen, such as a bacterium. Thus, an improved and/or faster identification of the pathogen may also be highly advantageous, as the aqueous sample may be specifically treated.

In a preferred embodiment, the separated pathogens are still viable, preferably the viability is determined by multiplying the pathogens under suitable conditions, more preferably by plating of the pathogens on a suitable growth plate, even more preferably wherein at least one colony forming unit (CFU) is obtained by plating.

It is also preferred that the pathogens are multiplied under suitable condition in an additional step after step (c) and before step (d). The multiplication has the advantage that the pathogens are present in an even higher concentration. Thus, the identification by standard methods, as described below, is facilitated. It is more preferred that the pathogens are plated on suitable growth plates. For example, the pathogens may be identified by methods, such as PCR, next generation sequencing, mass spectrometry, and/or ELISA. Of course a combination of techniques may also be used.

As already stated above, the definitions and the embodiments regarding the superparamagnetic iron-based particles, in particular the SPIONs, the magnetic attractability, the sample, the duration of step (b) and/or (c), the pathogens, and/or the magnetic field equally apply to all methods as disclosed herein. Particularly preferred embodiments are referred to in embodiment 60 below. Specifically, the pathogen binding peptide may show the same characteristics as the target binding peptide, wherein the target is a pathogen. All embodiments regarding the linkage between the superparamagnetic iron-based particle and the target binding peptide equally apply to the linkage between the superparamagnetic iron-based particle and the pathogen binding peptide.

For completeness, the pathogen binding peptide may have a sequence comprising 12-30 amino acids having one motif selected from SEQ ID NO: 5-8 or 27-47 , preferably the pathogen binding peptide may have a sequence comprising 12-30 amino acids having one motif selected from SEQ ID NO: 5-8, more preferably the pathogen binding peptide may have a sequence comprising 15-25 amino acids having one motif selected from SEQ ID Nos:5-8, more preferably the pathogen binding peptide may have a sequence comprising 16-20 amino acids having one motif selected from SEQ ID Nos: 5-8, more preferably the pathogen binding peptide may have a sequence comprising 17-19 amino acids having one motif selected from SEQ ID Nos: 5-8, even more preferably the pathogen binding peptide may have a sequence comprising 17-19 amino acids having the motif of SEQ ID NO:5, even more preferably the pathogen binding peptide may have a sequence comprising 17-19 amino acids having the motif of SEQ ID NO:5 and the motif is N-terminally preceded by 4-6 amino acids, most preferably the pathogen binding peptide may have a sequence comprising 17-19 amino acids having the motif of SEQ ID NO:5 and wherein the motif is N-terminally preceded by SEQ ID NO:9 (RCQGR). It is strongly preferred that the pathogen binding peptide shows at least 70% sequence identity to a sequence comprised within a scavenger receptor cysteine-rich (SRCR) protein domain, preferably the pathogen binding peptide shows at least 70% sequence identity to a sequence comprised within a SRCR protein domain of DMBT-1 (GP340), and more preferably the pathogen binding peptide is comprised within the SRCR protein domain of DMBT-1 (GP340).

In an embodiment, the pathogen binding peptide has a sequence comprising one motif selected from SEQ ID Nos: 5-8 or 27-47 and the pathogen binding peptide comprises a sequence having at least 60% identity to the sequence of SEQ ID NO: 1 or 2, preferably pathogen binding peptide has a sequence comprising one motif selected from SEQ ID Nos: 5-8 or 27-47 and the pathogen binding peptide comprises a sequence having at least 65% identity to the sequence of SEQ ID NO:1 or 2.

It is even more preferred that the pathogen binding peptide has a sequence comprising one motif selected from SEQ ID Nos: 5-8 or 27-47 and the pathogen binding peptide comprises a sequence having at least 70% identity to the sequence of SEQ ID NO:1 or 2, more preferably the pathogen binding peptide has a sequence comprising one motif selected from SEQ ID Nos: 5-8 or 27-47 and the pathogen binding peptide comprises a sequence having at least 75% identity to the sequence of SEQ ID NO:1 or 2, more preferably pathogen binding peptide has a sequence comprising one motif selected from SEQ ID Nos: 5-8 or 27-47 and the pathogen binding peptide comprises a sequence having at least 80% identity to the sequence of SEQ ID NO:1 or 2, more preferably pathogen binding peptide has a sequence comprising one motif selected from SEQ ID Nos: 5-8 or 27-47 and the pathogen binding peptide comprises a sequence having at least 85% identity to the sequence of SEQ ID NO:1 or 2, more preferably pathogen binding peptide has a sequence comprising one motif selected from SEQ ID Nos: 5-8 or 27-47 and the pathogen binding peptide comprises a sequence having at least 90% identity to the sequence of SEQ ID NO:1 or 2, and even more preferably pathogen binding peptide has a sequence comprising one motif selected from SEQ ID Nos: 5-8 or 27-47 and the pathogen binding peptide comprises a sequence having at least 95% identity to the sequence of SEQ ID NO:1 or 2. It is even more preferred that the pathogen binding peptide has a sequence comprising one motif selected from SEQ ID Nos: 5-8 and the pathogen binding peptide comprises a sequence having at least 60%, preferably 65%, more preferably 70%, even more preferably 75%, even more preferably 80%, even more preferably 85%, even more preferably 90% and most preferably at least 90% identity to the sequence of SEQ ID NO:1 or 2

The method for identifying pathogens may be carried out in a flow through set-up. In a preferred embodiment, the flow speed of such a flow though set-up may be independently selected from minimal and/or maximal flow speed as disclosed above.

In a further aspect, a use of superparamagnetic iron-based particles for reducing the concentration of pathogens, and/or pathogen components in an aqueous or body fluid sample is disclosed.

Of course, the definitions and the embodiments equally apply to the use as to the method for reducing the concentration of pathogens and/or pathogen components. In particular the definitions and embodiments regarding the superparamagnetic iron-based particles, in particular the SPIONs, the magnetic attractability, the sample, the pathogens and/or pathogen components, the target binding peptide, the linkage between the superparamagnetic iron-based particles and the target binding peptide and/or the magnetic field equally apply to the use as to the method. Particularly preferred embodiments are referred to in embodiment 69 below.

In another aspect, a use of superparamagnetic iron-based particles for identifying pathogens in a blood sample is disclosed, wherein the superparamagnetic iron-based particles are linked to a target binding peptide, wherein the target is a pathogen. Of course, the definitions and the embodiments equally apply to the use as to the method for identifying pathogens. In particular the definitions and embodiments regarding superparamagnetic iron-based particles, in particular the SPIONs, the magnetic attractability, the sample, the pathogens, and/or the magnetic field equally apply to the methods and the uses as disclosed herein. Specifically, the pathogen binding peptide may show the same characteristics as the target binding peptide, wherein the target is a pathogen. All embodiments regarding the linkage between the superparamagnetic iron-based particle and the target binding peptide equally apply to the linkage between the superparamagnetic iron-based particle and the pathogen binding peptide. Particularly preferred embodiments are referred to in embodiment 72 below.

Finally, superparamagnetic ironoxide nanoparticles (SPIONs) are disclosed herein, wherein the SPIONs are linked to a target binding peptide, wherein the target is a pathogen and/or a pathogen component. Of course, the definitions and the embodiments as part of a method also apply to the SPIONs. In particular the definitions and embodiments regarding the SPIONs, the magnetic attractability, the pathogens and/or pathogen components, the target binding peptide, the linkage between the superparamagnetic iron-based particles and/or the magnetic field equally apply to the SPIONs. In a very strongly preferred embodiment, the SPIONs are magnetically attractable and the magnetic attractability is defined as a reduction of the SPION concentration in water by 65% to 99.95% when applying a magnetic field of 0.31 Tesla for three minutes in static condition. For example **Figure 7** shows a side-by-side comparison between SPIONs described in the art and the SPIONs as disclosed herein showing a very significant improvement in attractability. Particularly preferred embodiments are referred to in embodiment 75 below.

The invention is further described by the following embodiments:
1. A method for reducing the concentration of pathogens and/or pathogen components in an aqueous or body fluid sample, the method comprising the steps of:
   a. providing the aqueous or body fluid sample;
   b. incubating the sample with superparamagnetic iron-based particles, wherein the superparamagnetic iron-based particles are linked to a target binding peptide wherein the target is a pathogen or a pathogen component; and
   c. immobilising the superparamagnetic iron-based particles with a magnetic field and thereby separating the pathogen-bound and/or pathogen component-bound superparamagnetic iron-based particles from the sample;
   whereby a reduced concentration of pathogens and/or pathogen components in the sample is obtained.
2. The method of embodiment 1, wherein the superparamagnetic iron-based particles are magnetically attractable and wherein the magnetic attractability is characterized by a reduction of the superparamagnetic iron-based particle concentration in Ringer solution by 65% to 99.95% when applying a magnetic field of 0.31 Tesla for three minutes in static condition.
3. The method of embodiments 1 or 2, wherein the superparamagnetic iron-based particles are magnetically attractable and wherein the magnetic attractability is characterized by a reduction of the superparamagnetic iron-based particle concentration in Ringer solution by 65% to 99.95% when applying a magnetic field of 0.8 Tesla at a flow speed of 0.057 m/s.
4. The method of embodiments 2 or 3, wherein the concentration of the superparamagnetic iron-based particles is reduced by 65% to 99.95%, preferably by 70% to 99.95%, more preferably by 75% to 99.95%, more preferably by 80% to 99.95%, more preferably by 90% to 99.95%, more preferably by 91% to 99.95%, more preferably by 92% to 99.95%, more preferably by 93% to 99.95%, more preferably by 94% to 99.95%, more preferably by 95% to 99.95%, more preferably by 96% to 99.95%, more preferably by 97% to 99.95%, more preferably by 98% to 99.95%, more preferably by 99% to 99.95%, more preferably by 99.1% to 99.95%, and even more preferably by 99.2% to 99.95%.
5. The method of any of the preceding embodiments, wherein the superparamagnetic iron-based particles are selected from and iron oxide, iron (Fe), iron-cobalt, alnico, permalloy particles.
6. The method of any of the preceding embodiments, wherein the superparamagnetic iron-based particles are superparamagnetic ironoxide nanoparticles (SPIONs).
7. The method of embodiment 6, wherein the SPIONs are magnetically attractable and wherein the magnetic attractability is characterized by a reduction of the SPION concentration from 0.01 to 12 ug Fe/ml when incubating the SPIONs at a concentration of 300 ug Fe/ml in water and applying 0.31 Tesla for three minutes in static condition.
8. The method of embodiments 6 or 7, wherein the SPIONs are magnetically attractable and wherein the magnetic attractability is characterized by a reduction of the SPION concentration from 0.01 to 12 ug Fe/ml when incubating the SPIONs at a concentration of 17.5 ug Fe/ml in water and applying 0.8 Tesla at a flow speed of 0.057 m/s.
9. The method of embodiment 7 or 8, wherein the SPION concentration is reduced to a concentration from 0.01 to 10 ug Fe/ml, preferably from 0.01 to 8 ug Fe/ml, preferably from 0.01 to 7 ug Fe/ml, more preferably from 0.01 to 6 ug Fe/ml, preferably from 0.01 to 5 ug Fe/ml, more preferably from 0.01 to 4 ug Fe/ml, and even more preferably from 0.01 to 3 ug Fe/ml.
10. The method of any of embodiments 2-9, wherein the concentration of the superparamagnetic iron-based particles is determined by atomic emission spectroscopy.
11. The method of any of the preceding embodiments, wherein the aqueous or body fluid sample is a body fluid, waste water, ground water, or drinking water, preferably wherein the body fluid sample is a human body fluid, more preferably wherein the human body fluid is selected from blood, serum, plasma, lymph, urine, liquor, saliva and sputum, even more preferably wherein the human body fluid is blood or serum, even more preferably wherein the human body fluid is blood, and most preferably wherein the blood is from a septic patient.
12. The method of any of the preceding embodiments, wherein the sample is a blood sample, preferably wherein the blood sample is a mammalian blood sample, more preferably a primate blood sample, and even more preferably a human blood sample.
13. The method of any of preceding embodiments, wherein the concentration of pathogens, and/or pathogen components in the sample of step (c) is reduced by at least 20%, preferably at least 25%, more preferably at least 30%, more preferably at least 35%, even more preferably at least 40%, even more preferably at least 45%, and even more preferably at least 50%, compared to the sample of step (a).
14. The method of any of embodiments 6-13, wherein the sample is incubated in step (b) with a concentration from 0.002 mg Fe/ml to 5 mg Fe/ml superparamagnetic iron-based particles per volume of sample, preferably from 0.004 mg Fe/ml to 3 mg Fe/ml superparamagnetic iron-based particles, more preferably from 0.007 mg Fe/ml to 2 mg Fe/ml superparamagnetic iron-based particles, even more preferably from 0.01 mg Fe/ml to 1 mg Fe/ml superparamagnetic iron-based particles, and even more preferably from 0.01 mg Fe/ml to 0.15625 mg Fe/ml superparamagnetic iron-based particles.
15. The method of any of the preceding embodiments, wherein the sample is incubated in step (b) for a time period of at least 0.5 seconds, preferably at least 1 second, more preferably at least 2 seconds, more preferably at least 3 seconds, more preferably at least 10 seconds, more preferably at least 30 seconds, more preferably at least 1 minute, more preferably at least 2 minutes, more preferably at least 3 minutes, even more preferably at least 4 minutes, and most preferably for about 5 minutes.
16. The method of any of the preceding embodiments, wherein the sample is incubated in step (b) for a time period of at most 1 hour, preferably at most 45 minutes, more preferably at most 35 minutes, more preferably at most 30 minutes, more preferably at most 20 minutes, more preferably at most 15 minutes, more preferably at most 10 minutes, more preferably at most 7.5 minutes, and most preferably for about 5 minutes.
17. The method of any of the preceding embodiments, wherein the sample is incubated in step (b) for a time period from 0.5 second to 45 minutes, preferably from 1 second to 30 minutes, and more preferably from 3 second to 30 minutes.
18. The method of any of the preceding embodiments, wherein the pathogens comprise bacteria, fungi and/or viruses, preferably wherein the pathogens are bacteria.
19. The method of any of the preceding embodiments, wherein the pathogens comprise fungi, preferably Candida spp.
20. The method of any of the preceding embodiments, wherein the pathogens comprise gram-negative and/or a gram-positive bacteria, preferably consist of gram-negative and/or a gram-positive bacteria.
21. The method of embodiment 20, wherein the gram-negative bacteria are selected from one or more of *Escherichia coli, Pseudomonas spp., Klebsiella* spp., preferably wherein the gram-negative bacteria are selected from *Escherichia coli (E.coli) and Pseudomonas aeruginosa (P. aeruginosa),* and more preferably wherein the gram-negative bacteria are *E.coli.*
22. The method of any of embodiments 18, 20 or 21, wherein the gram-positive bacteria are selected from one or more of *Staphylococcus aureus, Serratia, Streptococcus spp., Listeria monocytogenes, Clostridium difficile, Enterobacter,* and preferably wherein the gram-positive bacteria is *Staphylococcus aureus* or *Serratia,* and most preferably wherein the gram-positive bacteria is *Staphylococcus aureus.*
23. The method of any of the preceding embodiments, wherein the pathogen components comprise endotoxins and/or pathogenic cell wall components.
24. The method of embodiment 23, wherein the endotoxins are derived from gram-negative bacteria, more preferably wherein the endotoxins are derived from of one or more of *Klebsiella* spp., *Escherichia coli (E.coli),* and *Pseudomonas aeruginosa,* more preferably wherein the endotoxins are LPS, more preferably wherein the endotoxins comprise *E.coli* LPS, and more preferably wherein LPS is selected from one or more of LPS O55:B5, LPS O26:B6, and LPS O111:B4.
25. The method of embodiment 23 or 24, wherein the pathogenic cell wall component is derived from gram-positive bacteria or fungi, preferably wherein the cell wall component is derived from gram-positive bacteria, more preferably wherein the cell wall component is LTA, teichoic acid and/or a peptidoglycan, even more preferably wherein the pathogenic cell wall component is LTA, even more preferably wherein the pathogenic cell wall component is LTA of *Staphylococcus aureus* and/or *Streptococcus pyogenes.*
26. The method of any of the preceding embodiments, wherein the target binding peptide has a sequence comprising 12-30 amino acids having one motif selected from SEQ ID NO: 5 (VEVLxxxxW), SEQ ID NO: 6 (VEILxxxxW), SEQ ID NO: 7 (VEIYxxxxW), SEQ ID NO:8 (VEVYxxxxW), SEQ ID NO: 27 (VEVLxQ), SEQ ID NO: 28 (VEIFxxxxDSSL), SEQ ID NO: 29 (VVVLxxxxW), SEQ ID NO: 30 (VEVRxxxxW), SEQ ID NO: 31 (VEISxxxxW), SEQ ID NO: 32 (VGVLxxxxW), SEQ ID NO: 33 (VEVSxxxxW), SEQ ID NO: 34 (VEVFxxxxW), SEQ ID NO 35 (VELRxxxxW), SEQ ID NO: 36 (VELFxxxxW), SEQ ID NO: 37 (VEVHxxxxW), SEQ ID NO: 38 (VELFxxxxW), SEQ ID NO: 39 (VEILxxxxW), SEQ ID NO: 40 (VEIFxxxxW, SEQ ID NO: 41 (VEFFxxxxW), SEQ ID NO: 42 (VELHxxxxW), SEQ ID NO: 43 (LEVRxxxxW), SEQ ID NO: 44 (VEVQxxxxW), SEQ ID NO: 45 (VELLxxxxW), SEQ ID NO: 46 (VELYxxxxPPAL), and SEQ ID NO: 47 (LEVFxxxxW), preferably wherein the target binding peptide has a sequence comprising 12-30 amino acids having one motif selected from SEQ ID NO: 5-8, more preferably wherein the target binding peptide has a sequence comprising 15-25 amino acids having one motif selected from SEQ ID Nos: 5-8, more preferably wherein the target binding peptide has a sequence comprising 16-20 amino acids having one motif selected from SEQ ID Nos: 5-8, more preferably wherein the target binding peptide has a sequence comprising 17-19 amino acids having one motif selected from SEQ ID Nos: 5-8, even more preferably wherein the target binding peptide has a sequence comprising 17-19 amino acids having the motif selected from SEQ ID NO:5, even more preferably wherein the target binding peptide has a sequence comprising 17-19 amino acids having the motif selected from SEQ ID NO:5 and wherein the motif is N-terminally preceded by 4-6 amino acids, most preferably wherein the target binding peptide has a sequence comprising 17-19 amino acids having the motif selected from SEQ ID NO:5 and wherein the motif is N-terminally preceded by SEQ ID NO:9 (RCQGR).
27. The method of any of the preceding embodiments, wherein the target binding peptide shows at least 70% sequence identity to a sequence comprised within a scavenger receptor cysteine-rich (SRCR) protein domain,
   preferably wherein the target binding peptide shows at least 70% sequence identity to a sequence comprised within a SRCR protein domain of DMBT-1 (GP340), and
   more preferably wherein the target binding peptide is comprised within the SRCR protein domain of DMBT-1 (GP340).
28. The method of any of the preceding embodiments, wherein the target binding peptide has a sequence comprising one motif selected from SEQ ID Nos: 5-8 or 27-47 and wherein the target binding peptide comprises a sequence having at least 60% identity to the sequence of SEQ ID NO:1, preferably wherein the target binding peptide has a sequence comprising one motif selected from SEQ ID Nos: 5-8 or 27-47 and wherein the target binding peptide comprises a sequence having at least 65% identity to the sequence of SEQ ID NO:1, more preferably wherein the target binding peptide has a sequence comprising one motif selected from SEQ ID Nos: 5-8 or 27-47 and wherein the target binding peptide comprises a sequence having at least 70% identity to the sequence of SEQ ID NO:1, more preferably wherein the target binding peptide has a sequence comprising one motif selected from SEQ ID Nos: 5-8 or 27-47 and wherein the target binding peptide comprises a sequence having at least 75% identity to the sequence of SEQ ID NO:1, more preferably wherein the target binding peptide has a sequence comprising one motif selected from SEQ ID Nos: 5-8 or 27-47 and wherein the target binding peptide comprises a sequence having at least 80% identity to the sequence of SEQ ID NO:1, more preferably wherein the target binding peptide has a sequence comprising one motif selected from SEQ ID Nos: 5-8 or 27-47 and wherein the target binding peptide comprises a sequence having at least 85% identity to the sequence of SEQ ID NO:1, more preferably wherein the target binding peptide has a sequence comprising one motif selected from SEQ ID Nos: 5-8 or 27-47 and wherein the target binding peptide comprises a sequence having at least 90% identity to the sequence of SEQ ID NO:1, and even more preferably wherein the target binding peptide has a sequence comprising one motif selected from SEQ ID Nos: 5-8 or 27-47 and wherein the target binding peptide comprises a sequence having at least 95% identity to the sequence of SEQ ID NO:1.
29. The method of any of the preceding embodiments, wherein the target binding peptide has a sequence comprising one motif selected from SEQ ID Nos: 5-8 or 27-47 and wherein the target binding peptide comprises a sequence having at least 60% identity to the sequence of SEQ ID NO:2, preferably wherein the target binding peptide has a sequence comprising one motif selected from SEQ ID Nos: 5-8 or 27-47 and wherein the target binding peptide comprises a sequence having at least 65% identity to the sequence of SEQ ID NO:2, more preferably wherein the target binding peptide has a sequence comprising one motif selected from SEQ ID Nos: 5-8 or 27-47 and wherein the target binding peptide comprises a sequence having at least 70% identity to the sequence of SEQ ID NO:2, more preferably wherein the target binding peptide has a sequence comprising one motif selected from SEQ ID Nos: 5-8 or 27-47 and wherein the target binding peptide comprises a sequence having at least 75% identity to the sequence of SEQ ID NO:2, more preferably wherein the target binding peptide has a sequence comprising one motif selected from SEQ ID Nos: 5-8 or 27-47 and wherein the target binding peptide comprises a sequence having at least 80% identity to the sequence of SEQ ID NO:2, more preferably wherein the target binding peptide has a sequence comprising one motif selected from SEQ ID Nos: 5-8 or 27-47 and wherein the target binding peptide comprises a sequence having at least 85% identity to the sequence of SEQ ID NO:2, more preferably wherein the target binding peptide has a sequence comprising one motif selected from SEQ ID Nos: 5-8 or 27-47 and wherein the target binding peptide comprises a sequence having at least 90% identity to the sequence of SEQ ID NO:2, and even more preferably wherein the target binding peptide has a sequence comprising one motif selected from SEQ ID Nos: 5-8 or 27-47 and wherein the target binding peptide comprises a sequence having at least 95% identity to the sequence of SEQ ID NO:2.
30. The method of any of the preceding embodiments, wherein the target binding peptide is a pathogen component binding peptide, preferably wherein the target binding peptide binds to a polysulfated and/or polyphosphorylated ligands located on the surface of the pathogen, and more preferably wherein the target binding peptide binds to a lipopolysaccharide (LPS), lipoteichoic acid (LTA) and/or heparansulfate, even more preferably wherein the target binding peptide binds to LPS and/or LTA.
31. The method of any of the preceding embodiments, wherein the superparamagnetic iron-based particles are superparamagnetic ironoxide nanoparticles (SPIONs), more preferably wherein the iron oxide is selected from Fe₃O₄, γ-Fe₂O₃, CoFe₂O₄ or a mixture thereof, more preferably wherein the iron oxide is Fe₃O₄.
32. The method of any of the preceding embodiments, wherein the superparamagnetic iron-based particles are linked to the target binding peptide covalently or non-covalently, preferably wherein the superparamagnetic iron-based particles are covalently linked to the target binding peptide via a connecting module; or preferably wherein the superparamagnetic iron-based particles are bound to the target binding peptide via a bond between a phosphate group on the target binding peptide and the superparamagnetic iron-based particle, preferably wherein the phosphate group is part of a phosphorylated C-terminal serine of the target binding peptide.
33. The method of embodiment 32, wherein the connecting module is hydroxyapatite or a serine-derived aldehyde at the terminus of the target binding peptide, and preferably wherein hydroxyapatite is covalently linked to an acidic amino acid at a terminus of the target binding peptide.
34. The method of embodiment 32, wherein the connecting module consists of an anchor unit and optionally a linker unit, preferably wherein the anchor unit is covalently linked to the superparamagnetic iron-based particle and the linker unit is covalently linked to the pathogen binding peptide; or preferably wherein the anchor unit is covalently linked to the superparamagnetic iron-based particle and the pathogen binding peptide.
35. The method of embodiment 34, wherein the anchor unit wherein the anchor unit is capable of covalently linking the superparamagnetic iron-based particle to the target binding peptide or the linker unit, preferably wherein the anchor unit is a molecule comprising an amino group and a silane, more preferably wherein the anchor unit is an aminosilane, more preferably wherein the aminosilane is (3-aminopropyl)-triethoxysilane (APTES), (3-aminopropyl)-diethoxy-methylsilane (APDEMS), (3-aminopropyl)-dimethyl-ethoxysilane (APDMES), or (3-aminopropyl)-trimethoxysilane (APTMS), even more preferably wherein the aminosilane is (3-Aminopropyl)triethoxysilan (APTES).
36. The method of embodiment 34 or 35, wherein the linker unit is capable of covalently linking the anchor unit to the target binding peptide, preferably wherein the linker unit is N-succinimidyl bromoacetate (SBA) or succinimidyl 3-(2-pyridyldithio)propionate) (SPDP), and preferably wherein the linker is SBA.
37. The method of any of embodiments 34-36, wherein the connecting module comprises, and preferably consists of, the anchor unit APTES and the linker unit SBA.
38. The method of any of the preceding embodiments, wherein the magnetic field applied in step (c) is from 100 to 1200 mT, preferably wherein the magnetic field is from 150 to 800 mT, more preferably wherein the magnetic field is from 200 mT to 600 mT, even more preferably wherein the magnetic field is from 250 mT to 400 mT, and most preferably wherein the magnetic field is around 310 mT.
39. The method of any of the preceding embodiments, wherein the superparamagnetic iron-based particles are immobilised in step (c) for a time period of at least 0.5 seconds, preferably at least 1 second, more preferably at least 2 seconds, even more preferably at least 3 seconds, more preferably at least 10 seconds, more preferably at least 20 seconds, more preferably at least 30 seconds, more preferably at least 45 seconds, more preferably at least 1 minute, even more preferably at least 2 minutes, and most preferably at least 3 minutes.
40. The method of any of the preceding embodiments, wherein the superparamagnetic iron-based particles are immobilised in step (c) for a time period of at most 30 minutes, preferably at most 25 minutes, more preferably at most 20 minutes, more preferably at most 15 minutes, and even more preferably at most 10 minutes.
41. The method of any of the preceding embodiments, wherein the superparamagnetic iron-based particles are immobilised in step (c) for a time period from 15 seconds to 20 minutes, preferably from 30 seconds to 15 minutes, and more preferably from 1 minute to 10 minutes.
42. The method of any of the preceding embodiments, wherein the method is carried out in a flow through set-up.
43. The method of embodiment 42, wherein the flow speed is at most 3 m/s, preferably at most 2.5 m/s, more preferably at most 2 m/s, even more preferably at most 1.5 m/s, even more preferably at most 1 m/s, even more preferably at most 0.9 m/s, even more preferably at most 0.8 m/s, even more preferably at most 0.7 m/s, even more preferably at most 0.6 m/s, even more preferably at most 0.5 m/s, even more preferably at most 0.4 m/s, even more preferably at most 0.35 m/s, even more preferably at most 0.3 m/s, even more preferably at most 0.25 m/s, even more preferably at most 0.2 m/s, even more preferably at most 0.15 m/s, even more preferably at most 0.1 m/s, even more preferably at most 0.09 m/s, even more preferably at most 0.08 m/s, even more preferably at most 0.07 m/s, and most preferably at most 0.06 m/s.
44. The method of embodiment 42 or 43, wherein the flow speed is at least 0.008 m/s, preferably at least 0.01 m/s, more preferably at least 0.02 m/s, even more preferably at least 0.025 m/s, even more preferably at least 0.03 m/s, even more preferably at least 0.035 m/s, even more preferably at least 0.04 m/s, even more preferably at least 0.045 m/s, even more preferably at least 0.05 m/s, and most preferably at least 0.055 m/s.
45. The method of any of the preceding embodiments, wherein the method is carried out using an extracorporeal blood-cleansing device.
46. The method of any of embodiments 6-45, wherein the SPIONs linked to a target binding peptide are capable of reducing the concentration of LPS in step (c) by at least 70%, preferably 80%, more preferably 90%, and even more preferably 95%, compared to the sample of step (a) comprising a LPS concentration of 10 EU/ml, wherein the reduction is determined after incubating the SPIONs linked to a target binding peptide at a concentration of 1 mg Fe/ml in Ringer solution followed by immobilising the SPIONs linked to a target binding peptide in a magnetic field of 0.31 Tesla for a time period of 30 seconds by using an assay based on the detection of recombinant factor C.
47. The method of any of embodiments 6-46, wherein the SPIONs linked to a target binding peptide are capable of reducing the concentration of LPS of step (c) by at least 60%, preferably at least 70%, more preferably at least 80%, and even more preferably at least 90%, compared to the sample of step (a) comprising a LPS concentration of 10 EU/ml, wherein the reduction is determined after incubating the SPIONs linked to a target binding peptide at a concentration of 1 mg Fe/ml in Ringer solution followed by immobilising the SPIONs linked to a target binding peptide in a magnetic field of 0.31 Tesla for a time period of 3 seconds by using an assay based on the detection of recombinant factor C.
48. The method of any of embodiments 6-47, wherein the SPIONs linked to a target binding peptide are capable of reducing the concentration of LPS of step (c) by at least 20%, preferably at least 30%, more preferably at least 35%, and even more preferably at least 40%, compared to the sample of step (a) comprising a LPS concentration of 10 EU/ml, wherein the reduction is determined after incubating the SPIONs linked to a target binding peptide at a concentration of 0.01 mg Fe/ml in Ringer solution followed by immobilising the SPIONs linked to a target binding peptide in a magnetic field of 0.31 Tesla for a time period of 30 seconds by using an assay based on the detection of recombinant factor C.
49. The method of any of embodiments 6-48, wherein the SPIONs linked to a target binding peptide are capable of reducing the concentration of LPS of step (c) by at least 20%, preferably at least 25%, even more preferably at least 30%, and even more preferably at least 35%, compared to the sample of step (a) comprising a LPS at a concentration of 10 EU/ml, wherein the reduction is determined after incubating the SPIONs linked to a target binding peptide at a concentration of 0.01 mg Fe/ml in Ringer solution followed by immobilising the SPIONs linked to a target binding peptide in a magnetic field of 0.31 Tesla for a time period of 3 seconds by using an assay based on the detection of recombinant factor C.
50. The method of any of embodiments 6-49, wherein the SPIONs linked to a target binding peptide are capable of reducing the concentration of E.coli of step (c) by at least 20%, preferably at least 25%, even more preferably at least 30%, even more preferably at least 35% even more preferably at least 40%, even more preferably at least 45%, and even more preferably at least 50%, compared to the sample of step (a) comprising *E.coli* at a concentration resulting in 650 colony forming units per ml of sample (CFU/ml), wherein the reduction is determined after incubating the SPIONs linked to a target binding peptide at a concentration of 0.1-1.0 mg Fe/ml in Ringer solution for a time period of 5 minutes followed by immobilising the SPIONs linked to a target binding peptide in a magnetic field of 0.31 Tesla for a time period of 3 min.
51. The method of any of embodiments 6-50, wherein the SPIONs linked to a target binding peptide are capable of reducing the concentration of *S.aureus* of step (c) by at least 50%, preferably at least 65%, even more preferably at least 70%, even more preferably at least 75% even more preferably at least 80%, even more preferably at least 85%, even more preferably at least 90%, and even more preferably at least 95%, compared to the sample of step (a) comprising *S.aureus* at a concentration resulting in around 1400 CFU/ml, wherein the reduction is determined after incubating the SPIONs linked to a target binding peptide at a concentration of 0.3 mg Fe/ml in Ringer solution for a time period of 5 minutes followed by immobilising the SPIONs linked to a target binding peptide in a magnetic field of 0.31 Tesla for a time period of 3 min.
52. The method of any of embodiments 6-51, wherein the SPIONs linked to a target binding peptide are capable of reducing the concentration of *E.coli* of step (c) by at least 20%, preferably at least 25%, even more preferably at least 30%, even more preferably at least 35% even more preferably at least 40%, even more preferably at least 45%, and even more preferably at least 50%, compared to the sample of step (a) comprising *E.coli* at a concentration resulting in around 1900 CFU/ml, wherein the reduction is determined after incubating the SPIONs linked to a target binding peptide at a concentration of 0.3 mg Fe/ml in human blood for a time period of 5 minutes followed by immobilising the SPIONs linked to a target binding peptide in a magnetic field of 0.31 Tesla for a time period of 3 min.
53. A method for identifying pathogens in an aqueous or body fluid sample, the method comprising the steps of
   a. providing the aqueous or body fluid sample;
   b. incubating the sample with superparamagnetic iron-based particles, wherein the superparamagnetic iron-based particles are linked to a pathogen binding peptide;
   c. immobilising the superparamagnetic iron-based particles with a magnetic field and thereby separating the pathogen-bound superparamagnetic iron-based particles from the sample; and
   d. identifying the separated pathogens.
54. The method of embodiment 54, wherein the superparamagnetic iron-based particles are magnetically attractable and wherein the magnetic attractability is characterized by a reduction of the superparamagnetic iron-based particle concentration in Ringer solution by 65% to 99.95% when applying a magnetic field of 0.31 Tesla for three minutes in static condition.
55. The method of embodiments 54 or 55, wherein the superparamagnetic iron-based particles are magnetically attractable and wherein the magnetic attractability is characterized by a reduction of the superparamagnetic iron-based particle concentration in Ringer solution by 65% to 99.95% when applying a magnetic field of 0.8 Tesla at a flow speed of 0.057 m/s.
56. The method of any of embodiments 53-55, wherein the pathogens are concentrated in step (c) and thereby the identification of the separated pathogens is facilitated.
57. The method of any of embodiments 53-56, wherein the separated pathogens are still viable, preferably wherein the viability is determined by multiplying the pathogens under suitable conditions, more preferably by plating of the pathogens on a suitable growth plate, and even more preferably wherein at least one colony forming unit (CFU) is obtained by plating.
58. The method of any of embodiments 53-57, wherein the pathogens are multiplied under suitable condition in an additional step after step (c) and before step (d), preferably wherein the pathogen are plated on suitable growth plates.
59. The method of any of embodiments 53-58, wherein the separated pathogen of step (d) are identified by a method selected from one or more of PCR, next generation sequencing, mass spectrometry, and ELISA.
60. The method of any of embodiments 53-59, wherein the superparamagnetic iron-based particles are further defined any of embodiments 2-9, 31-37 and 46-52, the sample is further defined in any of embodiments 11 or 12, the duration of step (b) is further defined in any of embodiments 15-17, the pathogens are further defined in any of embodiments 18-22, the duration of step (c) is further defined in any of embodiments 39-41, and/or the magnetic field is further defined in embodiment 38.
61. The method of any of embodiments 53-60, wherein the pathogen binding peptide has a sequence comprising 12-30 amino acids having one motif selected from SEQ ID NO: 5 (VEVLxxxxW), SEQ ID NO: 6 (VEILxxxxW), SEQ ID NO: 7 (VEIYxxxxW), SEQ ID NO:8 (VEVYxxxxW), SEQ ID NO: 27 (VEVLxQ), SEQ ID NO: 28 (VEIFxxxxDSSL), SEQ ID NO: 29 (VVVLxxxxW), SEQ ID NO: 30 (VEVRxxxxW), SEQ ID NO: 31 (VEISxxxxW), SEQ ID NO: 32 (VGVLxxxxW), SEQ ID NO: 33 (VEVSxxxxW), SEQ ID NO: 34 (VEVFxxxxW), SEQ ID NO 35 (VELRxxxxW), SEQ ID NO: 36 (VELFxxxxW), SEQ ID NO: 37 (VEVHxxxxW), SEQ ID NO: 38 (VELFxxxxW), SEQ ID NO: 39 (VEILxxxxW), SEQ ID NO: 40 (VEIFxxxxW, SEQ ID NO: 41 (VEFFxxxxW), SEQ ID NO: 42 (VELHxxxxW), SEQ ID NO: 43 (LEVRxxxxW), SEQ ID NO: 44 (VEVQxxxxW), SEQ ID NO: 45 (VELLxxxxW), SEQ ID NO: 46 (VELYxxxxPPAL) and SEQ ID NO: 47 (LEVFxxxxW), preferably wherein the target binding peptide has a sequence comprising 12-30 amino acids having one motif selected from SEQ ID NO: 5-8, more preferably wherein the pathogen binding peptide has a sequence comprising 15-25 amino acids having one motif selected from SEQ ID Nos:5-8, more preferably wherein the pathogen binding peptide has a sequence comprising 16-20 amino acids having one motif selected from SEQ ID Nos: 5-8, more preferably wherein the pathogen binding peptide has a sequence comprising 17-19 amino acids having one motif selected from SEQ ID Nos: 5-8, even more preferably wherein the pathogen binding peptide has a sequence comprising 17-19 amino acids having the motif of SEQ ID NO:5, even more preferably wherein the pathogen binding peptide has a sequence comprising 17-19 amino acids having the motif of SEQ ID NO:5 and wherein the motif is N-terminally preceded by 4-6 amino acids, most preferably wherein the pathogen binding peptide has a sequence comprising 17-19 amino acids having the motif of SEQ ID NO:5 and wherein the motif is N-terminally preceded by SEQ ID NO:9 (RCQGR).
62. The method of any of embodiments 53-61, wherein the pathogen binding peptide shows at least 70% sequence identity to a sequence comprised within a scavenger receptor cysteine-rich (SRCR) protein domain,
   preferably wherein the pathogen binding peptide shows at least 70% sequence identity to a sequence comprised within a SRCR protein domain of DMBT-1 (GP340), and more preferably wherein the pathogen binding peptide is comprised within the SRCR protein domain of DMBT-1 (GP340).
63. The method of any of embodiments 53-62, wherein the pathogen binding peptide has a sequence comprising one motif selected from SEQ ID Nos: 5-8 or 27-47 and wherein the pathogen binding peptide comprises a sequence having at least 60% identity to the sequence of SEQ ID NO:1, preferably wherein the pathogen binding peptide has a sequence comprising one motif selected from SEQ ID Nos: 5-8 or 27-47and wherein the pathogen binding peptide comprises a sequence having at least 65% identity to the sequence of SEQ ID NO:1, more preferably wherein the pathogen binding peptide has a sequence comprising one motif selected from SEQ ID Nos: 5-8 or 27-47 and wherein the pathogen binding peptide comprises a sequence having at least 70% identity to the sequence of SEQ ID NO:1, more preferably wherein the pathogen binding peptide has a sequence comprising one motif selected from SEQ ID Nos: 5-8 or 27-47 and wherein the pathogen binding peptide comprises a sequence having at least 75% identity to the sequence of SEQ ID NO:1, more preferably wherein the pathogen binding peptide has a sequence comprising one motif selected from SEQ ID Nos: 5-8 or 27-47 and wherein the pathogen binding peptide comprises a sequence having at least 80% identity to the sequence of SEQ ID NO:1, more preferably wherein the pathogen binding peptide has a sequence comprising one motif selected from SEQ ID Nos: 5-8 or 27-47 and wherein the pathogen binding peptide comprises a sequence having at least 85% identity to the sequence of SEQ ID NO:1, more preferably wherein the pathogen binding peptide has a sequence comprising one motif selected from SEQ ID Nos: 5-8 or 27-47 and wherein the pathogen binding peptide comprises a sequence having at least 90% identity to the sequence of SEQ ID NO:1, and even more preferably wherein the pathogen binding peptide has a sequence comprising one motif selected from SEQ ID Nos: 5-8 or 27-47 and wherein the pathogen binding peptide comprises a sequence having at least 95% identity to the sequence of SEQ ID NO:1.
64. The method of any of embodiments 53-63, wherein the pathogen binding peptide has a sequence comprising one motif selected from SEQ ID Nos: 5-8 or 27-47 and wherein the pathogen binding peptide comprises a sequence having at least 60% identity to the sequence of SEQ ID NO:2, preferably wherein the pathogen binding peptide has a sequence comprising one motif selected from SEQ ID Nos: 5-8 or 27-47 and wherein the pathogen binding peptide comprises a sequence having at least 65% identity to the sequence of SEQ ID NO:2, more preferably wherein the pathogen binding peptide has a sequence comprising one motif selected from SEQ ID Nos: 5-8 or 27-47 and wherein the pathogen binding peptide comprises a sequence having at least 70% identity to the sequence of SEQ ID NO:2, more preferably wherein the pathogen binding peptide has a sequence comprising one motif selected from SEQ ID Nos: 5-8 or 27-47 and wherein the pathogen binding peptide comprises a sequence having at least 75% identity to the sequence of SEQ ID NO:2, more preferably wherein the pathogen binding peptide has a sequence comprising one motif selected from SEQ ID Nos: 5-8 or 27-47 and wherein the pathogen binding peptide comprises a sequence having at least 80% identity to the sequence of SEQ ID NO:2, more preferably wherein the pathogen binding peptide has a sequence comprising one motif selected from SEQ ID Nos: 5-8 or 27-47 and wherein the pathogen binding peptide comprises a sequence having at least 85% identity to the sequence of SEQ ID NO:2, more preferably wherein the pathogen binding peptide has a sequence comprising one motif selected from SEQ ID Nos: 5-8 or 27-47 and wherein the pathogen binding peptide comprises a sequence having at least 90% identity to the sequence of SEQ ID NO:2, and even more preferably wherein the pathogen binding peptide has a sequence comprising one motif selected from SEQ ID Nos: 5-8 or 27-47 and wherein the pathogen binding peptide comprises a sequence having at least 95% identity to the sequence of SEQ ID NO:2.
65. The method of any of embodiments 53-64, wherein the method is carried out in a flow through set-up.
66. The method of embodiment 65, wherein the flow speed is at most 3 m/s, preferably at most 2.5 m/s, more preferably at most 2 m/s, even more preferably at most 1.5 m/s, even more preferably at most 1 m/s, even more preferably at most 0.9 m/s, even more preferably at most 0.8 m/s, even more preferably at most 0.7 m/s, even more preferably at most 0.6 m/s, even more preferably at most 0.5 m/s, even more preferably at most 0.4 m/s, even more preferably at most 0.35 m/s, even more preferably at most 0.3 m/s, even more preferably at most 0.25 m/s, even more preferably at most 0.2 m/s, even more preferably at most 0.15 m/s, even more preferably at most 0.1 m/s, even more preferably at most 0.09 m/s, even more preferably at most 0.08 m/s, even more preferably at most 0.07 m/s, and most preferably at most 0.06 m/s.
67. The method of embodiment 65 or 66, wherein the flow speed is at least 0.008 m/s, preferably at least 0.01 m/s, more preferably at least 0.02 m/s, even more preferably at least 0.025 m/s, even more preferably at least 0.03 m/s, even more preferably at least 0.035 m/s, even more preferably at least 0.04 m/s, even more preferably at least 0.045 m/s, even more preferably at least 0.05 m/s, and most preferably at least 0.055 m/s.
68. Use of superparamagnetic iron-based particles for reducing the concentration of pathogens and/or pathogen components in an aqueous or body fluid sample, wherein the superparamagnetic iron-based particles are linked to a target binding peptide.
69. The use of embodiment 68, wherein the superparamagnetic iron-based particles for are further defined in any of embodiments 2-9, 31-37 and 46-52, the sample is further defined in any of embodiments 11 or 12, the pathogens are further defined in any of embodiments 18-22, the pathogen components are further defined in any of embodiments 23-25, the target binding peptide is further defined in any of embodiments 26-30, and/or the magnetic field is further defined in embodiment 38.
70. The use of embodiment 68 or 69, wherein the concentration of pathogens and/or pathogen components in the sample is reduced by at least 20%, preferably at least 25%, preferably at least 30%, preferably at least 35%, preferably at least 40%, preferably at least 45%, more preferably at least 50%, when comparing said concentration at the start and at the end of the use.
71. Use of superparamagnetic iron-based particles for identifying pathogens in an aqueous or body fluid sample, wherein the superparamagnetic iron-based particles are linked to a target binding peptide, wherein the target is a pathogen.
72. The use of embodiment 70, wherein the superparamagnetic iron-based particles are further defined by any one of embodiments 2-9, 31-37 and 46-52, the sample is further defined in any of embodiments 11 or 12, the pathogens are further defined in any of embodiments 18-22, and/or the magnetic field is further defined in embodiment 38.
73. Superparamagnetic ironoxide nanoparticles (SPIONs), wherein the SPIONs are linked to a target binding peptide, wherein the target is a pathogen and/or a pathogen component.
74. The SPIONs of embodiment 72, wherein the SPIONs are magnetically attractable and wherein the magnetic attractability is defined as a reduction of the SPION concentration in water by 65% to 99.95% when applying a magnetic field of 0.31 Tesla for three minutes in static condition.
75. The SPIONs of embodiment 72 or 73, wherein the SPIONs are further defined in any of embodiments 6-9, 31-37 and 46-52.

### Description of Figures

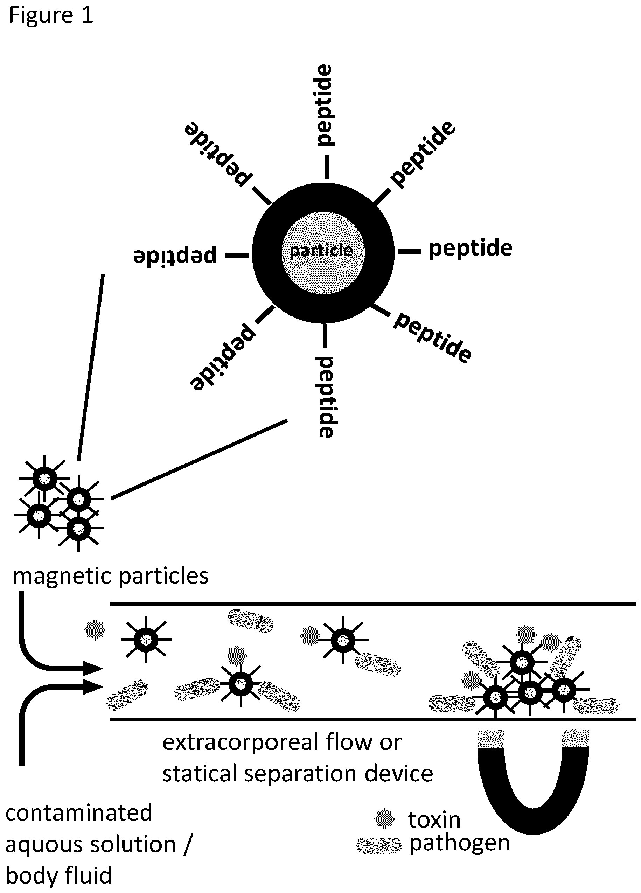

**Fig. 1****:** Schematic overview of the magnetic particles as disclosed herein and the methods for reducing the concentration of pathogens and/or toxins in an aqueous solution or body fluid sample. The magnetic particles are linked to a target binding peptide, wherein the target is a pathogen or pathogen component.
**Fig. 2**: Overview of scanning electron microscopy images for different particles. a) and b) SPION^{APTES} (top panel); c) SPION^{Cit} (bottom left); d) SPION^{HAp} (bottom right). Scale bars in each image are 200nm in length, e) TEM-Image from Particles binding to E.coli; Transmission electron microscopy pictures were taken from samples of separated E.coli using SPION^{APTES-SBA-SPP04}
**Fig. 3**: Energy-dispersive X-ray spectroscopy (EDX) analysis for a) SPION^{APTES} b) SPION^{HAp} and c) SPION^{Cit} . Silicon found in b) and c) is related to the preparation on a Si-wafer.
**Fig. 4**: X-ray diffraction patterns for SPION^{APTES} , SPION^{Cit} and SPION^{Hap}. Typical peaks for the structure of iron oxide are present in all particle types and hydroxyapatite peak is observed in SPION^{HAP}. Indexation of the peaks was performed according to Schwertmann et al. (2003) and Sneha et al. (2015)
**Fig. 5**: Fourier transform infrared (FTIR) signal of SPION^{APTES} , SPION^{Cit} and SPION^{HAP}. Arrows highlight the peaks that were used for identification according to Pretsch et al.
**Fig. 6**: Pathogen-binding peptide found on different particles (SPION^{APTES}, SPION^{Hap}, SPION^{APTES-SBA}). Data are expressed as means ± SD experiment was performed in triplicates.
**Fig. 7**: Magnetic attractability of the particles according to the present disclosure and Karawacka et al. (2018). (left) Magnetic attractability is assessed by determining the remaining Fe concentration in solutions exposed to a magnet with 310 mT for 3 minutes. Concentration of Fe in the supernatant of different particle types (SPION^{APTES}, SPION^{APTES-SBA-SPP04} and SPION^{APTES-SBA-SPP19}) compared to particles as obtained by following Karawacka et al. (2018). The controls were not exposed to the magnet at represent the input. (right) Magnetic attractability is assessed by determining the remaining Fe concentration in solutions exposed to a magnet with 0.8T at a flow rate of 6 ml/min (0.057 m/s). Concentration of Fe before and after separation for SPION^{APTES-SBA-SPP04}. As negative control water was used.
**Fig. 8**: Endotoxin binding to particles for different LPS types (concentration 10 EU/ml), exposure times and iron concentrations. a) 30 s shaking, 1 mg Fe/ml; b) 3 s shaking, 1 mg Fe/ml; c) 30 s, 0.01 mg Fe/ml; d) 3 s shaking, 0.01mg Fe/ml.
**Fig. 9**: Binding of LTA in aqueous solution. (left) LTA-depletion for SPION^{APTES} und SPION^{APTES-SBA-SPP04}/SPION^{APTES-SBA-SPP19}.
**Fig. 10****:** Separation efficiency of micromer^{LPS} for SPION^{APTES- SBA-SPP04} and SPION^{HAp} compared to SPION^{APTES}. Experiment was performed in triplicates of three individual experiments. Results were averaged.
**Fig. 11****:** Depletion of *E.coli* for SPION^{APTES-SBA-SPP04} and SPION^{HAp} in aqueous medium. The amount of bacteria in the supernatant was determined.
**Fig. 12****:** Depletion of *Staphylococcus aureus* for SPION^{APTES}, SPION^{APTES-SBA-SPP04} and SPION^{APTES-SBA-SPP19} from aqueous medium. (top) The CFU values of the controls and the particle supernatants are shown as well as the CFU of the re-plated particles loaded with bacteria; (bottom) Influence of SPION^{APTES-SBA-SPP04} and SPION^{APTES-SBA-SPP19} on the cultivation of *Staphylococcus aureus* after plating the separated bacteria.
**Fig. 13****:** Depletion of *Pseudomonas aeruginosa* by SPION^{APTES}, SPION^{APTES-SBA-SPP04} and SPION^{APTES-SBA-SPP19} from aqueous medium. (top) The CFU values of the controls and the particle supernatants are shown as well as the CFU of the re-plated particles loaded with bacteria; (bottom) Influence of SPION^{APTES-SBA-SPP04} and SPION^{APTES-SBA-SPP19} on the cultivation of *Pseudomonas aeruginosa* after plating the separated bacteria.
**Fig. 14****:** Depletion of *Serratia marcescens* by SPION^{APTES-SBA-SPP04} from aqueous medium. The CFU values of the controls and the particle supernatants are shown as well as the CFU of the re-plated particles loaded with bacteria.
**Fig. 15****:** Separation efficiency of bacteria from blood (stabilization EDTA or citrate) using SPION^{APTES-SBA-Pep} compared to an untreated sample. The experiment was performed in triplicates of three individual experiments results were averaged. In citrate-stabilized blood, the number of CFUs per ml decreased from 1913 CFUs/ml to 927 CGU/ml when treated with SPION^{APTES-SBA-SPP04}, Thus, a reduction in bacterial load of around 52% was achieved. Furthermore, a replating of the extracted bacteria by SPION^{APTES-SBA-SPP04} resulted in 913 CFUs/ml. In comparison, the number of CFUs per ml decreased from 2867 CFUs/ml to 1733 CFU/ml when treated with SPION^{APTES-SBA-SPP04} in EDTA-containing blood. Thus, only a reduction of around 40% was achieved. Furthermore, a replating of the extracted bacteria by SPION^{APTES-SBA-SPP04} in EDTA-containing blood resulted in 1066 CFUs/ml.
**Fig. 16****:** Comparison of the bacterial extraction efficiency. Left: Plating of the untreated sample resulting in dispersed single colonies, Right: Plating of the concentrated sample after enrichment of the bacteria with SPIONs (concentrated via magnetism) resulting a dense bacterial lawn.

### Examples

Pathogenic microorganisms, such as bacteria, can contaminate drinking water and cause sepsis by their presence, and also through the presence of toxins, e.g. lipopolysaccharides (LPS) derived from the bacteria's outer cell membrane. Consequently, huge amount of cytokines are released, often resulting in multiple organ dysfunctions and death despite treatment. A novel experimental approach is to remove pathogens and/or pathogen components by using magnetic particles. For this purpose, the inventors synthesized iron-based particles, especially superparamagnetic ironoxide particles (SPION). In one example, the SPIONs were coated with an (3-Aminopropyl)-triethoxysilan (APTES) layer using a co-precipitation method. As a linker for the APTES-coated superparamagnetic iron oxide (SPION^{APTES}), N-succinimidyl bromoacetate (SBA) was used to bind short peptide sequences to amino groups on these particles. The results showed a quantitative reduction of pathogens, such as Gram-positive and Gram-negative bacteria as well as a reduction of pathogen components, such as LPS and LTA by using a peptide-functionalized SPION^{APTES} (SPION^{APTES-SBA-SPP04} and SPION^{APTES-SBA-SPP19}). In the light of these results, the inventors aim to use these particles for example in extracorporeal clearance, as well as the removing of the pathogens from other liquid sables such as drinking water.

### Abbreviations:

SPION: superparamangentic ironoxide particles
SPION*^{cit}*: SPIONs coated with citrate
SPION^{*Ha*p}: SPIONs coated with calcium phosphate
SPION^{APTES}: SPIONs coated with 3-Aminopropyltriethoxysilan
SPION^{APTES-SBA-SPP}: SPIONs coated with 3-Aminopropyltriethoxysilan with linked Peptide (SPP followed by the number of the peptide)
PDI: polydispersity index
ζ: zetapotential
Xv: susceptinitity (volume specific)
Micromer^{LPS}: micromer^{R} particles decorated with Lipopolysaccharides
SPION^{HAp} and SPION^{APTES} served as control particles.

### Example 1 - Generation of superparamagnetic iron oxide (SPION) particles with linked peptide (SPION^{APTES-SBA-SPP04}) - a physiochemical characterization

As a fast and reliable method of synthesizing particles, the commonly used co-precipitation method was carried out. With this technique it was possible to reproduce the synthesis of different particles with minimal changes in the overall parameters. An *in-situ* coating process was used for SPION^{APTES} as described before. For SPION^{HAp} a two-step procedure, with intermediate citrate coating of iron oxide particles (SPION^{Cit}) was used. SPION^{Cit} have a stable coating with citrate showing no visible agglomeration and good storage capabilities. This can be an advantage to prevent agglomeration in the second step, especially if they are compared to bare iron oxide particles.

The hydrodynamic diameter (HD), polydispersity index (PDI), Z potential as well as the susceptibility are listed in **Table 1.** The mean hydrodynamic diameter of SPION^{APTES} was found to be around 153 nm and around 175 nm for SPION^{HAp}. The PDI of the used batches was below 0.2 which indicates a narrow size distribution. In biomedical applications the size of the particles has to be considered very carefully before releasing particles into e.g. human blood. In this approach, the separation of pathogens and pathogen components is supposed to be taking place in e.g. an extracorporeal (*in vitro*) blood stream comparable to dialysis setups. Therefore, particles shall not to be too small in order to remove them very efficiently by magnetic means.

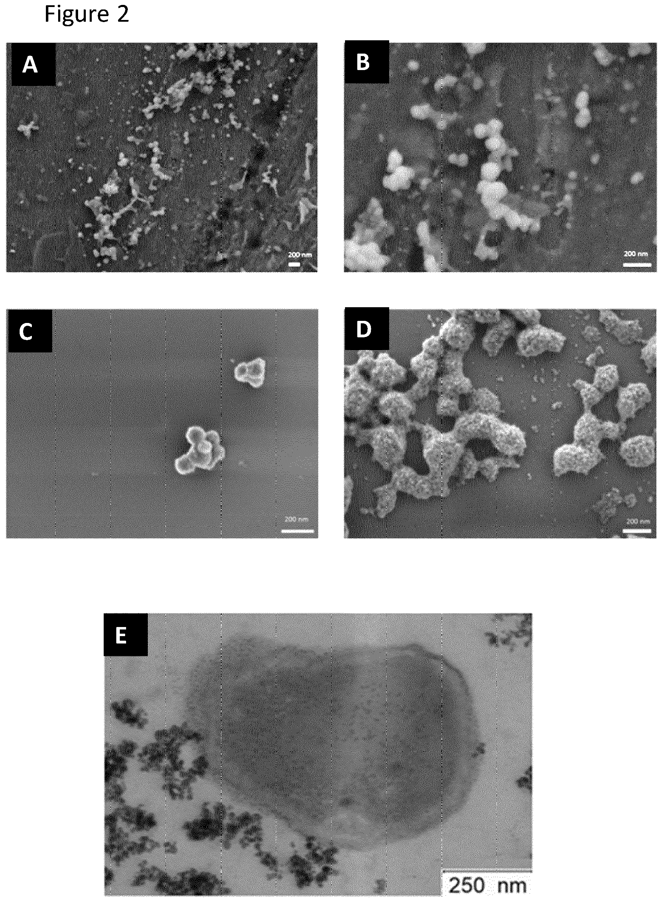

Scanning electron microscopy (SEM) was applied to examine the morphology of the particles. Because of aggregation during the process of drying, the HD differs from the one seen in the SEM-images. It is also clear that the HD is not the physical diameter of the particles, but includes also the surrounding hydration layer. In case of SPION^{Cit} and SPION^{APTES}, the SEM-diameter is smaller compared to their HD (see **Fig. 2**); SPION^{HAp} increased in size because of aggregation. Some smaller particles in the images of SPION^{APTES} and SPION^{HAp} are debris, which occurs during the drying process. Dispersions are considered aggregation stable when the Z-potential exceeds a value of +/- 30 mV, which is fulfilled for SPION^{Cit} and SPION^{APTES} (see **Tab. 1**). SPION^{HAp} were close to this value, but showed precipitation. The aggregation stability is influenced by pH, salt concentration, peptides and other molecules bound to the particles. SPION^{APTES} and SPION^{Cit} particles can be stored at a concentration from 1 to 9 mg/ml and a pH of 4 to 7.5 without any precipitation for more than three months. SPION^{HAp} has a tendency to show precipitation independent of concentration at this pH range after four days. No changes in size were observed for the particles after a period of three months. All particles had a high volume magnetic susceptibility of more than 4.5*10⁻³ in SI units as seen in **Tab.1.** Differences are related to the individual synthesis, affecting the core size, crystallinity or the size of the coating layer.

**Tab. 1: Size, PDI, zeta potential and susceptibility**

| | **SPION^{Cit}** | **SPION^{APTES}** | **SPION^{HAp}** |
|---|---|---|---|
| Hydrodynamic diameter [nm] | 109 ± 1 | 153.3 ± 1 | 174.5 ± 1 |
| PDI | 0.189 | 0.147 | 0.117 |
| ζ[mV] | -46.9 ± 0.9 | + 51.7 ± 1.15 | -29.2 ± 0.37 |
| Xv ·10⁻³ [SI units] | 6.7 | 5.6 | 4.9 |

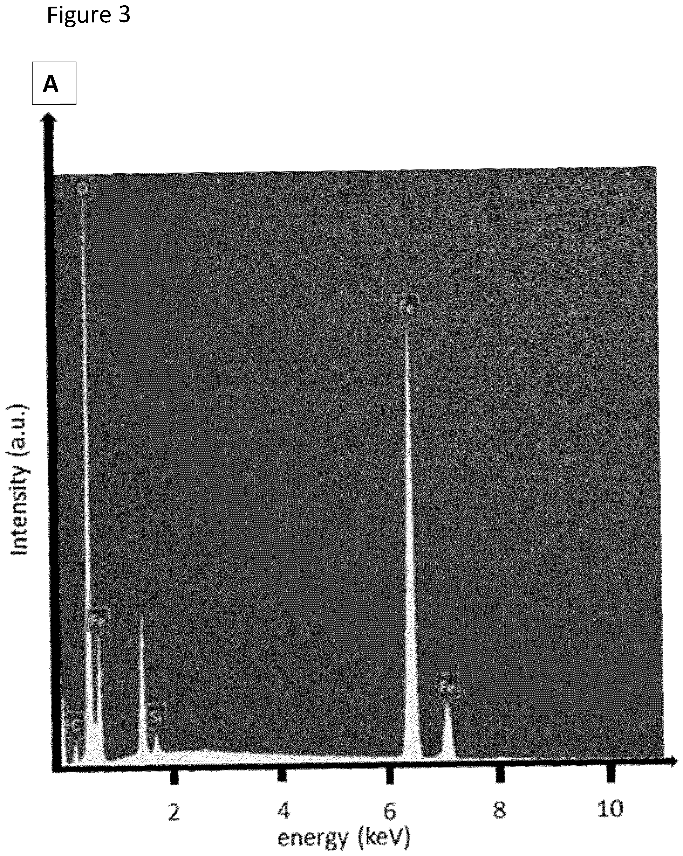

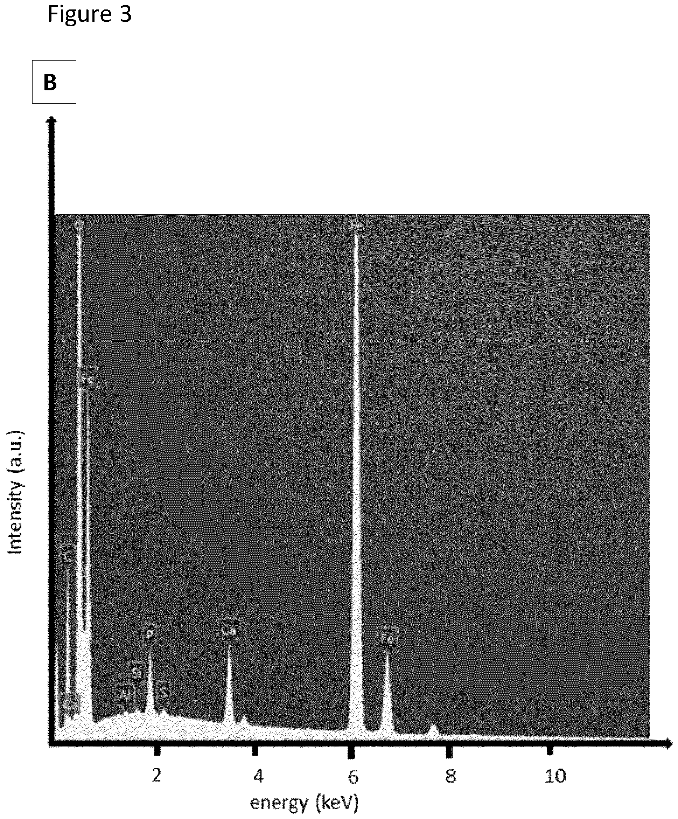

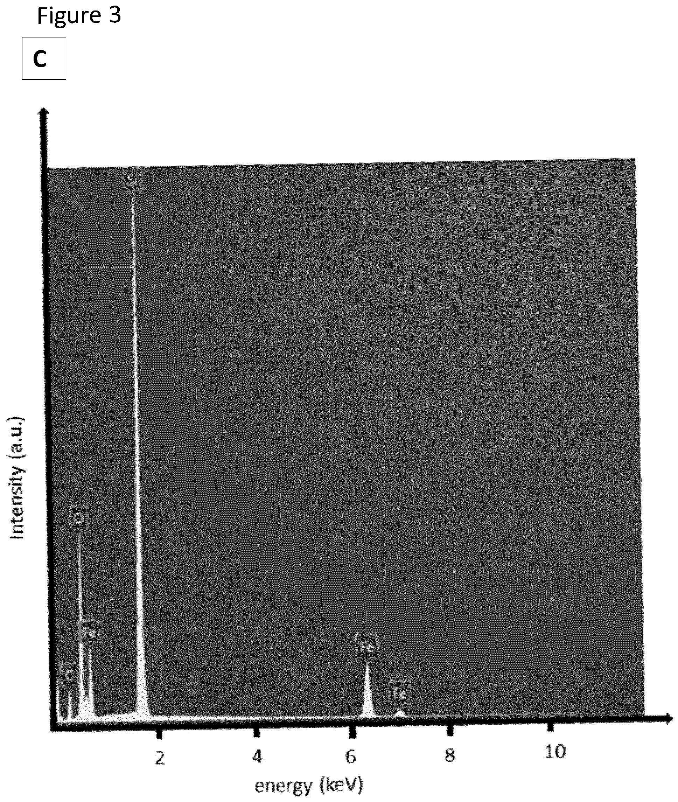

Furthermore, Energy-dispersive X-ray spectroscopy (EDX) analysis was used for the elemental analysis and chemical characterization of a) SPION^{APTES} b) SPION^{HAp} and c) SPION^{Cit} (**Fig. 3**). Silicon found in b) c) is related to the preparation on a Si-wafer. EDX analysis detected the Fe and O in all particles as shown. Silicon was detected in SPION^{APTES} and calcium and phosphorus in SPION^{HAp}. The EDX analysis confirms the results measured by inductively coupled plasma atomic emission spectroscopy (AES/ICP)-Analysis (data not shown).

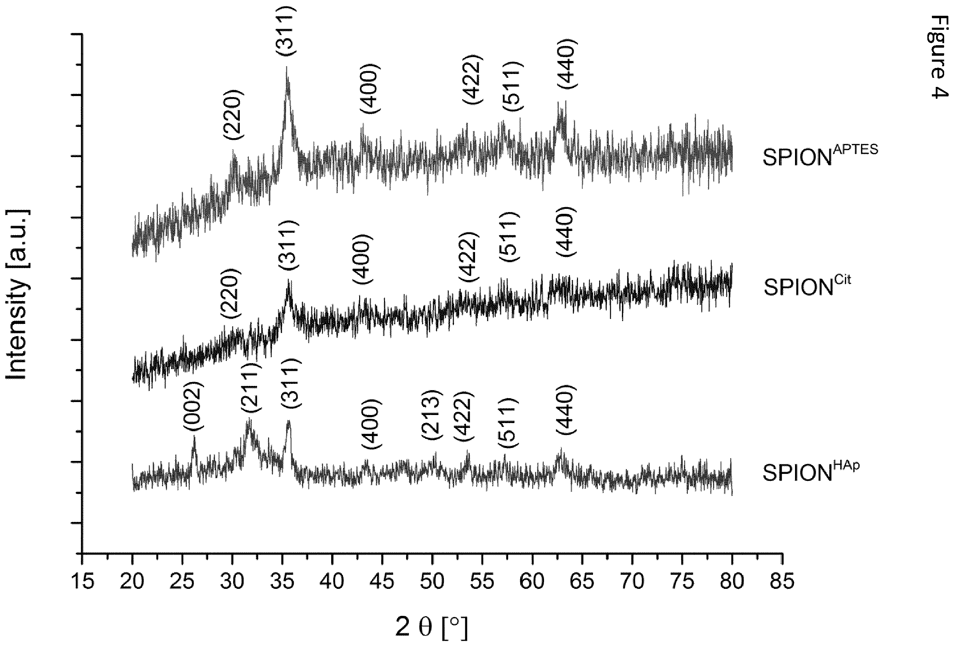

X-ray diffraction (XRD) patterns (see **Fig. 4**) confirm the presence of magnetite/maghemite related characteristic peaks at (311) and (400), (422), (511) as well as (440) in all particle sample [32]. The presence of peaks that are characteristic for hydroxyapatite can be found for SPION^{HAp} at approximately 26°, 32°, 49° and 53° and are indicative for the successful HAp coating formation. The most dominant peak for hydroxyapatite coated particles was obtained at 20 = 32.4° corresponding to (211). It should be taken into account that in case of SPION^{Hap} the peaks for maghemit are close to the one of hydroxyapatite. The relatively low peaks are indicating a lower crystallinity of the hydroxyapatite on the particles. Hence, XRD patterns (**Fig. 4**) confirmed the presence of magnetite/maghemite-related characteristic peaks at (311) and (400), (422), (511), as well as (440), in particle samples SPION^{APTES}, SPION^{Cit} and SPION^{HAP}.

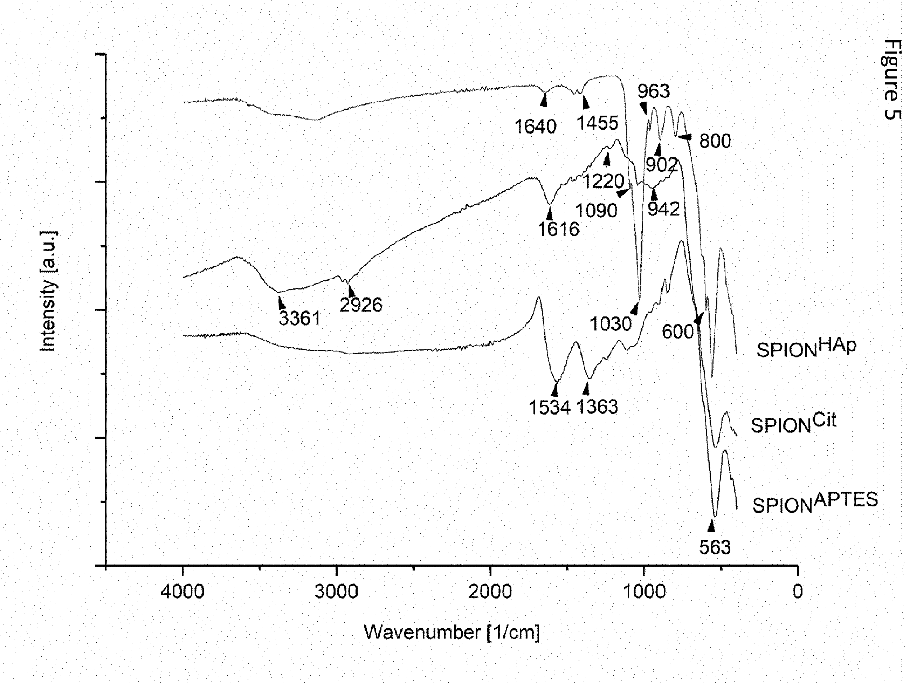

The Fourier transform infrared (FTIR) spectra of SPION^{APTES}, SPION^{Cit} and SPION^{HAp} can be seen in **Fig. 5**. The most present band for all particles is the Fe-O stretching band at 536 cm⁻¹. The C=O vibration from the COOH is shifted to an intense band at about 1534 cm⁻¹ for SPION^{Cit} [38]. The peak of SPION^{Cit} at 1363 cm⁻¹ is associated with the symmetric stretching of CO from the COOH group. The spectrum of SPION^{HAp} shows bands at 600, 942 and 1090 cm⁻¹, corresponding to the bands for PO4⁻³.

In a next step, the inventors assessed the efficiency of covalently linking a peptide to the particles. The efficiency to bind peptide to the particles can vary greatly based on the chemoselective linker used. One possible candidate is SBA, which has been used in a previous study (Karawacka et al (2018)).

As shown in **Fig. 6**, the results showed a binding of 0.08 µmol peptide/mg Fe for SPION^{APTES-SBA}, while SPION^{APTES} and SPION^{HAp} showed a binding of 0.046 µmol peptide/mg Fe and 0.055 µmol peptide/mg Fe, respectively. Thus, SPION^{APTES-SBA} showed significantly more binding of peptide per mg Fe. This amount of found peptide on SPION^{APTES-SBA} is near the theoretical amount of amino groups and higher than the measured amount of amino groups. All measurements can be disturbed by the background caused by remaining particles of the indirect method which was used. As mentioned above, different batches can have fluctuations in the amount of bound peptide because of losses during the process. If no release of the peptide can be detected in the second washing steps, which was the case in every tested batch, the amount of peptide was considered to be bound stably. Binding of more than 0.08 µmol peptide/mg Fe for SPION^{APTES-SBA} can also be achieved.

Considering that the amount of bound peptide was significantly higher on the SPION^{APTES-SBA} than on SPION^{APTES} or SPION^{HAp}, it can be assumed that the linker increased the amount of peptide bound to the particles. Consequently, the chemoselective linker SBA improves the covalent linkage between the SPION and the peptide, however, the chemoselective linker is not necessary.

In summary, all particles as disclosed herein had a high magnetic susceptibility. Differences were related to the individual synthesis, affecting the core size, crystallinity, or the size of the coating layer. SPION^{APTES} (and the functionalized SPION^{APTES-SBA-SPP}) and SPION^{HAp} could be easily magnetically separated from water at a pH of 7.4, which was key for the subsequent experiments. The results show a good size distribution and stability for the different particle systems. The susceptibility Xv for the different particles is comparable for SPION^{HAp} and SPION^{APTES} (see **Table 1**) and both can be magnetically separated.

### Example 2 - Improvement of SPION particles compared to Karawacka et al. (2018)

Base on the SPION particles described in Karawacka et al. (2018), the SPION preparation has been further improved so that the SPIONs show a better magnetic attractability/separability and are more metastable in solution. The synthesis described by Karawacka et al. was based on a synthesis of Zaloga et al. 2014. The improved synthesis as disclosed herein uses the same stoichiometric ratios. However, the preparation volume of the synthesis was changed from 10 ml to 50 ml. In addition, the speed of mixing the components and the setup volume was changed that the stirring was constant at 450 rpm during synthesis. Furthermore, the subsequent clean-up was performed by multiple washing with DI water with direct dispersion in DI water and was not performed by dialysis or after drying.

Compared to Karawacka et al. (2018) the amount of Si on the particles could be increased by 25%. This results in an increase of the reactive, i.e. available -NH2 groups for the subsequent functionalization with succinimidyl bromine acetate (SBA). This leads to a higher degree of functionalization with the peptides.

The educt quantity of the SBA was increased by a factor of four and the time of functionalization was doubled compared to Karawacka et al. (2018). In the subsequent binding of the peptides (SPP04 and SPP19), an increase in the binding to peptide and thus an increase in the peptide content from 0.06 µmol peptides /mg Fe for Karawacka et al. (2018) to over 0.085 µmol peptides/mg Fe for SPP04 and 0.082 µmol peptides /mg Fe for SPP19 was achieved by doubling the reaction time. This improvement in the method resulted in an increase in peptide binding of 37% and 42%, respectively. Furthermore, this improvements was consistently at around 40% and thus it is expected that a functionalization with another peptide is expected to also show a similar increase in functionalization by changing the reaction conditions as described above.

Furthermore, the particle size increased in comparison to the SPIONs as disclosed by Karawacka et al. (2018). It is notes that the size of the particles before and after functionalization must be distinguished. Karawacka et. al. (2018) synthesized SPION^{APTES} with a diameter between 100 - 190 nm. SPION^{APTES} as disclosed herein have a diameter between 180 - 250 nm. Karawacka et al. (2018) is silent on the particle size (diameter) after functionalization with peptide. SPION^{APTES-SBA-SPP04}/SPION^{APTES-SBA-SPP19}as disclosed herein are between 1.0 and 2.7 µm depending on pH. In neutral pH (e.g. around 7), a diameter of 2.54 µm is achieved for SPION^{APTES-SBA-SPP04}. A magnetic separation of the particles could not be achieved with the particles produced by Karawacka et al. (2018). This is why the particles in **Figure 7** of Karawacka et al. (2018) were centrifuged in order to separate the LPS bound particles from water. The efficiency of magnetic separation was compared and contrasted in the static condition and tested in the flow condition herein. Particles according to Karawacka et. al. (2018) could not be separated magnetically due to their properties as described above. However, a magnetic and fast separation is key for depleting pathogens or pathogen components from liquids.

For testing and comparing the depletion efficiency of the SPIONs as disclosed herein and the SPIONs of Karawacka et al. (2018), the inventors performed atomic emission spectrometry (AES) measurements of the supernatants after the different separation experiments. **Fig. 7****.** shows a fundamentally better separations with the improved SPION particles as disclosed herein. The reason for the substantially improved depletion is mainly the size of the particles and their metastability in medium. In static condition, the particles of Karawacka et. al. (2018) can be reduced to only one third of the original iron quantity (reduction from 300 µg Fe/ml to 120 µg Fe/ml). In comparison, the SPP04 and SPP19 functionalized SPIONs as disclosed herein reach a significantly lower value of 2.8 and 2.3 µg Fe/ml, respectively. Consequently, around 99% of the particles as disclosed herein were separated by magnetic exposure. This is in strong contrast to the particles as obtained by following the protocol as disclosed in Karawacka et al. (2018) by which only a reduction by around 60% is achieved. Similarly, when carrying out the experiment under flow condition with a flow speed of 6 ml/min (0.057 m/s), an original Fe concentration of 17.5 µg Fe/ml could be reduced to 2.2 µg Fe/ml when using the particles as disclosed herein. Thus, a reduction by 87% could be achieved.

### Example 3 - SPION^{APTES-SBA-SPP04} specifically binds pathogen components

In order to evaluate general parameters of the concentration and incubation time with SPIONs, the inventors analysed conditions that are representative for these parameters. A high and a low particle concentration, as well as two exposure conditions were tested (agitation for 30 sec and 3 sec; particle concentration of 0.01 mg Fe/ml and 1 mg Fe/ml) were tested with the peptide exposing SPION^{APTES-SBA-SPP04} and non-peptide exposing SPION^{HAp}. The concentration of peptide for the functionalized particles was assumed to be 0.08 µmol peptide/mg Fe, as assessed above. As other particles have been reported to bind certain amounts of LPS unspecifically, SPION^{HAp} are interesting for our experiments concerning this concern as a control.

An amount of 10 EU/ml for the different types of LPS was used, which is equal to an endotoxin amount of 1 ng/ml. Interestingly, studies have shown that the amount of endotoxin found in plasma is higher in patients that show a sepsis than in healthy patients. Said amount is ranging from 2 pg/ml to about 5 ng/ml for healthy individuals and patients with sepsis, respectively. In case of a severe septic shock it can be 1000 to 10000 times higher than in healthy patients (Jeong et al (2019); Reich et al. (2016); Rhodes et al. (2017)). The median level of endotoxin for non-survivors was found to be about 0.5 ng/ml and 0.2 ng/ml for survivors. Therefore, the reduction of endotoxin should be as efficient as possible.

The efficiency of the separation is shown in **Fig. 8**. As other studies reported that ions and peptides as well as other substances could mask the true concentration of LPS in regular used tests, we used spiking controls (Herrmann et al. (2013) and Lee et al (2014)). Spiking controls (8 and 5 EU/ml) were carried out to see if any interference occurred for all tested particles. No interference between LPS free water and sample supernatant (sample not treated with LPS before) were detected during this study (data not shown). At 1 mg Fe/ml, SPION^{APTES-SBA-SPP04} completely removed the LPS below the detection limit of the used assay after 30 s of agitation (**Fig. 8A**) Thus, all LPS was bound by SPION^{APTES-SBA-SPP04}. In case of SPION^{HAp} a reduction to 8% remaining LPS was achieved for high concentrations in the best case (**Fig. 8A**). For the same amount of particles, shaking for 3 s was not as effective as 30 sec. However, only up to 6% LPS was remaining in the samples, which were incubated with SPION^{APTES-SBA-SPP04} at 3 sec incubation (**Fig. 8B**). Thus, 94% of the LPS was bound by SPION^{APTES-SBA-SPP04}(**Fig. 8B**).

By reducing the amount of particles to 0.01 mg Fe/ml and therefore the amount of peptide in the same extend to a 100-fold lower concentration the binding of LPS was reduced to about 50% for SPION^{APTES-SBA-SPP04} (**Fig. 8C****, D**). Thus, even at a very low concentration of 0.01 mg Fe/ml, the SPION^{APTES-SBA-SPP04} particles reduced the amount of all three LPS types in the solution by at least 48% when incubated for 30 seconds and by at least 40% when incubated for 3 seconds. At the same time, not a greater extent of binding could achieved, leading to the assumption that at this concentration SPION^{APTES-SBA-SPP04} cannot bind more LPS. This value varied in a range of up to 20% between the different LPS types used in this study. At low particle concentrations the separation efficiency dropped for both particles. As seen in **Fig. 8D** low particle concentration in combination short exposure time lead to only weak reduction of the endotoxins for SPION^{Hap} (at best 10% reduction). Thus, particularly at a low concentration of SPION^{APTES-SBA-SPP04} particles (0.01 mg Fe/ml) and at a short incubation time of 3 seconds there is a very significant difference between SPION^{APTES-SBA-SPP04} and SPION^{Hap} particles in the ability to bind LPS. While SPION^{APTES-SBA-SPP04} particles were still able to bind to at least 40% of LPS (all LPS types tested) SPION^{Hap} particles were only able to bind to up to 10% of LPS. In summary, the study showed that SPION^{APTES-SBA-SPP04} were able to remove LPS significantly better than SPION^{HAp}.

In summary, the separation efficiency of LPS is illustrated **Fig. 8**. At 1 mg Fe/ml, SPION^{APTES-SBA-SPP04} completely removed the endotoxin below the detection limit of the used assay after 30 s of agitation. Even at a low concentration of 0.01 mg Fe/ml and 3 seconds incubation SPION^{APTES-SBA-SPP04} particles still removed at least 40% of LPS from the sample and SPION^{APTES-SBA-SPP04} particles performed significantly better than the control SPION^{Hap} particles.

The binding of a further relevant toxin (from gram-positive bacteria) was also investigated with the improved particles as disclosed herein, which was not described in Karawacka et al. (2018). These experiments were performed in aqueous Ringer' solution. **Fig. 9** shows the depletion of lipoteichonic acids (LTA) by SPION^{APTES}, SPION^{APTES-SBA-SPP04}/SPION^{APTES-SBA-SPP19}, using 0.3 mg Fe/ml per particle. For this experiment, 2.5 ng/ml LTA were used and incubated for 5 minutes at 37°C on a shaker at 700 rpm. Subsequently, the particles were separated magnetically for 3 minutes. For SPION^{APTES} no significant LTA depletion could be detected. SPION^{APTES-SBA-SPP04}/SPION^{APTES-SBA-SPP19}could bind 0.75 ng/ml LTA and 0.97 ng/ml, respectively. Thus, a reduction of LTA by 30% and 29%, respectively, could be detected by using the improved particles SPION^{APTES-SBA-SPP04}/SPION^{APTES-SBA-SPP19} as disclosed herein.

### Example 4 - SPION^{APTES-SBA-SPP04} specifically separates artificial gram-negative bacteria (Micromer^{LPS})

For a better understanding of the separation process, the inventors tried to remove artificial micromers to simulate bacteria. For this, LPS coated polystyrene/polymethacrylate Micromer^{®} particles (micromer^{LPS}) were used and removed from a water suspension instead of living bacteria. Micromer^{LPS} had a size of about 1 µm corresponding to the size of some bacteria e.g. *S. aureus.* This experiment was performed by optical density measurements at 620 nm, as it is done for actual bacteria.

As shown in **Fig. 10**, using magnetic separation, the inventors reduced the amount of micromer^{LPS} using SPION^{APTES-SBA-SPP04} from an initial amount of micromer^{LPS} 0.1 mg/ml to 0.023 mg/ml. In contrast, only a minor reduction to about 0.059 mg/ml remaining micromer^{LPS} was achieved using SPION^{APTES}. This minor effect might originate from physical effects that occur during the magnetic separation. For SPION^{HAp}, a reduction to about 0.073 mg/ml was detected. Based on these results, it has been shown that during the incubation time, SPION^{APTES-SBA-SPP04} effectively bound to the micromer^{LPS}, resulting in a significantly higher rate of separation when compared to SPION^{APTES} and SPION^{HAp} particles, which do not harbor the pathogen binding peptide. Thus, SPION^{APTES-SBA-SPP04} can be used for separating bacteria specifically. Therefore, it is plausible based on Example 4 that SPION^{APTES-SBA-SPP04} is able to specifically bind to gram-negative bacteria and immobilise gram-negative bacteria in a liquid solutions including blood. Furthermore, successful separation of several gram-positive and gram-negative bacteria is demonstrated below.

### Example 5 - SPIONAffES-SBA-SPP04 separates E.coli, S.aureus and P.aeruginosa from an aqueous solution

In addition to the removal of LPS and artificial bacteria, the inventors also set out to remove actual bacteria from aqueous solutions. Karawacka et. al. (2018) did not perform separations of whole bacteria. The step of plating out the particles after the separation is of high diagnostic importance, because it allows extract a high concentration of bacteria and thus an improvement of the pathogen diagnosis. The identification of the bacterial is of great therapeutic value as an anti-bacterial therapy can be tailored after the assessment of the (mixture of) bacteria, which infected the blood.

**Fig. 11** shows a magnetic separation experiment of *E.coli* from aqueous medium (Ringer solution). *E.coli* were incubated with particles for 5 minutes and then separated for 3 minutes. Supernatants were plated out. A concentration-related dependence of the separation efficiency is provided in **Figure 11****.** At 1.0 mg Fe/ml, a reduction to 2 CFU/ml was achieved with SPION^{APTES-SBA-SPP14}, respectively. A reduction of the particle amount to 0.1 mg Fe/ml led to a reduced bacterial separation 170 CFUs). SPION^{HAP} showed a much lower separation efficiency in both concentrations (552 CFUs at 0.1 mg Fe/ml, and 372 CFUs at 1 mg Fe/ml).

Based on the values from the experiment shown in **Fig. 11****,** a reference amount of 0.3 mg Fe/ml was chosen as the test concentration for the further experiments. This was retained for all further bacterial depletion experiments.

For testing the separation of *S.aureus* from a sample, particles were functionalized with the peptides SPP04 and SPP19 for this experiment. The bacterial suspension was adjusted to an OD (measured at 600nm) of 0.13 and then diluted 1:800 with Ringer's solution. Both functionalized particles showed a high depletion of the bacteria and thus a subsequent plating was possible. It could also be shown that the particles have no toxic effect on the bacteria. For *Staphylococcus aureus* (gram+) a reduction from 1393 CFU/ml to 27 CFU/ml for SPION^{APTES-SBA-SPP04} and 73 CFU/ml for SPION^{APTES-SBA-SPP19} could be achieved by separation as shown in **Fig. 12****.**

When plating the bacteria loaded particles (also called re-plating), CFU values in the range of the positive control were found for SPIO^{NAPTES-SBA-SPP04} and SPION^{APTES-SBA-SPP19}. In the case of SPION^{APTES-SBA-SPP04}, the number of CFU is even increased. Analogous observations are shown in **Fig. 13** for the gram negative bacterium *Pseudomonas aeruginosa* reduction from 5320 CFU/ml to 2 CFU/ml for SPION^{APTES-SBA-SPP04} and 7 CFU/ml for SPION^{APTES-SBA-SPP19} could be achieved. For *Serratia marcescens* (gram-) a reduction from 1300 CFU/ml to 26 CFU/ml for SPION^{APTES-SBA-SPP04} could be achieved as shown in **Fig. 14****.** Redispersed particles that were plated out yielding a recovery of 1227 CFU/ml for SPION^{APTES-SBA-SPP04} which is similar to the positive control.

### Example 6 - SPION^{APTES-SBA-SPP04} separates E.coli from blood

In further experiments, the inventors demonstrate herein that it is possible to separate and extract *E coli* from blood and additionally to plate out the bacteria-bound particles again. For this experiment, *E coli* was added to blood and then 0.3 mg Fe/ml particles were used to extract/separate the bacteria. Thus, the remaining bacterial load in the blood was tested as a first aspect of this experiment. Furthermore, citrate and EDTA-stabilized blood was used and compared, as EDTA is expected to hinder the binding of the peptide to the bacteria. Therefore, the depletion of bacteria is expected to be lower in EDTA-stabilized blood compared to citrate-stabilized blood. The overall bacterial load in citrate stabilized blood amounted to 1913 colony forming units per milliliter (CFU/ml) **(****Fig. 15****).** SPION^{APTES-SBA-SPP04} particles reduced the concentration to 927 CFU/ml in citrate-stabilized blood. 927 CFU/ml was the lowest concentration of bacterial load in blood measured in this experiment and shows a reduction of *E.coli* concentration of 51.5% when using SPIO^{NAPTES-SBA-SPP04} particles in a sample. This is surprising and shows the great efficiency of SPIO^{NAPTES-SBA-SPP04} particles, even when used in blood, and not only in water or Ringer solution. As the concentration of colony forming units is one way of measuring the bacterial load in the blood, SPIO^{NAPTES-SBA-SPP04} particles in citrate stabilized blood showed the most efficient reduction of bacterial load. For completeness, in EDTA-stabilized blood, the amount of CFUs was reduced from 2867 CFU/ml to 1733 CFU/ml by SPION^{APTES-SBA-SPP04}. Thus, a reduction of around 40% was achieved.

In a second aspect of the experiment, bacteria bound to the particles were recovered from the blood and (re-)plated in order to assess the plating efficiency. The plating efficiency also demonstrates that the separated bacteria from blood are still viable after separation. This is particularly valuable as this experiment can be used to assess the bacterial identity in blood. After having identified the bacterial identity of a patient's blood sample, the treatment can be tailored towards said specific bacteria. In EDTA- and citrate-stabilized blood, the bacteria were still viable and could be plated. In citrate stabilized blood, 913 CFU/ml for SPIO^{NAPTES-SBA-SPP04} resulted from the plating experiment.

Furthermore, bacteria could also be recovered by re-plating in EDTA-stabilized blood (1066 CFU/ml for SPION^{APTES-SBA-SPP04}).

In summary, the separation efficiency is highest in citrate-stabilized blood when using SPION^{APTES-SBA-SPP04}. Consequently, the SPIONs as disclosed herein are capable of separating pathogens such as gram-negative bacteria from blood, when functionalized with a pathogen binding peptide.

### Example 7 - SPION^{APTES-SBA-SPP04} are capable of concentrating viable bacteria.

In this experiment, an approach as used with common blood cultures in order to identify pathogens was chosen. The total volume was set to 10 ml. The bacterial suspension was adjusted to an OD (measured at 600 nm) of 0.13 with Ringer's solution analogous to the previous experiments. A sample, which was not treated with SPIONs, served as a control. A concentration of 0.3 mg Fe/ml SPIO^{NAPTES-SBA-SPP04} was used. Incubation was performed for 30 minutes at room temperature on a rolling incubator. The particles were then magnetically separated for 3 minutes at 310 mT. 0.5 ml Ringer's solution was added to the particle pellets and vortexed. **Figure 16** shows the plating of both samples. A total of 50 µl was plated out for each sample. It is clearly visible that individual dispersed CFUs are still visible (control, left side, no SPIONs were used) while on the right side a dense bacterial lawn is readily visible (sample concentrated with SPIONs), which indicates a strong concentration of viable bacteria. Compared to common blood cultures, bacterial pathogens can be detected faster by using this method, because the amplification step of the bacteria using blood culture bottles could be omitted.

### Example 8 - Alternative linkage of the peptide to the SPION (SPION^{Hap-pS-peptide})

In previous experiments, the peptide was bound to the C-terminal cysteine of the peptide via the SBA linker. In this experiment, the peptide was bound to the SPION via an alternative binding process. The peptide had a C-terminal phorphoserine group (pS). With the alternative procedure, the reaction step via the SBA linker can be omitted, which makes the entire synthesis much quicker and more efficient. It was possible to bind 0.03 µmol/ mg Fe of the peptide SPPpS (SEQ ID NO: 3 SSP04-pS, in comparison to SPP04, wherein the C-terminal cysteine replaced with phosphoserine) to SPION^{HAP} , as also disclosed herein. The reaction was performed in borate buffer pH 8.5 for 24h at 700 rpm and 25°C. Supernatants and wash steps were quantified in the UV spectrometer to calculate the amount of peptide on the particles. In conclusion an alternative method to covalently link the peptide to the SPION has been tested and it is expected that said particles can be used equally well to immobilize pathogens or pathogen components from aqueous solution and body fluids.

### Materials and Methods

### Materials

Iron(II) chloride tetrahydrate, iron(III) chloride hexahydrate, sodium sulphate anhydrous, n-hexane and sodium dihydrogenphosphate monohydrate were purchased from Merck KGaA, Germany. 3-mercaptopropionic acid, dichloromethane, N-hydroxysuccinimide, N,N'-dicyclohexylcarbodiimide, anhydrous N,N'-dimethylformamide and sodium citrate were obtained from Sigma-Aldrich Chemie GmbH, Germany. 25% (v/v) Ammonia, (3-aminoprpyl)triethoxysilane, methanol, citric acid, sodium hydroxide, 1N hydrochloric acid, 32% (v/v) hydrochloric acid, acetonitrile, ethanol, sodium bicarbonate anhydrous, 35% (v/v) hydrogen peroxide, chloroform, ethyl acetate, disodium hydrogenphosphate anhydrous, boric acid, 1,4-dithiothreitol, Triton X-100, calcium chloride dihydrate, 1-Ethyl-3-(3-dimethylaminopropyl) carbodiimide-hydrochloride, calcium nitrate tetrahydrate and di-Ammonium hydrogen phosphate were purchased from Carl Roth GmbH & Co.- KG, Germany. Minisart NML syringe filters (0.2 and 0.8 jam pore size); Peptide RKQGRVEVLYRASWGTVC (SEQ ID NO:1) and RKQGRVEILYRGSWGTVC (SEQ ID NO:2) were synthesised by Gene Cust, Luxemburg. E. coli LPS O55:B5 (LPS 1) and E. coli LPS O26:B6 (LPS 3) was provided by SigmaAldrich. E. coli LPS O111:B4 (LPS 2) was provided by Mikrobiologisches Institut - Klinische Mikrobiologie, Immunologie und Hygiene Universitätsklinikum Erlangen und Friedrich-Alexander-Universität. Micromer^{®} Particles (50 mg/ml) have been purchased from micromod Partikeltechnologie GmbH, NdFeB magnet provided by supermangnete.de. The water used for all experiments came from a Milli-Q system (Merck KGaA, Darmstadt, Germany). E. coli was provided by Mikrobiologisches Institut - Klinische Mikrobiologie, Immunologie und Hygiene Universitätsklinikum Erlangen und Friedrich-Alexander-Universität. Ringer solution was provided by Fresenius GmbH.

### Particle Synthesis

### Synthesis of citrate coated iron oxide particles (SPION^{cit})

A modified protocol to synthesize citrate coated iron oxide particles was used. Briefly, 1 g of iron (II) chloride and 2 g iron (III) chloride (ratio 1:2) were dissolved in 50 ml water and stirred with 250 rpm under an argon atmosphere to prevent oxidation. Iron oxide was precipitated by addition of ammonia solution. After ten minutes of stirring, a solution bearing 4.4 g of sodium citrate in 10 ml was added and the mixture stirred for 30 minutes at 90°C and 450 rpm. To remove excess sodium citrate, the cooled SPIONs were sterile filtered through a 0.2 pm pore diameter syringe filter and dialysed against water with 100 kDa Spectra/Por^{®} dialysis-tubes from Spectrum Laboratories INC. Any other 100 kDa dialysis-tubing could have been also used. The dialysed particles were kept in water at 4°C until further use.

### Synthesis of HAp coated iron oxide particles (SPION^{HAp})

This synthesis was carried out according to Mondal et al. (2013 and 2017). SPION^{Cit} containing 160 mg Fe were dispersed in water and 60 mg of calcium nitrate tetrahydrate were added to the dispersion. The pH of the dispersion at 40 °C was adjusted to 9.1 by addition of NaOH (1 N). After 10 min, 30 mg of di-ammonium hydrogen phosphate were dissolved in 5 ml water and then added to the dispersion. The dispersion was kept at 40 °C for 1 hour and 250 rpm, before it was cooled to room temperature and magnetically washed with water for three times. The final dispersion was stored at 4 °C until further use.

### Synthesis of APTES coated iron oxide particles (SPION^{APTES})

The synthesis of SPION^{APTES} was performed in a modified one step co-precipitation process (Friedrich et al. (2019)). An aqueous solution of FeCl2 and FeCl3 (ratio 1:2) was stirred and heated to 90 °C under argon atmosphere. Precipitation was initiated by the addition of 25% ammonia. Afterwards, the particles were stirred at 70 °C for 15 minutes and then (3-aminopropyl)triethoxysilane (APTES) was added to the mixture. The dispersion was stirred for another 3 h before it was cooled down to RT. The particles were magnetically separated and washed three times with water. Finally, the APTES coated particles were filtered through a 0.8 pm pore diameter syringe filter and stored at 4 °C until further use.

### Particle functionalization

### Linkage of SBA to SPION^{APTE5} (SPION^{APTES-SBA})

SBA was purchased commercially (TCS0852-100MG; VWR). Of course SBA could also be self-made. The binding of SBA to SPION^{APTES} were performed according to a modified process as recently described (Karawacka et al. (2018)). In this first step, SPION^{APTES} containing 1 mg Fe were diluted in 950 µl with borate buffer (0.05 M pH 8.5; prepared from boric acid and basified with 2 N NaOH). Afterwards, SBA stock solution was added (50 µl, 20 mM in dry DMF) and the dispersion was shaken at 1400 rpm and at RT for 1 h. Next, supernatants were magnetically decanted and washed with 1 ml of borate buffer.

### Binding of peptides to SPION^{APTES-SBA}

Functionalized SPION^{APTES-SBA-SPP} were prepared using SPION^{APTES-SBA}, The final concentration in 1 ml of particle dispersion was 0.1 µmol peptide (as shown in SEQ ID NO: 1 or 2, SPP04 or SPP19) and 1 mg Fe/ml. The dispersions were shaken at RT with 1400 rpm for 2 h. Supernatants were collected as well as the supernatants of two washing steps (1 ml buffer) and the absorption was measured at 280 nm. A calibration curve was taken at concentrations of 0.01 to 0.1 µmol peptide/ml in borate buffer. The amount of particle bound peptide was calculated by subtraction of peptide detected in supernatants after functionalization and washing steps from the given peptide amount.

SPION^{APTES} without SBA as a linker molecule were used as a control. UV-Vis measurements were taken with a Libra S22 instrument (Biochrom Ltd., Cambridge, UK). Borat buffer in different concentrations were used as a blank for each measurement.

### Particle Characterization

### Iron quantification

For determination of the iron concentration, atomic emission spectroscopy (AES) was used with an Agilent 4200 MP-AES (Agilent Technologies, Santa Clara, CA, USA). A commercially available iron solution (1.000 mg/l, Bernd Kraft, Duisburg, Germany) served as external standard. Samples were diluted in a ratio of 1:20. To 20 µl diluted sample, 80 µl 65% nitric acid were added. The mixture was heated to 95 °C for 10 minutes and diluted with water to 2 ml afterwards. Measurements were done at a wavelength of 371.993 nm. These measurements were done in triplicate, the results were averaged.

### Silicon, calcium and phosphorus quantification

The content of silicon, calcium and phosphorus were determined by means of Inductively Coupled Plasma Atom Emission Spectrometry (ICP-AES, Circo CCD, Spectro Analytical Instruments GmbH, Germany). Briefly, to 100 mg of lyophilized particles, 10 ml of a mixture containing hydrofluoric acid, nitric acid and hydrochloric acid (ratio 1:1:1) was added. Afterwards, samples were diluted to a total volume of 100 ml with water. Silicon was measured at a wavelength of 251.612 nm, calcium at 396.847 nm and phosphorus at 177.495 nm. These measurements were done in triplicate, the results were averaged.

### Hydrodynamic particle size and Zeta potential

Dynamic light scattering (DLS) was performed with a Zetasizer Nano ZS (Malvern Panalytical, Almelo, Netherlands) to determine the hydrodynamic particle size of the NP at 25 °C in water (refractive index 1.33; viscosity 0.8872 mPa-s; backscattering mode at 173°). The same device was used to measure the particle's aqueous zeta potential with 78.5 as dielectric constant. Measurements were done in triplicate at an iron concentration of 50 µg/ml and pH of 7.4, the results were averaged.

### SEM Imaging

Scanning electron microscopy (SEM) images of the particles were taken with a Zeiss Auriga SEM (Carl Zeiss, Oberkochen, Germany) operated at an acceleration voltage of 3 kV. Samples were diluted to an iron concentration of 100 ug/ml, dropped on a silica wafer and lyophilized prior to the measurements.

### TEM Imaging

Transmission electron microscopy (TEM) images were taken by a CM30 TEM/STEM (Philips, Netherlands) operating at 300 kV. Specimens were prepared by drop casting of diluted nanoparticle dispersions onto carbon-coated copper grids (Plano, Germany).The used CCD camera was Tietz Fast Scan-F114.

### Magnetic susceptibility

As an indicator for the SPIONs' magnetizability, the magnetic susceptibility at an iron concentration of 1 mg/ml was measured with a MS2G magnetic susceptibility meter (Bartington Instruments, Oxfordshire, UK).

### XRD Analysis

The Rigaku MiniFlex 600 XRD was used to perform a 0/20-measurment to determine the phases and crystal structures present in the specimen. Powders were obtained from particle dispersions by lyophilisation at 1 mbar (Alpha 1-2 LDplus, Martin Christ Gefriertrocknungsanlagen). Powders were placed on a specimen holder and softly pressed. A Cu-Kα1 beam was used as the X-ray source (wavelength λ=1.54059 nm) and the angular range was 20° to 80° with a step size of 0.03°/second.

### FTIR Measurements

Fourier transform infrared (FTIR) measurements were recorded with an Alpha-P FTIR device (Bruker) equipped with an ATR crystal. Wavelength range was 400-4000 cm⁻¹ with a resolution of 4 cm⁻¹, 128 sample scans, 64 background scans. OPUS software (Bruker) was used for background subtraction and baseline correction.

### Estimation of the available amino groups on particles

The estimation of amino groups on iron-oxide based particles was done according to an existing protocol for the determination of amino group on silica-particles (Zaloga et al. 2014). Briefly, 20 µl of the sample (concentration 0.1 mg Fe/ml) was added to 190 µl of borate buffer (0.1 M, pH 8.0). An amount of 90 µl fluorescamine, dissolved in acetonitrile to a concentration of 1 mg/ml, was added to the dispersion. Calibration samples were prepared from fresh APTES in a concentration range of 0.004 to 0.1 mM. Measurements were performed at an excitation wavelength of 385 nm and an emission wavelength of 535 nm in a Filter-Max F5 Plate reader (Molecular Devices). SPION^{HAp} with no amino groups served as negative control. Significances are represented by asterisks (* p < 0.05, ** p < 0.005).

### Magnetic attractability of functionalized particles

For the experiment in static condition, 1 ml of particle solution was prepared in 2 ml Eppendorf tubes. All samples were analyzed for iron content using AES. Particle dispersions were adjusted so that the concentration was 0.3 mg Fe/ml for SPION^{APTES} and sPION^{APTES-SBA-SPP04}. As a control, an unseparated sample was measured by AES. The test samples were separated for three minutes using a permanent magnet with a strength 310 mT. The supernatants of the separated samples were removed and prepared for AES. 20 µl sample of sample was dissolved in and 80 µl nitric acid (HNO3) and then diluted and measured. Based on the results, the amount of iron originally present in the sample was calculated and then represented in **Figure 7****.**

For the experiment under flow condition, 0.4 ml of particle solution (6 mg Fe/ml) was injected into a flow. As a control, an unseparated sample was measured by AES. The test samples were separated using a electromagnet with a strength of 0.8 T. Samples have been collected in a tube after separation and were analyzed using AES. 20 µl sample of sample was dissolved in and 80 µl nitric acid (HNO3) and then diluted and measured. Based on the results, the amount of iron originally present in the sample was calculated and then represented in Figure 7.

### Studies of LPS (endotoxin) binding

Binding studies of endotoxin were performed with EndoZyme Recombinant Factor C Assay kit (Hyglos, Germany). In this kit, LPS is detected by recombinant Factor C, thus excluding possible interferences by p-glucans. Calibration curves for the three different LPSs have been prepared in endotoxin free water. SPIO^{NAPTES-SBA-SPP04} was used in this study as its efficacy has been described previously. For this experiment, we used two different iron concentrations (1 mg Fe/ml and 0.01 mg Fe/ml) and two incubation parameters (30 s at 1400 rpm and 37°C as well as 3 s at RT). Three different LPSs were added to 200 µl of the different particle dispersions. The final concentration of LPS was set to 10 EU/ml in each tested sample. Spiking controls (8 and 5 EU/ml) were carried out to see if any interference occurred for all tested particles.

Supernatants were produced using a neodymium magnet (310 mT at separation side) for three minutes. 50 µl of each supernatant were mixed with 50 µl of the assay reagent (80% v/v assay buffer, 10% v/v substrate, 10% v/v enzyme) in black, endotoxin-free 96-well plates. The reaction was monitored for 90 minutes at 37 °C in 15 min intervals by recording the fluorescence at an excitation wavelength of 360 nm and an emission at 465 nm in a Filter-Max F5 Plate reader (Molecular Devices).

### Separation of micromer^{LPS j}

For this experiment micromer^{®} particles (micromer) were incubated with endotoxin to receive endotoxin coated micromer particles (micromer^{LPS}). Any other polymeric particles of similar size could have been used as an alternative. It has been reported, that endotoxins stick to surfaces and can be attached to polymeric particles. Therefore, particles bearing 1 mg particles/ml was incubated with 1 µg/ml endotoxin LPS3 for 2 h at 1400 rpm. After centrifugation the particles were washed several times.

SPION^{APTES-SBA-SPP04} and unmodified SPION^{HAP} with a Fe concentration of 1 mg/ml were incubated with 0.1 mg micromer^{LPS} for 2 h at 1400 rpm and 37 °C. SPION^{APTES} served as a control. A calibration curve was prepared with different concentrations of micromer^{LPS}. The calibration samples and the magnetically decanted supernatants were measured in the reader at 620 nm absorption wavelength like it is used for the OD measurements of bacteria (Brenner et al. 2005 and Chen et al. 2011). Significances of SPION^{APTES-SBA-SPP04} and SPION^{HAP} particles compared to control SPION^{APTES} are represented by asterisks (* p < 0.05, ** p < 0.005).

### Separation of E.coli from blood

In this experiment we used blood from health volunteers and added an amount of bacteria to it. A concentration of 0.3 mg Fe/ml was used for the particles. To 0.8 ml of blood, (citrate or EDTA stabilized) 0.1 bacteria suspension in Ringer's solution have been added. After 5 min. of incubation, 0.1 ml of particles (3 mg Fe/ml) in Ringer's solution have been added. Samples were incubated for 5 minutes on a shaker at 700 rpm. After incubation, the particles have been removed by placing a magnet (310 mT) behind the samples. After 3 min separation of the particles the supernatant were plated out. As a negative control only 0.2 ml Ringer's solution was added to blood. The positive control consisted of 0.8 ml blood and 0.1 ml bacteria suspension and another 0.1 ml Ringer's solution. In addition the particles were washed one time after separation and then redispersed in Ringer's solution and plated out.

After 16 h of incubation, the CFUs (colony forming units) on the plates have been counted. The samples have been compared to the amount found in the positive control.

### Statistics

For statistics, an Student's t-test was performed in MS Excel (Microsoft, Redmond, WA, USA). Asterisks refer to statistical significance of * p < 0.05, ** p < 0.05.

**Sequence Table**

| SEQ ID NO | Sequence name | Sequence |
|---|---|---|
| 1 | SPP04 | RKQGRVEVLYRASWGTVC |
| 2 | SPP19 | RKQGRVEILYRGSWGTVC |
| 3 | SPP04-pS | RKQGRVEVLYRASWGTV[pS] |
| 4 | DMBT-1 | MGISTVILEMCLLWGQVLSTGGWIPRTTDYASLIPSEVPLDPTVAEGSPFPSESTLEST VAEGSPISLESTLESTVAEGSLIPSESTLESTVAEGSDSGLALRLVNGDGRCQGRVEIL YRGSWGTVCDDSWDTNDANVVCRQLGCGWAMSAPGNAWFGQGSGPIALDDVRC SGHESYLWSCPHNGWLSHNCGHGEDAGVICSAAQPQSTLRPESWPVRISPPVPTEG SESSLALRLVNGGDRCRGRVEVLYRGSWGTVCDDYWDTNDANVVCRQLGCGWAM SAPGNAQFGQGSGPIVLDDVRCSGHESYLWSCPHNGWLTHNCGHSEDAGVICSAP QSRPTPSPDTWPTSHASTAGPESSLALRLVNGGDRCQGRVEVLYRGSWGTVCDDS WDTSDANVVCRQLGCGWATSAPGNARFGQGSGPIVLDDVRCSGYESYLWSCPHNG WLSHNCQHSEDAGVICSAAHSWSTPSPDTLPTITLPASTVGSESSLALRLVNGGDRC QGRVEVLYRGSWGTVCDDSWDTNDANVVCRQLGCGWAMLAPGNARFGQGSGPIV LDDVRCSGNESYLWSCPHNGWLSHNCGHSEDAGVICSGPESSLALRLVNGGDRCQG RVEVLYRGSWGTVCDDSWDTNDANVVCRQLGCGWAMSAPGNARFGQGSGPIVLD DVRCSGHESYLWSCPNNGWLSHNCGHHEDAGVICSAAQSRSTPRPDTLSTITLPPST |
| | | VGSESSLTLRLVNGSDRCQGRVEVLYRGSWGTVCDDSWDTNDANVVCRQLGCGW ATSAPGNARFGQGSGPIVLDDVRCSGHESYLWSCPHNGWLSHNCGHHEDAGVICSV SQSRPTPSPDTWPTSHASTAGPESSLALRLVNGGDRCQGRVEVLYRGSWGTVCDDS WDTSDANVVCRQLGCGWATSAPGNARFGQGSGPIVLDDVRCSGYESYLWSCPHNG WLSHNCQHSEDAGVICSAAHSWSTPSPDTLPTITLPASTVGSESSLALRLVNGGDRC QGRVEVLYQGSWGTVCDDSWDTNDANVVCRQLGCGWAMSAPGNARFGQGSGPIV LDDVRCSGHESYLWSCPHNGWLSHNCGHSEDAGVICSASQSRPTPSPDTWPTSHA STAGSESSLALRLVNGGDRCQGRVEVLYRGSWGTVCDDYWDTNDANVVCRQLGCG WAMSAPGNARFGQGSGPIVLDDVRCSGHESYLWSCPHNGWLSHNCGHHEDAGVIC SASQSQPTPSPDTWPTSHASTAGSESSLALRLVNGGDRCQGRVEVLYRGSWGTVCD DYWDTNDANVVCRQLGCSWATSAPGNARFGQGSGPIVLDDVRCSGHESYLWSCPH NGWFSHNCGHHEDAGVICSASQSQPTPSPDTWPTSHASTAGSESSLALRLVNGGDR CQGRVEVLYRGSWGTVCDDYWDTNDANVVCRQLGCGWATSAPGNARFGQGSGPI VLDDVRCSGHESYLWSCPHNGWLSHNCGHHEDAGVICSASQSQPTPSPDTWPTSR ASTAGSESTLALRLVNGGDRCRGRVEVLYQGSWGTVCDDYWDTNDANVVCRQLGC GWAMSAPGNAQFGQGSGPIVLDDVRCSGHESYLWSCPHNGWLSHNCGHHEDAGV ICSAAQSQSTPRPDTWLTTNLPALTVGSESSLALRLVNGGDRCRGRVEVLYRGSWGT VCDDSWDTNDANVVCRQLGCGWAMSAPGNARFGQGSGPIVLDDVRCSGNESYLW SCPHKGWLTHNCGHHEDAGVICSATQINSTTTDWWHPTTTTTARPSSNCGGFLFYA SGTFSSPSYPAYYPNNAKCVWEIEVNSGYRINLGFSNLKLEAHHNCSFDYVEIFDGSL NSSLLLGKICNDTRQIFTSSYNRMTIHFRSDISFQNTGFLAWYNSFPSDATLRLVNLN SSYGLCAGRVEIYHGGTWGTVCDDSWTIQEAEVVCRQLGCGRAVSALGNAYFGSGS GPITLDDVECSGTESTLWQCRNRGWFSHNCNHREDAGVICSGNHLSTPAPFLNITRP NTDYSCGGFLSQPSGDFSSPFYPGNYPNNAKCVWDIEVQNNYRVTVIFRDVQLEGG CNYDYIEVFDGPYRSSPLIARVCDGARGSFTSSSNFMSIRFISDHSITRRGFRAEYYSS PSNDSTNLLCLPNHMQASVSRSYLQSLGFSASDLVISTWNGYYECRPQITPNLVIFTI PYSGCGTFKQADNDTIDYSNFLTAAVSGGIIKRRTDLRIHVSCRMLQNTWVDTMYIA NDTIHVANNTIQVEEVQYGNFDVNISFYTSSSFLYPVTSRPYYVDLNQDLYVQAEILH SDAVLTLFVDTCVASPYSNDFTSLTYDLIRSGCVRDDTYGPYSSPSLRIARFRFRAFHF LNRFPSVYLRCKMVVCRAYDPSSRCYRGCVLRSKRDVGSYQEKVDVVLGPIQLQTPP RREEEPR |
| 5 | Motif 1 | VEVLXXXXW |
| 6 | Motif 2 | VEILXXXXW |
| 7 | Motif 3 | VEIYXXXXW |
| 8 | Motif 4 | VEVYXXXXW |
| 9 | N-terminally | RCQGR |
| | preceding sequence | |
| 10 | Inner motif | YRGS |
| 11 | SRCR domain 6 of DMBT1 | RLVNGSDRCQGRVEVLYRGSWGTVCDDSWDTNDANVVCRQLGCGWATSAPGNAR FGQGSGPIVLDDVRCSGHESYLWSCPHNGWLSHNCGHHEDAGVICS |
| 12 | Exemplary SRCR of DMBT1 | RCQGRVEVLYRGSWGTVC |
| 13 | DMBT1 of mucin, partial [Bos taurus] | ALRLVNGSDRCQGRVEVLYGGSWGTVCDDSWDTNDANVVCRQLGCGWAISAPGD ARFGQGSGPIVLDDVGCSGYETYLWSCSHSPWNTHNCGHSEDASVICSASQTQSTV VPDWLYPTTDYGTESGLALRLVNGGDRCQGRVEVLYRGSWGTVCDDSWDTNDSNV VCRQLGCGWAISAPGNARFGQGSGPIVLDDVGCSGYETYLWSCSHNPWNTHNCGH SEDASVICSASQTQSTVVPDLWYPTTDYGTESGLALRLVNGSDRCQGRVEVLYRGS WGTVCDDSWDTNDANVVCRQLGCGWGISAPGDARFGQGSGPIVLDDVGCSGYET YLWSCSHNPWNTHNCGHSEDASVICSASQTQSTVVPDLWYPTTDYGTESGLALRLV NGSDRCQGRVEVLYGGSWGTVCDDSWDTNDANVVCRQLGCGSGISAPGDARFGQ GSGPILLDDVGCSGYETYLWSCSHSPWNSHNCGHSKDASVICSAAQINSSTPGWQP PQTTTTQTPGVNFSTPDWLSPTTTPTQTPGVNFSTPDWLSPTTTPTQTPGVNFSTP DWLSPTTTPTQTPGVNFSTPDWLSPTTTPTQTPGVNFSTPDWLSPTTT |
| 14 | DMBT1 of Mus musculus | MGISTVIFEICLLWGQILSTASQTAVPTDGTDSGLAVRLVNGGDRCQGRVEILYQGS WGTVCDDSWDLNDANVVCRQLGCGLAVSAPGNARFGQGSGPIVMDDVACGGYED YLWRCSHRGWLSHNCGHQEDAGVICSDSQTSSPTPGWWNPGGTNNDVFYPTEQT TAEQTTIPDYTPIGTDSGLAVRLVNGGDRCQGRVEILYQGSWGTVCDDSWDVSDAN VVCRQLGCGWAVSAPGNAYFGQGQGPIVLDDVACGGYENYLWSCSHQGWLSHNC GHQEDAGVICSASQSSSPTPGWWNPGGTNNDVFYPTEQTTAGTDSGLAVRLVNGG DRCQGRVEILYQGSWGTVCDDSWDTNDANVVCRQLGCGWAVSAPGNAYFGPGSG SIVLDDVACTGHEDYLWRCSHRGWLSHNCGHHEDAGVICSASQSSSPTPDVFYPTD QTTAEQTTVPDYTPIGTDSGLAVRLVNGGDRCQGRVEILYQGSWGTVCDDSWDLN DANVVCRQLGCGLAVSAPGSARFGQGTGPIVMDDVACGGYEDYLWRCSHRGWLSH NCGHHEDAGVICSASQSSSPTPDVFYPTDQTTAEQTTVPDYTPIGTDSGLAVRLVNG GDRCQGRVEILYQGSWGTVCDDSWDLNDANVVCRQLGCGLAVSAPGSARFGQGTG PIVMDDVACGGYEDYLWRCSHRGWLSHNCGHHEDAGVICSASQSSSPTPDVFYPTD QTTAEQTTVPDYTTIGTENSLAVRLENGGDRCQGRVEILYQGSWGTVCDDSWDLN DANVVCRQLGCGLAVSAPGSARFGQGTGPIVMDDVACGGYEDYLWRCSHRGWLSH NCGHHEDAGVICSASQSSSPTPDVFYPTDQTTVEQTTVPDYTPIGTENSLAVRLENG GDRCQGRVEILYQGSWGTVCDDSWDTKDANVVCRQLGCGWAVSAPGNAYFGPGS GSIVLDDVACTGHEDYLWSCSHRGWLSHNCGHHEDAGVICSDAQIQSTTRPDLWP TTTTPETTTELLTTTPYFDWWTTTSDYSCGGLLTQPSGQFSSPYYPSNYPNNARCSW KIVLPNMNRVTVVFTDVQLEGGCNYDYILVYDGPEYNSSLIARVCDGSNGSFTSTGN FMSVVFITDGSVTRRGFQAHYYSTVSTNYSCGGLLTQPSGQFSSPYYPSNYPNNARC SWEILVPNMNRVTVVFTDVQLEGGCNYDYILVYDGPQYNSSLIARVCDGSNGSFTST GNFMSVVFITDGSVTRRGFQAHYYSTVSTTPPVPIPTTDDYSCGGLLTLPSGQFSSPH YPSNYPNNARCSWEILVPNMNRVTVAFTDVQLEGGCNYDYILVYDGPEYNSSLIARV CDGSNGSFTSTGNFMSVVFITDGSVTRRGFQAHYYSTVSTNYSCGGLLTQPSGQFSS PHYPSNYPNNVRCSWEILVPSMNRVTVAFTDVQLEGGCSFDYILVYDGPEYNSSLIAP VCDGFNGSFTSTGNFMSVVFITDGSVTRRGFQAYYYSTVSTPPSFHPNITGNDSSLAL RLVNGSNRCEGRVEILYRGSWGTVCDDSWGISDANVVCRQLGCGSALSAPGNAWF GQGSGLIVLDDVSCSGYESHLWNCHHPGWLVHNCRHSEDAGVICALPEVTSPSPGW WTTSPSYVNYTCGGFLTQPSGQFSSPFYPGNYPNNARCLWNIEVPNNYRVTVVFRDL |
| | | QLERGCSYDYIEIFDGPHHSSPLIARVCDGSLGSFTSTSNFMSIRFITDHSITARGFQA HYYSDFDNNTTNLLCQSNHMQASVSRSYLQSMGYSARDLVIPGWNSSYHCQPQITQ REVIFTIPYTGCGTIKQADNETINYSNFLRAVVSNGIIKRRKDLNIHVSCKMLQNTWV NTMYITNNTVEIQEVQYGNFDVNISFYTSSSFLFPVTSSPYYVDLDQNLYLQAEILHS DASLALFVDTCVASPHPNDFSSLTYDLIRSGCVRDDTYQSYSSPSPRVSRFKFSSFHFL NRFPSVYLQCKLVVCRAYDTSSRCYRGCVVRSKRDVGSYQEKVDVVLGPIQLQSPSK EKRSLDLAVEDVKKPASSQAVYPTAAIFGGVFLAMVLAVAAFTLGRRTHIDRGQPPST KL |
| 15 | DMBT1 from rat Isoform 1 | MGISIVIFEICLLWGQILSTASQSRSSTPDWWNHGGTINDVIYDTQETPEVTTTQVP DSTSIGTDSGLAVRLVNGGDRCRGRVEILYQGSWGTMCDDGTDSGLAVRLVNGGD RCRGRVEILYQGSWGTMCDDSWDINDANVVCRQLGCGWALSAPGSAQFGQGLGPI VLDDVACRGHEAYLWSCSHRGWLSHNCGHQEDAGVICSDSQTSSPTPGWWNPGG TNNDVIYDTQETTETSQTSSPTPDWWNHGGTINDVIYDTQETTEGTDSGLAVRLVN GGDRCRGRVEILYQGSWGTVCDDSWDINDANVVCRQLGCGWALSAPGSAQFGQG SGSIVLDDVACRGHEAYLWSCSHRGWLSHNCGHQEDAGVICSYSQTSSPTPDSQTS SPTPGWWNPGGTNNDVSYGPEQTTDATDSGLAVRLVNGGDRCQGRVEILYQGSW GTVCDDSWDTKDANVVCRQLVCGWALSAPGSAHFGQGSGSIVLDDVACTGHEAYL WSCSHRGWLSHNCGHHEDAGVICSDAQTQSTTWPDMWPTTTPETTTDWWTTKY SSSVPTTQFPTIADWWTTPSPEYTCGGLLTLPYGQFSSPYYPGSYPNNARCLWKIFVS SMNRVTVVFTDVQLEGGCNYDYILVFDGPENNSSLIARVCDGFNGSFTSTQNFMSVV FITDGSVTRRGFQADYYSTPISTSTTSPTTFPIVTDWWTTPSPEYTCGGLLTLPYGQF SSPYYPGSYPNNARCLWKIFVPSMNRVTVVFTDVQLEGGCNYDYILGFDGPEYNSSLI ARVCDGSNGSFTSTQNFMSVVFITDGSVTRRGFQADYYSTPIRTSTTPPTTFPIITGN DSSLVLRLVNGTNRCEGRVEILYRGSWVPCADDSWDINDANVVCRQLGCGSALSAP GNAWFGQGSGLIVLDDVSCSGYESHLWNCRHPGWLVHNCRHVEDAGVICSLPDPT PSPGPVWTSPPFVNYTCGGFLTGLSGQFSSPYYPGSYPNNARCLWNIEVPNNYRVTV VFRDVQLEGGCNYDYIEIFDGPHHSSPLIARVCDGAMGSFTSTSNFMSVRFTTDHSV TRRGFRADYYSDFDNNTTNLLCLSNHMRASVSRSYLQSMGYSSRDLVIPGWNVSYQ CQPQITQREVIFTIPYTGCGTTKQADNETINYSNFLKAAVSNGIIKRRKDLHIHVSCK MLQNTWVNTMYITNNTVEIQEVQYGNFDVNISFYTSSSFLYPVTSSPYYVDLDQNLY LQAEVLHSDTSLALFVDTCVASPHPNDFSSLTYDLIRSGCIRDETYQSYSSPSPRITRF KFSSFHFLNRFPSVYLQCKLVVCRANDVSSRCYRGCVVRSKRDVGSYQEKVDVVLGPI QLQSPSKEKRSLDLAVADVEKPASSQEVYPTAAIFGGVFLALVVAVAAFTLGRKTRTA RGQPPSTKM |
| 16 | DMBT1 from rat Isoform 2 | MGISIVIFEICLLWGQILSTASQSRSSTPDWWNHGGTINDVIYDTQETPEVTTTQVP DSTSIGTDSGLAVRLVNGGDRCRGRVEILYQGSWGTMCDDSWDINDANVVCRQLG CGWALSAPGSAQFGQGLGPIVLDDVACRGHEAYLWSCSHRGWLSHNCGHQEDAGV ICSDSQTSSPTPGWWNPGGTNNDVIYDTQETTETSQTSSPTPDWWNHGGTINDVI YDTQETTEGTDSGLAVRLVNGGDRCRGRVEILYQGSWGTVCDDSWDINDANVVCR QLGCGWALSAPGSAQFGQGSGSIVLDDVACRGHEAYLWSCSHRGWLSHNCGHQED AGVICSYSPTSSPTPGWWNPGFTNSDVSYRTELPTDDSQTSSPTPDSQTSSPTPGW WNPGGTNNDVSYGPEQTTDATDSGLAVRLVNGGDRCQGRVEILYQGSWGTVCDDS WDTKDANVVCRQLVCGWALSAPGSAHFGQGSGSIVLDDVACTGHEAYLWSCSHRG WLSHNCGHHEDAGVICSDAQTQSTTWPDMWPTTTPETTTDWWTTKYSSSVPTTQ FPTIADWWTTPSPEYTCGGLLTLPYGQFSSPYYPGSYPNNARCLWKIFVSSMNRVTV VFTDVQLEGGCNYDYILVFDGPENNSSLIARVCDGFNGSFTSTQNFMSVVFITDGSV TRRGFQADYYSTPISTSTTSPTTFPIVTDWWTTPSPEYTCGGLLTLPYGQFSSPYYPG SYPNNARCLWKIFVPSMNRVTVVFTDVQLEGGCNYDYILGFDGPEYNSSLIARVCDG SNGSFTSTQNFMSVVFITDGSVTRRGFQADYYSTPIRTSTRNDSSLVLRLVNGTNRC EGRVEILYRGSWGTVCDNSWDINDANVVCRQLGCGSALSAPGNAWFGQGSGLIVLD DVSCSGYESHLWNCRHPGWLVHNCRHVEDAGVICSLPDPTPSPGPVNYTCGGFLTG |
| | | LSGQFSSPYYPGSYPNNARCLWNIEVPNNYRVTVVFRDVQLEGGCNYDYIEIFDGPH HSSPLIARVCDGAMGSFTSTSNFMSVRFTTDHSVTRRGFRADYYSDFDNNTTNLLCL SNHMRASVSRSYLQSMGYSSRDLVIPGWNVSYQCQPQITQREVIFTIPYTGCGTTKQ ADNETINYSNFLKAAVSNGIIKRRKDLHIHVSCKMLQNTWVNTMYITNNTVEIQEVQ YGNFDVNISFYTSSSFLYPVTSSPYYVDLDQNLYLQAEVLHSDTSLALFVDTCVASPHP NDFSSLTYDLIRSGCIRDETYQSYSSPSPRITRFKFSSFHFLNRFPSVYLQCKLVVCRA NDVSSRCYRGCVVRSKRDVGSYQEKVDVVLGPIQLQSPSKEKRSLDLAVADVEKPAS SQEVYPTAAIFGGVFLALVVAVAAFTLGRKTRTARGQPPSTKM |
| 17 | DMBT1 from pig Isoform 1 | MGTSAVILEICLLLSQVLTTVSSTTQTESTTEDRTQITETAFWETQTINSVSESDLPGT HASSFHTEEPLTTIAAEGTEWDLALRLVNGGDRCQGRVEILYQGSWGTVCDDSWDT NDANVVCRQLGCGWAVSAPGSARFGQGLGPILLDDLRCSGHETYLWSCPHSGWKT HNCGHQEDAGVICSGAQRSSTVIPDWWYTTTRSQTAHIRSTIPAWWHPTTTTAAR TEWDLALRLVNGGDRCQGRVEVLYQGSWGTVCDDSWDTNDANVVCRQLGCGWA VSAPGSARFGQGSGPILLDDLRCSGHETYLWSCPHSGWNTHNCGHHEDAGVICSDA QRSSTVIPDWWYTTTPSQTAHIRSTIPAWWHPTTTTAARTEWDLALRLVNGGDRC QGRVEVLYQGSWGTVCDDSWDTNDANVVCRQLGCGWAVSAPGSARFGQGSGPILL DDLRCSGHETYLWSCPHSGWNTHNCGHHEDAGVICSDAQRSSTVIPDWWYTTTPS QTWWHPTTTTAASPSSPCGGFLTSASGTFSSPSYPGLYPNNANCVWEIEVNSGYRIN LGFNNLQLEVHINCIYDYIEIFDESPGSNTSLGKICNHTSQIFTSSYNRMTVRFRSDGS VQKPGFSAWYNSFPRDASLRLVNSNSSYPSCAGRVEIYQGGRWGTVCDDGWDIQD AQVVCRQLGCGNAVSAPGNAYFGPGSGPITLDDVACSGTESTLWQCRNRGWFSHN CGHSEDAGVICSVPAFTTTPPATNYSCGGFLSQAAGGFNSPFYPGNYPNNANCVWDI EVQNNYRVTVVFRDVQLESGCNFDYIEVFDGPYRSSPLLARVCNGASGSFTSSSNFM SIRFISDVSVTRAGFRANYYSSPSSGSTRLHCLQNHMQASVSTSYLQSLGYSARDLVI PGWEWSYQCQPQITSTQVTFTIPYSSCGTIQRVDNDTITYSNSLRAAVSSGIIKRKKD LNMYVSCRMLQNTWVNTVYIANDTLEVQNVQYGNFDVNISFFTSSSFLYPVRSSPYY VDLNQNLYLQAELLHANASLALFVDTCVASPYPNDFTTLTYDLIRSGCVKDETYQSYS QPSPRIVRFKFSSFHFLSRFPSVYLQCKMVVCRAFDSSSRCRRGCVVRSKRDVGSYQE KVDVVLGPIQLQTLHAEKRSLDQPAVDLEEKASAQGSYDGAAISAGIFLVVVLAVAAF TLGRRGRAADGQPLISKT |
| 18 | DMBT1 from pig Isoform 2 | MGTSAVILEICLLLSQVLTTVSSTTQTESTTEDRTQITETAFWETQTINSVSESDLPGT HASSFHTEEPLTTIAAEGTEWDLALRLVNGGDRCQGRVEILYQGSWGTVCDDSWDT NDANVVCRQLGCGWAISAPGSARFGQGSGPILLDDLRCSGHETYLWSCPHSGWKT HNCGHQEDAGVICSGAQRSSTVIPAWWHPTTTTTATSSFHTEEPLTTIAAEGTEWD LALRLVNGGDRCQGRVEILYQGSWGTVCDDSWDTNDANVVCRQLGCGWAVSAPG SARFGQGLGPILLDDLRCSGHETYLWSCPHSGWKTHNCGHQEDAGVICSGAQRSST VIPDWWYTTTRSQTAHIRSTIPAWWHPTTTTAARTEWDLALRLVNGGDRCQGRVE VLYQGSWGTVCDDSWDTNDANVVCRQLGCGWAVSAPGSARFGQGSGPILLDDLRC SGHETYLWSCPHSGWNTHNCGHHEDAGVICSDAQRSSTVIPDWWYTTTPSQTAHI RSTIPAWWHPTTTTAARTEWDLALRLVNGGDRCQGRVEVLYQGSWGTVCDDSWD TNDANVVCRQLGCGWAVSAPGSARFGQGSGPILLDDLRCSGHETYLWSCPHSGWN THNCGHHEDAGVICSDAQRSSTVIPDWWYTTTPSQTWWHPTTTTAASPSSPCGGF LTSASGTFSSPSYPGLYPNNANCVWEIEVNSGYRINLGFNNLQLEVHINCIYDYIEIFD ESPGSNTSLGKICNHTSQIFTSSYNRMTVRFRSDGSVQKPGFSAWYNSFPRDASLRL VNSNSSYPSCAGRVEIYQGGRWGTVCDDGWDIQDAQVVCRQLGCGNAVSAPGNAY FGPGSGPITLDDVACSGTESTLWQCRNRGWFSHNCGHSEDAGVICSVPAFTTTPPA |
| | | TNYSCGGFLSQAAGGFNSPFYPGNYPNNANCVWDIEVQNNYRVTVVFRDVQLESGC NFDYIEVFDGPYRSSPLLARVCNGASGSFTSSSNFMSIRFISDVSVTRAGFRANYYSSP SSGSTRLHCLQNHMQASVSTSYLQSLGYSARDLVIPGWEWSYQCQPQITSTQVTFTI PYSSCGTIQRVDNDTITYSNSLRAAVSSGIIKRKKDLNMYVSCRMLQNTWVNTVYIA NDTLEVQNVQYGNFDVNISFFTSSSFLYPVRSSPYYVDLNQNLYLQAELLHANASLAL FVDTCVASPYPNDFTTLTYDLIRSGCVKDETYQSYSQPSPRIVRFKFSSFHFLSRFPSV YLQCKMVVCRAFDSSSRCRRGCVVRSKRDVGSYQEKVDVVLGPIQLQTLHAEKRSLD QPAVDLEEKASAQGSYDGAAISAGIFLVVVLAVAAFTLGRRGRAADGQPLISKT |
| 19 | DMBT1 16-mer | QGRVEVLYRGSWGTVC |
| 20 | DMBT1 residues involved in binding | xRVEVLYxxSWxxxx |
| 21 | Motif 5 | VEILxxxxWGTV |
| 22 | Motif 6 | VEVLxxxxWGTV |
| 23 | Motif 7 | VEIYxxxxWGTV |
| 24 | Motif 8 | VEILYRGSWGTV |
| 25 | Motif 9 | VEVLYRGSWGTV |
| 26 | Motif 10 | VEIYHGGTWGTV |
| 27 | Motif 11 | VEVLxQ |
| 28 | Motif 12 | VEIFxxxxDSSL |
| 29 | Motif 13 | VWLxxxxW |
| 30 | Motif 14 | VEVRxxxxW |
| 31 | Motif 15 | VEISxxxxW |
| 32 | Motif 16 | VGVLxxxxW |
| 33 | Motif 17 | VEVSxxxxW |
| 34 | Motif 18 | VEVFxxxxW |
| 35 | Motif 19 | VELRxxxxW |
| 36 | Motif 20 | VELFxxxxW |
| 37 | Motif 21 | VEVHxxxxW |
| 38 | Motif 22 | VELFxxxxW |
| 39 | Motif 23 | VEILxxxxW |
| 40 | Motif 24 | VEIFxxxxW |
| 41 | Motif 25 | VEFFxxxxW |
| 42 | Motif 26 | VELHxxxxW |
| 43 | Motif 27 | LEVRxxxxW |
| 44 | Motif 28 | VEVQxxxxW |
| 45 | Motif 29 | VELLxxxxW |
| 46 | Motif 30 | VELYxxxxPPAL |
| 47 | Motif 31 | LEVFxxxxW |

### List of references

WO 2005/079834
Ali, A., et al., Synthesis, characterization, applications, and challenges of iron oxide particles. Nanotechnol Sci Appl, 2016. 9: p. 49-67.End, C., et al., DMBT1 functions as pattern-recognition molecule for poly-sulfated and poly-phosphorylated ligands. Eur J Immunol, 2009. 39(3): p. 833-42.
Brenner, N.R.K., J. R. Staley, G. Garrity, Bergey's Manual of Systematic Bacteriology. Vol. 2. 2005: Springer US.
Chen, Y. and Y. Zhang, Fluorescent quantification of amino groups on silica particle surfaces. Anal Bioanal Chem, 2011. 399(7): p. 2503-9.
Derrington, I.M., et al., Nanopore DNA sequencing with MspA. Proc Natl Acad Sci USA, 2010. 107(37):p. 16060-5.
End C, Bikker F, Renner M, Bergmann G, Lyer S, Blaich S, Hudler M, Helmke B, Gassler N, Autschbach F, Ligtenberg AJ, Benner A, Holmskov U, Schirmacher P, Nieuw Amerongen AV, Rosenstiel P, Sina C, Franke A, Hafner M, Kioschis P, Schreiber S, Poustka A, Mollenhauer J. DMBT1 functions as pattern-recognition molecule for poly-sulfated and poly-phosphorylated ligands. Eur J Immunol. 2009 Mar;39(3):833-42.
Friedrich, B., et al., Peptide functionalized magnetic particles for experimental sepsis therapy. Infection, 2019: p. 13.
Hartshorn KL, White MR, Mogues T, Ligtenberg T, Crouch E, Holmskov U. Lung and salivary scavenger receptor glycoprotein-340 contribute to the host defense against influenza A viruses. Am J Physiol Lung Cell Mol Physiol. 2003 Nov;285(5):L1066-76
Herrmann, I.K., et al., Endotoxin removal by magnetic separation-based blood purification. Adv Healthc Mater, 2013. 2(6): p. 829-35.
Jeong, J., et al., Bioactive calcium phosphate materials and applications in bone regeneration. Biomater Res, 2019. 23: p. 4.
Kang, J. H.; Super, M.; Yung, C. W.; Cooper, R. M.; Domansky, K.; Graveline, A. R., et al., An extracorporeal blood-cleansing device for sepsis therapy. Nat Med 2014, 20 (10), 1211-1216.
Karawacka, W., et al., SPIONs functionalized with small peptides for binding of lipopolysaccharide, a pathophysiologically relevant microbial product. Colloids Surf B Biointerfaces, 2019. 174: p. 95-102.
Kunitomo, T.and Shoji, H., Endotoxin removal by toraymyxin. Blood Purification in Intensive Care 2001, 132, 415-420.
Lee, J.J., et al., Synthetic ligand-coated magnetic particles for microfluidic bacterial separation from blood. Nano Lett, 2014. 14(1): p. 1-5.
Leito JT, Ligtenberg AJ, Nazmi K, de Blieck-Hogervorst JM, Veerman EC, Nieuw Amerongen AV. A common binding motif for various bacteria of the bacteria-binding peptide SRCRP2 of DMBT1/gp-340/salivary agglutinin. Biol Chem. 2008 Sep;389(9): 1193-200.
Ligtenberg AJ, Karlsson NG, Veerman EC. Deleted in malignant brain tumors-1 protein (DMBT1): a pattern recognition receptor with multiple binding sites. Int J Mol Sci. 2010;11(12):5212-33.
Liu, Y., et al., Kinetics of (3-aminopropyl)triethoxylsilane (APTES) silanization of superparamagnetic iron oxide particles. Langmuir, 2013. 29(49): p. 15275-82.
Northrop, BH, Frayne SH, Choudhary U Thiol-Maleimide "Click"Chemistry: Evaluating the Influence of Solvent, Initiator, and Thiol on the Reaction Mechanism, Kinetics, and Selectivity. Polym. Chem., 2015,6, 3415-3430
Malamud D, Abrams WR, Barber CA, Weissman D, Rehtanz M, Golub E. Antiviral activities in human saliva. Adv Dent Res. 2011 Apr;23(1):34-7.
Mondal, B.M., Apurba Dey, Sudit S. Mukhopadhyay, Studies on Processing and Characterization of Hydroxyapatite Biomaterials from Different Bio Wastes. Journal of Minerals & Materials Characterization & Engineering,, 2012: p. 55-67.
Mondal, S., et al., Hydroxyapatite Coated Iron Oxide Particles: A Promising Nanomaterial for Magnetic Hyperthermia Cancer Treatment. Nanomaterials (Basel), 2017. 7(12).
Prakobphol A, Xu F, Hoang VM, Larsson T, Bergstrom J, Johansson I, Frängsmyr L, Holmskov U, Leffler H, Nilsson C, Borén T, Wright JR, Strömberg N, Fisher SJ. Salivary agglutinin, which binds Streptococcus mutans and Helicobacter pylori, is the lung scavenger receptor cysteine-rich protein gp-340. J Biol Chem. 2000 Dec 22;275(51):39860-6.
Reich, J., et al., Masking of endotoxin in surfactant samples: Effects on Limulus-based detection systems. Biologicals, 2016. 44(5): p. 417-22.
Rhodes, A., et al., Surviving Sepsis Campaign: International Guidelines for Management of Sepsis and Septic Shock: 2016. Crit Care Med, 2017. 45(3): p. 486-552.
Schwertmann, R. M. C. U., The Iron Oxides: Structure, Properties, Reactions, Occurrences and Uses: Structure, Properties, Reactions, Occurences and Uses. 2003.
Sneha, M.and Sundaram, N. M., Preparation and characterization of an iron oxide-hydroxyapatite nanocomposite for potential bone cancer therapy. Int J Nanomedicine 2015, 10 Suppl 1, 99-106.
Wu K, Su D, Liu J, Saha R, Wang JP. Magnetic nanoparticles in nanomedicine: a review of recent advances. Nanotechnology. 2019 Dec 13;30(50):502003. doi: 10.1088/1361-6528/ab4241. Epub 2019 Sep 6. PMID: 31491782.
Wu Z., Van Ryk D., Davis C., Abrams W.R., Chaiken I., Magnani J., Malamud D. Salivary agglutinin inhibits HIV type 1 infectivity through interaction with viral glycoprotein 120, AIDS Research and Human Retroviruses, (2003)19(3), pp. 201-209.
Zainol Abidin, M., Goh, P., Ismail, A., Said, N., Othman, M., Hasbullah, H., Abdullah, M., Ng, B., Sheikh Abdul Kadir, S., Kamal, F. Polysulfone/Iron Oxide Nanoparticles Ultrafltration Membrane for Adsorptive Removal of Phosphate from Aqueous Solution. Journal of Membrane Science and Research, 2019; 5(1): 20-24.
Zaloga, J., et al., Development of a lauric acid/albumin hybrid iron oxide particle system with improved biocompatibility. International Journal of Nanomedicine, 2014. 9: p. 4847-4866.
Zhang, Q.W., * Timothy R. Smith, William E. Hurst, and Thomas Sulpizio, Endotoxin Removal Using a Synthetic Adsorbent of Crystalline Calcium Silicate Hydrate. Biotechnol. Prog., 2005: p. 1220-122.

## Claims

1. A method for reducing the concentration of pathogens and/or pathogen components in an aqueous or body fluid sample, the method comprising the steps of:
a. providing the aqueous or body fluid sample;
b. incubating the sample with superparamagnetic iron-based particles, wherein the superparamagnetic iron-based particles are linked to a target binding peptide, wherein the target is a pathogen or a pathogen component; and
c. immobilising the superparamagnetic iron-based particles with a magnetic field and thereby separating the pathogen-bound and/or pathogen component-bound superparamagnetic iron-based particles from the sample;
whereby a reduced concentration of pathogens and/or pathogen components in the sample is obtained,
wherein the superparamagnetic iron-based particles are magnetically attractable and wherein the magnetic attractability is **characterized by** a reduction of the superparamagnetic iron-based particle concentration in Ringer solution by 65% to 99.95% when applying a magnetic field of 0.31 Tesla for three minutes in static condition.

2. The method of any of the preceding claims, wherein the superparamagnetic iron-based particles are selected from and iron oxide, iron (Fe), iron-cobalt, alnico, permalloy particles, preferably wherein the superparamagnetic iron-based particles are superparamagnetic ironoxide nanoparticles (SPIONs).

3. The method of any of the preceding claims, wherein the sample is a body fluid sample, waste water, ground water, or drinking water, preferably wherein the body fluid sample is a human body fluid, more preferably wherein the human body fluid is selected from blood, serum, plasma, lymph, urine, liquor, saliva and sputum, even more preferably wherein the human body fluid is blood or serum, even more preferably wherein the human body fluid is blood, and most preferably wherein the blood is from a septic patient.

4. The method of any of preceding claims, wherein the concentration of pathogens and/or pathogen components in the sample of step (c) is reduced by at least 20%, preferably at least 25%, more preferably at least 30%, more preferably at least 35%, even more preferably at least 40%, even more preferably at least 45%, and even more preferably at least 50%, compared to the sample of step (a).

5. The method of any of the preceding claims, wherein the pathogens comprise bacteria, fungi and/or viruses, preferably wherein the pathogens are bacteria, preferably wherein the pathogens comprise gram-negative and/or a gram-positive bacteria, more preferably wherein the gram-negative bacteria are selected from one or more of *Escherichia coli, Pseudomonas spp.,* and *Klebsiella* spp., and/or the gram-positive bacteria are selected from one or more of *Staphylococcus aureus, Serratia, Streptococcus spp., Listeria monocytogenes, Clostridium difficile,* and *Enterobacter.*

6. The method of any of the preceding claims, wherein the pathogen components comprise endotoxins and/or pathogenic cell wall components,
preferably wherein the endotoxins are derived from gram-negative bacteria, more preferably wherein the toxins are derived from of one or more of *Klebsiella* spp., *Escherichia coli (Ecoli),* and *Pseudomonas aeruginosa,* more preferably wherein the endotoxins comprise LPS, preferably wherein the endotoxins consist of LPS, even more preferably wherein the endotoxins comprise *E.coli* LPS, and even more preferably wherein LPS is selected from one or more of LPS 055:B5, LPS O26:B6, and LPS 0111:B4; and/or
preferably wherein the pathogenic cell wall component is derived from one or more of gram-positive bacteria, fungi and viruses, preferably wherein the cell wall component is derived from gram-positive bacteria, more preferably wherein the cell wall component is LTA, teichoic acid and/or a peptidoglycan, even more preferably wherein the pathogenic cell wall component is LTA, even more preferably wherein the pathogenic cell wall component is LTA of *Staphylococcus aureus* and/or *Streptococcus pyrogenes.*

7. The method of any of the preceding claims, wherein the target binding peptide has a sequence comprising 12-30 amino acids having one motif selected from SEQ ID NO: 5 (VEVLxxxxW), SEQ ID NO: 6 (VEILxxxxW), SEQ ID NO: 7 (VEIYxxxxW) and SEQ ID NO:8 (VEVYxxxxW), preferably wherein the target binding peptide has a sequence comprising 15-25 amino acids having one motif selected from SEQ ID Nos:5-8, more preferably wherein the target binding peptide has a sequence comprising 16-20 amino acids having one motif selected from SEQ ID Nos: 5-8, more preferably wherein the target binding peptide has a sequence comprising 17-19 amino acids having one motif selected from SEQ ID Nos: 5-8, even more preferably wherein the target binding peptide has a sequence comprising 17-19 amino acids having the motif of SEQ ID NO:5, even more preferably wherein the target binding peptide has a sequence comprising 17-19 amino acids having the motif of SEQ ID NO:5 and wherein the motif is N-terminally preceded by 4-6 amino acids, most preferably wherein the target binding peptide has a sequence comprising 17-19 amino acids having the motif of SEQ ID NO:5 and wherein the motif is N-terminally preceded by SEQ ID NO:9 (RCQGR).

8. The method of any of the preceding claims, wherein the superparamagnetic iron-based particles are covalently linked to the target binding peptide via a connecting module; or wherein the superparamagnetic iron-based particles are bound to the target binding peptide via a bond between a phosphate group on the target binding peptide and the superparamagnetic iron-based particle;
preferably wherein the connecting module is hydroxyapatite or a serine-derived aldehyde at a terminus of the target binding peptide, and more preferably wherein hydroxyapatite is covalently linked to an acidic amino acid at a terminus of the target binding peptide; or
preferably wherein the connecting module consists of an anchor unit and optionally a linker unit, more preferably wherein the anchor unit is covalently linked to the superparamagnetic iron-based particle and to the target binding peptide or to the linker unit, even more preferably wherein the anchor unit is a molecule comprising an amino group and a silane, even more preferably wherein the anchor unit is an aminosilane, more preferably wherein the aminosilane is (3-aminopropyl)-triethoxysilane (APTES), (3-aminopropyl)-diethoxy-methylsilane (APDEMS), (3-aminopropyl)-dimethyl-ethoxysilane (APDMES), or (3-aminopropyl)-trimethoxysilane (APTMS), even more preferably wherein the aminosilane is (3-Aminopropyl)triethoxysilan (APTES);
optionally wherein the linker unit is covalently linking the anchor unit and to the target binding peptide, preferably wherein the linker unit is N-succinimidyl bromoacetate (SBA) or succinimidyl 3-(2-pyridyldithio)propionate) (SPDP), and preferably wherein the linker is SBA.

9. The method of any of the preceding claims, wherein the superparamagnetic iron-based particles are SPIONs and wherein the SPIONs linked to a target binding peptide are capable of reducing the concentration of LPS in step (c) by at least 70%, preferably 80%, more preferably 90%, and even more preferably 95%, compared to the sample of step (a) comprising a LPS concentration of 10 EU/ml, wherein the reduction is determined after incubating the SPIONs linked to a target binding peptide at a concentration of 1 mg Fe/ml in Ringer solution followed by immobilising the SPIONs linked to a target binding peptide in a magnetic field of 0.31 Tesla for a time period of 30 seconds by using an assay based on the detection of recombinant factor C.

10. A method for identifying pathogens in an aqueous or body fluid sample, the method comprising the steps of
a. providing the aqueous or body fluid sample;
b. incubating the sample with superparamagnetic iron-based particles, wherein the superparamagnetic iron-based particles are linked to a pathogen binding peptide;
c. immobilising the superparamagnetic iron-based particles with a magnetic field and thereby separating the pathogen-bound superparamagnetic iron-based particles from the sample; and
d. identifying the separated pathogens,
wherein the superparamagnetic iron-based particles are magnetically attractable and wherein the magnetic attractability is **characterized by** a reduction of the superparamagnetic iron-based particle concentration in Ringer solution by 65% to 99.95% when applying a magnetic field of 0.31 Tesla for three minutes in static condition.

11. The method of claim 10, wherein the separated pathogens are still viable, preferably wherein the viability is determined by multiplying the pathogens under suitable conditions, more preferably by plating of the pathogens on a suitable growth plate, and even more preferably wherein at least one colony forming unit (CFU) is obtained by plating.

12. The method of claims 10 or 11, wherein the sample is further defined in claim 3, the pathogens are further defined in claim 5, the pathogen-binding peptide shows the same characteristics as the target-binding peptide as defined in claim 7, and/or the superparamagnetic iron-based particles are defined in claim 2 and/or 8.

13. Use of superparamagnetic iron-based particles for reducing the concentration of pathogens and/or pathogen components in an aqueous or body fluid sample, wherein the superparamagnetic iron-based particles are linked to a target binding peptide,
wherein the target is a pathogen or a pathogen component,
wherein the superparamagnetic iron-based particles are magnetically attractable, and wherein the magnetic attractability is **characterized by** a reduction of the magnetic particle concentration in Ringer solution by 65% to 99.95% when applying a magnetic field of 0.31 Tesla for three minutes in static condition,
preferably wherein the sample is further defined in claim 3, the target binding peptide is defined in claim 7, the pathogens are defined in claim 5, the pathogen components are defined in claim 6, and/or the superparamagnetic iron-based particles are defined in claim 2 and/or 8.

14. Use of superparamagnetic iron-based particles for identifying pathogens in a blood sample,
wherein the superparamagnetic iron-based particles are linked to a target binding peptide, wherein the target is a pathogen,
wherein the superparamagnetic iron-based particles are magnetically attractable, and wherein the magnetic attractability is **characterized by** a reduction of the magnetic particle concentration in Ringer solution by 65% to 99.95% when applying a magnetic field of 0.31 Tesla for three minutes in static condition,
preferably wherein the sample is further defined in claim 3, preferably wherein the pathogens are further defined in claim 5, the pathogen-binding peptide shows the same characteristics as the target-binding peptide as defined in claim 7, and/or the superparamagnetic iron-based particles are defined in claims 2 and/or 8.

15. Superparamagnetic ironoxide nanoparticles (SPIONs), wherein the SPIONs are linked to a target binding peptide, wherein the target is a pathogen and/or a pathogen component, wherein the SPIONs are magnetically attractable and wherein the magnetic attractability is **characterized by** a reduction of the SPION concentration in water by 65% to 99.95% when applying a magnetic field of 0.31 Tesla for three minutes in static condition.
